# EUROPÄISCHE PATENTSCHRIFT

(11) **EP 0 657 166 B1**
(45) Veröffentlichungstag und Bekanntmachung des Hinweises auf die Patenterteilung: **09.04.2003**
(21) Anmeldenummer: 94119146.2
(22) Anmeldetag: 05.12.1994
(51) Int. Cl.: A61K 31/70, A61K 31/495, A61P 31/12

(54) **Kombinationspräparate, enthaltend ein Chinoxalin und ein Nukleosid**
Combination containing a quinoxaline and a nucleoside
Composition contenant un quinoxaline et un nucléosid

(30) Priorität: 09.12.1993 DE 4342024
(43) Veröffentlichungstag der Anmeldung: 14.06.1995
(73) Patentinhaber: Aventis Pharma Deutschland GmbH, 65929 Frankfurt am Main (DE)
(72) Erfinder: Meichsner, Christoph, Dr., D-65835 Liederbach (DE); Riess, Günther, Dr., D-65795 Hattersheim (DE); Kleim, Jörg-Peter, Dr., D-65779 Kelkheim (DE); Rösner, Manfred, Dr., D-65817 Eppstein (DE); Paessens, Arno, Dr., D-42781 Haan (DE); Blunck, Martin, Dr., D-42113 Wuppertal (DE)

(56) Entgegenhaltungen:
- EP-A- 0 509 398

## Beschreibung

Gegenstand der vorliegenden Erfindung sind Kombinationspräparate enthaltend mindestens ein Chinoxalin und mindestens ein Nukleosid.
Chinoxaline sind eine altbekannte Verbindungsklasse (O. Hinsberg, J. Liebigs Ann. Chem. 237, 327 (1986)).
In der Patentliteratur sind Chinoxalinderivate für verschiedene medizinische Anwendungen beschrieben.
Die österreichische Patentschrift 28 48 48 (19.12.67) nennt 1-N-Dialkylaminoalkyl-3,4-dihydro-chinoxalin-2(1H)-one als Spasmolytika.
Antiinflammatorisch wirkende 4-N-Aroyl-, Arylacyl- und Arylsulfonyl-3,4-dihydro-chinoxalin-2(1H)-one werden in einer Reihe von Patentanmeldungen der japanischen Firma Sumitomo Chem. Co. Ltd. beschrieben (JA 17 137/69 (11.4.66), JA 17 136/69 (8.4.66), JA 7 008/422 (9.8.66), BE 706 623 (16.11.66)). 3,4-Dihydro-chinoxalin-2(1H)-on-3-carboxamide sind in der US-Patentanmeldung US 3 654 275 (4.4.72) enthalten. Sie wirken ebenfalls antiinflammatorisch. Als antihypertensive und antisekretorische Reagenzien werden Pyridinylalkyl- tetrahydro-pyrazino[1,2-a]chinoxalinon-derivate in den US- Anmeldungen US 4 203 987 (21.5.79) und 4 032 639 (22.3.76) von American Home Prod. Corp. beschrieben. Eine europäische Patentanmeldung von Pfizer Inc. (EP 266 102 A (30.10.86)) beinhaltet 4-N-Benzotsulfonyl-3,4-dihydro-chinoxalin-2(1H)-on-1-alkylcarbonsäurenals Aldose-Reduktase-Inhibitoren.
Antivirale Aktivität wurde bisher jedoch noch nicht nachgewiesen.
Ein weiteres Dokument betreffend Chinoxaline ist die EP 0 509 398, auf die an dieser Stelle ausdrücklich Bezug genommen wird.
Es wurde nun überraschenderweise gefunden, daß Chinoxaline der Formeln I und la, 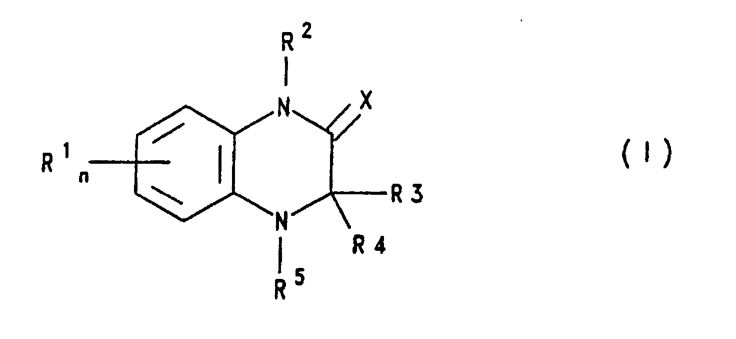 sowie deren tautomere Formen der allgemeinen Formel la 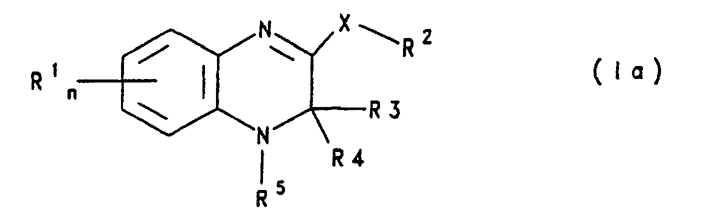 sowie deren physiologisch verträgliche Salze
oder Prodrugs in Kombination mit mindestens einem Nukleosid eine antivirale Wirkung insbesondere gegen Retroviren, wie zum Beispiel das 'Human tmmunodeficiency Virus' (HIV) aufweisen.

In den erfindungsgemäßen Verbindungen der Formel I bzw. la bedeuten:
1)
   - n: null,
   eins
   oder zwei,
die einzelnen Substituenten R¹ unabhängig voneinander
Fluor, Chlor, Brom, Trifluormethyl, Hydroxy, C1 - C4-Alkyl, C1 - C4-Alkoxy, (C1 - C4-Alkoxy)-(C1 - C4-alkoxy), C1- C4-Alkylthio, Nitro, Amino, C1 - C4-Alkylamino, Di(C1 - C4-alkyl)amino, Piperidino, Morpholino, 1-Pyrrolidinyl, 4-Methylpiperazinyl, C1 - C4-Acyl, C1 - C4-Acyloxy, C1 - C4-Acylamino, Cyano, Carbamoyl, Carboxy, (C1 - C4-Alkyl)-oxycarbonyl, Hydroxysulfonyl, Sulfamoyl
oder
einen mit bis zu zwei
voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C1 - C4-Alkyl, C1 - C4-Alkoxy, (C1 - C4-Alkyl)-oxycarbonyl, Phenyl, Phenoxy,
sein kann,
- R²: Wasserstoff und R⁵
C1 - C6-Alkyl,
gegebenenfalls substituiert mit Fluor,
Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C2 - C6-Alkenyl,
gegebenenfalls substituiert mit Fluor, Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C3 - C8-Allenyl,
C3 - C8-Alkinyl,
gegebenenfalls substituiert mit Fluor,
Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C3 - C8-Cycloalkyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C3 - C8-Cycloalkenyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
(C3 - C6-Cycloalkyl)-(C1 - C2-alkyl),
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
(C3 - C6-Cycloalkenyl)-(C1 - C2-alkyl),
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C1 - C6-Alkylcarbonyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, C1 - C4-Alkenylamino, Di(C1 - C4-alkyl)amino, 1-Pyrrolidinyl, Piperidino, Morpholino, 4-Methylpiperazin-1-yl, C1 - C4-Alkylthio, Oxo, Thioxo, Carboxy, Carbamoyl;
C2 - C6-Alkenylcarbonyl, gegebenenfalls substituiert durch Fluor, Chlor oder Hydroxy;
(C3 - C6-Cycloalkyl)carbonyl,
(C5 - C6-Cycloalkenyl)carbonyl,
(C3 - C6-Cycloalkyl)-(C1 - C2-alkyl)carbonyl,
(C5 - C6-Cycloalkenyl)-(C1 - C2-alkyl)carbonyl,
C1 - C6-Alkyloxycarbonyl, gegebenenfalls substituiert durch Fluor, Chlor, Brom, Hydroxy, C1 - C4-Alkoxy, C1 - C4-Alkylamino,
Di(C1 - C4-alkyl)amino, C1 - C4-Alkylthio;
C2 - C6-Alkenyloxycarbonyl, gegebenenfalls substituiert durch Fluor, Chlor, Hydroxy, C1 - C4-Alkoxy;
C2 - C6-Alkinyloxycarbonyl, gegebenenfalls substituiert durch Fluor, Chlor, Hydroxy, C1 - C4-Alkoxy;
C1 - C6-Alkylthiocarbonyl, gegebenenfalls substituiert durch Fluor, Chlor, Hydroxy, C1 - C4-Alkoxy;
C2 - C6-Alkenylthiocarbonyl, gegebenenfalls substituiert durch Fluor, Chlor, Hydroxy, C1 - C4-Alkoxy;
C1 - C6-Alkylamino- und Di(C1 - C6-alkyl)aminocarbonyl, gegebenenfalls substituiert durch Fluor, Chlor, Hydroxy, C1 - C4-Alkoxy;
Pyrrolidin-1-yl, Morpholino-, Piperidino-, Piperazinyl-, oder 4-Methylpiperazin-1-yl-carbonyl;
C2 - C6-Alkenylamino- und Di(C1 - C6-alkenyl)aminocarbonyl, gegebenenfalls substituiert durch Fluor, Chlor, Hydroxy, C1 - C4-Alkoxy;
C1 - C4-Alkylsulfonyl, gegebenenfalls substituiert durch Fluor, Chlor, Hydroxy, C1 - C4-Alkoxy;
C1 - C4-Alkenylsulfonyl;
oder mit bis zu zwei voneinander unabhängigen Resten R⁶ substituiertes Aryl, Arylcarbonyl, (Arylthio)carbonyl, Aryloxycarbonyl, Arylaminocarbonyl, (Arylamino)thiocarbonyl, Arylsulfonyl, Arylalkylaminocarbonyl, Arylalkyl, Arylalkenyl, Arylalkylcarbonyl, Arylalkoxycarbonyl, Aryl(alkylthio)carbonyl, wobei der Alkylrest jeweils 1 bis 3 C-Atome enthalten kann und R⁶ wie oben definiert ist
oder mit bis zu zwei voneinander unabhängigen Resten R⁶ substituiertes 1- oder 2-Naphthylmethyl, 2-, 3- oder 4-Picolyl, 2- oder 3-Furylmethyl, 2-oder 3-Thienylmethyl, 2- oder 3-Pyrrolylmethyl, 2-, 3- oder 4-Pyridylcarbonyl, 2- oder 3-Furylcarbonyl, 2- oder 3-Thienylcarbonyl, 2- oder 3-Thienylacetyl, 2-, 3- oder 4-Picolyloxycarbonyl, 2- oder 3-Furylmethyloxycarbonyl, 2- oder 3-Thienylmethyloxycarbonyl,
und
- R³ und R⁴: gleich oder verschieden, unabhängig voneinander Wasserstoff, C1 - C4-Alkyl, gegebenenfalls substituiert mit Fluor, Chlor, Hydroxy, Amino, Mercapto, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
C2 - C6-Alkenyl, gegebenenfalls substituiert mit Fluor oder Chlor;
C3 - C6-Cycloalkyl, gegebenenfalls substituiert mit Fluor, Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
C3 - C8-Cycloalkenyl, gegebenenfalls substituiert mit Fluor oder Chlor;
mit bis zu zwei voneinander unabhängigen Resten R⁶ substituiertes Aryl, Benzyl, Heteroaryl oder Heteroarylmethyl bedeuten,
R³ und R⁴ können ferner auch
Teil eines gesättigten oder ungesättigten carbo- oder heterocyclischen Ringes mit 3 bis 6 C-Atomen sein, der gegebenenfalls mit Fluor, Chlor, Hydroxy, Amino, C1 - C4-Acyloxy, Benzoyloxy, C1 - C4-Alkoxy, Oxo, Thioxo, Carboxy, Carbamoyl substituiert sein kann, und
- X: bedeutet Sauerstoff oder Schwefel.

In einer bevorzugten Gruppe von Verbindungen der Formel I bzw. la bedeuten:
2)
   - n: null,
   eins
   oder zwei,
die einzelnen Substituenten R¹ unabhängig voneinander
Fluor, Chlor, Brom, Trifluormethyl, Hydroxy, C1 - C4-Alkyl, C1 - C4-Alkoxy, (C1 - C4-Alkoxy)-(C1 - C2-alkoxy), C1- C4-Alkylthio, Nitro, Amino, C1 - C4-Alkylamino, Di(C1 - C4-alkyl)amino, Piperidino, Morpholino, 1-Pyrrolidinyl, 4-Methylpiperazinyl, C1 - C4-Acyl, C1 - C4-Acyloxy, C1 - C4-Acylamino, Cyano, Carbamoyl, Carboxy, (C1 - C4-Alkyl)-oxycarbonyl, Hydroxysulfonyl, Sulfamoyl
oder
einen mit bis zu zwei
voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C1 - C4-Alkyl, C1 - C4-Alkoxy, (C1 - C4-Alkyl)-oxycarbonyl, Phenyl, Phenoxy
sein kann,
R² Wasserstoff und R⁵
C1 - C6-Alkyl
gegebenenfalls substituiert mit C1 - C4-Alkoxy oder C1 - C4-Alkylthio;
C2 - C6-Alkenyl,
gegebenenfalls substituiert mit Oxo;
C3 - C6-Allenyl;
C3 - C8-Alkinyl, insbesondere 2-Butinyl;
C3 - C6-Cycloalkyl;
C5 - C6-Cycloalkenyl;
(C3 - C6-Cycloalkyl)-(C1 - C2-alkyl), insbesondere Cyclopropylmethyl, gegebenenfalls substituiert mit C1 - C4-Alkyl;
(C3 - C6-Cycloalkenyl)-(C1 - C2-alkyl), insbesondere Cyclohexenylmethyl;
C1 - C6-Alkylcarbonyl,
gegebenenfalls substituiert mit Fluor,
Chlor, Hydroxy, Benzyloxy, Phenoxy, C1 - C4-Alkoxy,
C1 - C4-Alkylamino, C1 - C4-Alkenylamino, Di(C1 - C4-alkyl)amino, 1-Pyrrolidinyl, Piperidino, Morpholino, 4-Methylpiperazin-1-yl, C1 - C4-Alkylthio;
C2 - C6-Alkenylcarbonyl;
C1 - C6-Alkyloxycarbonyl, gegebenenfalls substituiert durch Fluor, Chlor, Brom, Hydroxy, C1 - C4-Alkoxy, C1 - C4-Alkylamino, Di(C1 - C4-alkyl)amino, C1 - C4-Alkylthio;
C2 - C6-Alkenyloxycarbonyl, insbesondere Vinyloxycarbonyl, Allyloxycarbonyl, Isopropenyloxycarbonyl, Butenyloxycarbonyl, Pentenyloxycarbonyl;
C2 - C6-Alkinyloxycarbonyl, insbesondere Propinyloxycarbonyl, Butinyloxycarbonyl;
C1 - C6-Alkylthiocarbonyl;
C2 - C6-Alkenylthiocarbonyl, insbesondere Allylthiocarbonyl;
C1 - C6-Alkylamino- und Di(C1 - C6-alkyl)aminocarbonyl;
Pyrrolidin-1-yl, Morpholino-, Piperidino-, Piperazinyl-, oder 4-Methylpiperazin-1-yl-carbonyl;
C2 - C6-Alkenylamino- und Di(C1 - C6-alkenyl)aminocarbonyl;
C1 - C4-Alkylsulfonyl;
C1 - C4-Alkenylsulfonyl;
oder mit bis zu zwei voneinander unabhängigen Resten R⁶ substituiertes Aryl, insbesondere Phenyl,
Arylcarbonyl, insbesondere Benzoyl, (Arylthio)carbonyl, Aryloxycarbonyl, Arylaminocarbonyl, (Arylamino)thiocarbonyl, Arylalkylaminocarbonyl, Arylsulfonyl,
Arytalkyl, insbesondere Benzyl, Phenylethyl, Arylalkenyl,
Arylalkylcarbonyl, Arylalkoxycarbonyl, Aryl(alkylthio)carbonyl, wobei der Alkylrest jeweils 1 bis 3 C-Atome enthalten kann und R⁶ wie oben definiert ist
oder mit bis zu zwei voneinander unabhängigen Resten R⁶ substituiertes 1- oder 2-Naphthylmethyl, 2-, 3- oder 4-Picolyl, 2- oder 3-Furylmethyl, 2-oder 3-Thienylmethyl, 2- oder 3-Pyrrolylmethyl,
2-, 3- oder 4-Pyridylcarbonyl, 2- oder 3-Furylcarbonyl, 2- oder 3-Thienylcarbonyl, 2- oder 3-Thienylacetyl, 2-, 3- oder 4-Picolyloxycarbonyl, 2- oder 3-Furylmethyloxycarbonyl, 2- oder 3-Thienylmethyloxycarbonyl,
und
- R³ und R⁴: gleich oder verschieden, unabhängig voneinander
Wasserstoff,
C1 - C4-Alkyl,
gegebenenfalls substituiert mit Hydroxy, Mercapto,
C1 - C4-Alkoxy, C1 - C4-Alkylthio, C1 - C4-Alkylsulfonyl, C1-C4-Alkylsulfinyl, Carboxy, Carbamoyl;
C2 - C6-Alkenyl,
mit bis zu zwei voneinander unabhängigen Resten R⁶ substituiertes Aryl, Benzyl, Thienyl oder Thienylmethyl, wobei R⁶ wie oben definiert ist, bedeuten,
R³ und R⁴ können auch
Teil eines gesättigten oder ungesättigten carbo- oder heterocyclischen Ringes mit 3 bis 6 C-Atomen sein, der gegebenenfalls mit Oxo oder Thioxo substituiert sein kann und
- X: bedeutet Sauerstoff oder Schwefel.

Eine ganz besondere Bedeutung haben Verbindungen der Formel I oder la wie oben beschrieben, dadurch gekennzeichnet, daß die genannten Substituenten bedeuten:
- n: null oder
eins
die einzelnen Substituenten R¹ unabhängig voneinander
Fluor, Chlor, Brom, C1 - C2-Alkyl, C1 - C2-Alkoxy, C2 - C4-Acyl, Cyano
- R²: Wasserstoff und R⁵
C2-C6-Alkenyl
C3 - C8-Alkinyl, insbesondere 2-Butinyl;
(C3 - C6-Cycloalkyl)-(C1 - C2-alkyl), insbesondere Cyclopropylmethyl, gegebenenfalls substituiert mit C1 - C4-Alkyl;
(C3 - C6-Cycloalkenyl)-(C1 - C2-alkyl), insbesondere Cyclohexenylmethyl;
C2 - C6-Alkylcarbonyl,
C2 - C6-Alkenylcarbonyl;
C1 - C6-Alkyloxycarbonyl;
C2 - C6-Alkenyloxycarbonyl, insbesondere Vinyloxycarbonyl, Allyloxycarbonyl, Isopropenyloxycarbonyl, Butenyloxycarbonyl, Pentenyloxycarbonyl;
C2 - C6-Alkinyloxycarbonyl, insbesondere Propinyloxycarbonyl, Butinyloxycarbonyl;
C2 - C6-Alkenylthiocarbonyl, insbesondere Allylthiocarbonyl;
C1 - C4-Alkylsulfonyl;
C1 - C4-Alkenylsulfonyl;
oder mit bis zu zwei voneinander unabhängigen Resten R⁶ substituiertes Arylalkyl, insbesondere Benzyl oder Arylalkenyl, wobei der Alkylrest jeweils 1 bis 3 C-Atome und der Alkenylrest 2-3 C-Atome enthalten kann
oder mit bis zu zwei voneinander unabhängigen Resten R⁶ substituiertes 1-Naphthylmethyl, 2- oder 3-Picolyl, 2-Furylmethyl, 2- oder 3-Thienylmethyl,
wobei R⁶
Fluor, Chlor, Brom, Cyano, C1 - C2-Alkyl, C1 - C2-Alkoxy
und
- R³ und R⁴: gleich oder verschieden, unabhängig voneinander
Wasserstoff,
C1 - C4-Alkyl,
gegebenenfalls substituiert mit Hydroxy, Mercapto, C1 - C4-Alkoxy, C1 - C2-Alkylthio und X bedeutet Sauerstoff oder Schwefel.

Die in den vorangegangenen Definitionen genannten Alkylgruppen können geradkettig oder verzweigt sein. Sofern nicht anders definiert, enthalten sie vorzugsweise 1-8, besonders bevorzugt 1-6, insbesondere 1-4 C-Atome. Beispiele sind die Methyl-, Ethyl-, Propyl-, 1-Methylethyl-, Butyl-, 1-Methylpropyl-, 2-Methylpropyl-, 1,1-Dimethylethylgruppe und ähnliche.

Die in den vorangegangenen Definitionen genannten Alkenylgruppen können geradkettig oder verzweigt sein und enthalten 1 bis 3 Doppelbindungen. Sofern nicht anders definiert, enthalten diese Gruppen vorzugsweise 2-8, insbesondere 2-6 C-Atome. Beispiele sind die 2-Propenyl-, 1-Methylethenyl, 2-Butenyl-, 3-Butenyl-, 2-Methyl-2-propenyl-, 3- Methyl-2-butenyl, 2,3-Dimethyl-2-butenyl, 3,3-Dichlor-2-propenyl, Pentadienylgruppe und ähnliche.

Die in den vorangegangenen Definitionen genannten Alkinylgruppen können geradkettig oder verzweigt sein und enthalten 1 bis 3 Dreifachbindungen. Sofern nicht anders definiert, enthalten sie vorzugsweise 2-8, besonders bevorzugt 3-6 C-Atome. Beispiele sind die 2-Propinyl- und 3-Butinylgruppe und ähnliche.

Die in den vorangegangenen Definitionen genannten Cycloalkyl- und Cycloalkenylgruppen enthalten, sofern nicht anders definiert, vorzugsweise 3-8, besonders bevorzugt 4-6 C-Atome. Beispiele sind die Cyclopropyl-, Cyclobutyl-, Cyclopentyl-, Cyclopentenyl-, Cyclohexyl- oder Cyclohexenylgruppe.

Die in den vorangegangenen Definitionen genannten Acylgruppen können aliphatisch, cycloaliphatisch oder aromatisch sein. Sofern nicht anders definiert, enthalten sie vorzugsweise 1-8, besonders bevorzugt 2-7 C-Atome. Beispielhafte Acylgruppen sind die Formyl-, Acetyl-, Chloracetyl-, Trifluoracetyl-, Hydroxyacetyl-, Propionyl-, Butyryl-, Isobutyryl-, Pivaloyl-, Cyclohexanoyl- oder Benzoylgruppe.

Die in den vorausgegangenen Definitionen genannten Arylgruppen sind vorzugsweise aromatische Gruppen mit 6-14 C-Atomen, insbesondere mit 6-10 C-Atomen wie z.B. Phenyl, Naphthyl.

In den obengenannten heterocyclischen Ringen bzw. Heteroarylgruppen kommen als Heteroatome insbesondere zum Beispiel O, S, N in Betracht, wobei im Falle eines an dieser Stelle gesättigten N-haltigen Ringes N-Z vorliegt, worin Z, H oder R⁵ mit den jeweiligen oben beschriebenen Definitonen bedeutet.

Soweit nicht anders definiert, haben die heterocyclischen Ringe vorzugsweise 1-13 C-Atome und 1-6 Heteroatome, insbesondere 3-9 C-Atome und 1-4 Heteroatome.

Für die in den vorangegangenen Definitionen genannten Heteroarylgruppen kommen beispielsweise heteroaromatische Reste wie 2- oder 3- Thienyl, 2- oder 3-Furyl, 2-, 3- oder 4- Pyridyl, Pyrimidyl, Indolyl, Chinolyl oder Isochinolyl in Frage.

Die in den vorausgegangenen Definitionen aufgeführten Aralkylgruppen sind beispielsweise Benzyl, Phenylethyl, Naphthylmethyl oder Styryl.

Die obengenannten Substituenten R¹ bis R⁵ sind vorzugsweise 3-fach, besonders bevorzugt 2-fach, insbesondere einfach mit den jeweils angegebenen Substituenten substituiert.

Für die jeweiligen zusammengesetzten Substituentendefinitionen (wie z.B. Arylalkoxycarbonyl) sind die zuvor als bevorzugt beschriebenen Bereiche für die einzelnen Substituenten ebenfalls bevorzugt.

In Abhängigkeit von den verschiedenen Substituenten können Verbindungen der Formeln I und Ia mehrere asymmetrische Kohlenstoffatome besitzen.
Gegenstand der Erfindung sind deshalb sowohl die reinen Stereoisomeren als auch Mischungen derselben, wie z.B. das zugehörige Racemat.
Die reinen Stereoisomeren der Verbindungen der Formeln I und la lassen sich durch bekannte Methoden oder in Analogie zu bekannten Methoden direkt herstellen oder nachträglich trennen.

Erfindungsgemäß einsetzbar sind Zidovudin (AZT), und Lamivudin (3-TC®), insbesondere Zidovudin. Die genannten Nukleoside lassen sich nach allgemein bekannten Verfahren herstellen (vgl. Merck-Index, 11. Auflage Rahway, N.J. 1989, Drugs 45 (4), 488 ff., 45 (5), 637 ff., 1993, Gruds 44 (4), 656 ff., 1992, Clin. Pharmacol. Ther. 55, No. 2, 198, 1994, Antiviral-Chem. Chemother. 2, Nr. 3, 125-32, 1991, Antiviral-Rest. 23, Suppl. 1, 67, 1994, Abstracts of the 34^{th} JCAAC, Orlando 4.-7.10.94).
Die Verbindungen der Formeln I und la lassen sich nach bekannten Methoden oder Modifikationen derselben herstellen (s. z.B. Rodd's Chemistry of Carbon Compounds, S. Coffey, M. F. Ansell (Herausgeber); Elsevier, Amsterdam, 1989; Vol. IV Teil IJ, S. 301 - 311. Heterocyclic Compounds, R. C. Elderfield (Herausgeber); Wiley, New York, 1957; Vol. 6, S. 491 - 495).
Die Herstellung von Verbindungen der Formel I und la wie oben unter 1) - 2) erläutert, erfolgt z.B., indem
A) zur Herstellung von Verbindungen der Formel I mit X gleich Sauerstoff und den Resten R¹, R², R³, R⁴ und R⁵ wie in unter 1) - 2) definiert eine Verbindung der Formel II, 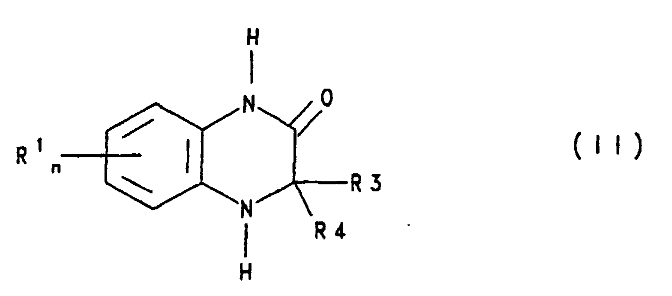 wobei für R¹, R³ und R⁴ die in unter 1) - 2) genannten Definitionen gelten, mit einer Verbindung der Formel III,

   R-Z (III)

   wobei R die oben unter 1) - 2) genannten Bedeutungen für R⁵ und R² mit Ausnahme von Wasserstoff, Hydroxy, C1 - C6-Alkoxy, Aryloxy, C1 - C6-Acyloxy, Amino, C1 - C6-Alkylamino, Di(C1 - C6-alkyl)amino, Arylamino, C1-C6-Acylamino hat und Z eine Abgangsgruppe ist umgesetzt wird,
   oder
indem B) Verbindungen der Formel I, mit X gleich Schwefel und R¹, R², R³, R⁴ und R⁵ wie unter 1) - 2) definiert hergestellt werden durch Reaktion einer Verbindung der Formel I, wobei X Sauerstoff ist und für R¹, R², R³, R⁴ und R⁵ die unter 1) - 2) genannten Definitionen gelten, mit einem Schwefelungsreagenz,
   oder
indem C)
   Verbindungen der Formel la, wobei X und die Reste R¹ bis R⁵ wie unter 1) - 2) definiert, hergestellt werden, indem man eine Verbindung der Formel IV, 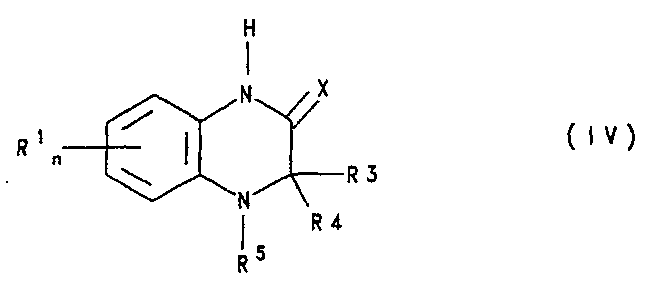 bzw. 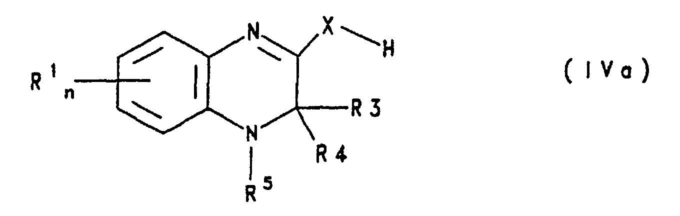 wobei für R¹, R³, R⁴ und R⁵ die unter 1) - 2) genannten Definitionen gelten, mit einer Verbindung der Formel III,

   R²-Z (III)

   wobei für R² die unter 1) - 2) für Formel I und la beschriebenen Definitionen mit Ausnahme von Wasserstoff, Hydroxy, C1 - C6-Alkoxy, Aryloxy, C1 - C6-Acyloxy, Amino, C1 - C6-Alkylamino, Di(C1 - C6-alkyl)amino, Arylamino, C1 - C6-Acylamino gelten und Z eine Abgangsgruppe ist umsetzt,
   oder
indem D)
   Verbindungen der Formel I mit X gleich Sauerstoff und den Resten R¹ bis R⁵ wie unter 1) - 2) definiert durch Cyclisierung einer Verbindung der Formel V 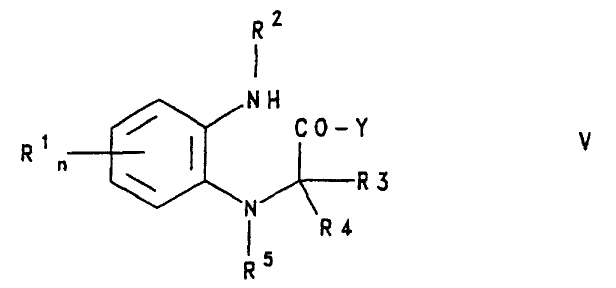 mit R¹ bis R⁵ wie unter 1) - 2) definiert und Y gleich Hydroxy, C1 - C4-Alkoxy, ggf. halogeniertes C1 - C4-Acyloxy, Chlor, Brom oder Jod, hergestellt werden, oder
indem E)
   Verbindungen der Formel I, wobei X Sauerstoff ist, R⁴ und R⁵ Wasserstoff sind und für R¹ bis R³ die unter 1) - 2) genannten Definitionen gelten, aus den Chinoxalinonen der Formel XI, 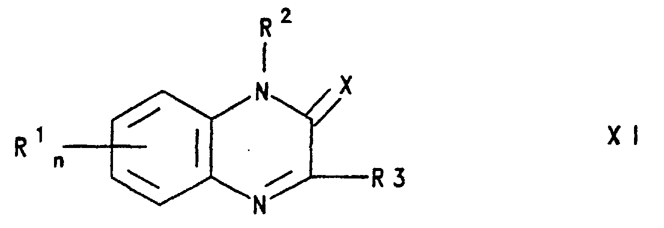 mit R¹ bis R³ wie unter 1) - 2) definiert, hergestellt werden, indem man an die C = N-Bindung Wasserstoff anlagert,
   oder
indem F)
   Verbindungen der Formel I, wobei X Sauerstoff und R¹ bis R⁵ wie unter 1) - 2) definiert, hergestellt werden aus Verbindungen der Formel VI, 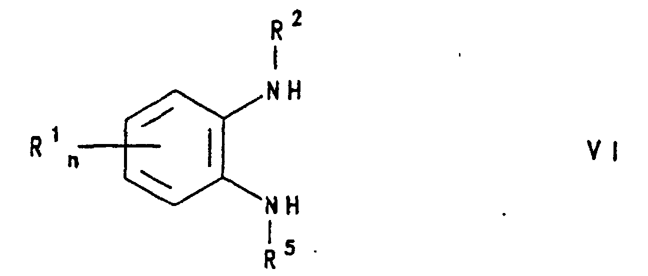 mit R¹, R² und R⁵ wie unter 1) - 2) definiert, durch Umsetzung mit Chloroform oder Bromoform und einer Carbonylverbindung der Formel XIII,

   R³-CO-R⁴ (XIII)

   mit R³ und R⁴ wie unter 1) - 2) definiert oder mit *α*-(Trihalogenmethyl)-alkanolen der Formel XIV,

   Hal₃C-C(OH)-R³R⁴ (XIV)

   worin Hal für Cl, Br oder J steht,
   in denen R³ und R⁴ wie unter 1) - 2) definiert sind,
   oder
indem G)
   Verbindungen der Formel I, mit X gleich Sauerstoff und R¹, R², R³, R⁴ und R⁵ wie unter 1) - 2) definiert, durch Reaktion einer Verbindung der Formel I, wobei X Sauerstoff ist, für R¹, R², R⁵ sowie für R³ und R⁴ die unter 1) - 2) genannten Definitionen gelten, außer daß mindestens einer der Reste R³ oder R⁴ Wasserstoff ist, mit einem Alkylierungsreagenz der Formel XV,

   R'-Z (XV)

   wobei R' die oben angegebenen Bedeutungen für R³ und R⁴ mit Ausnahme von Wasserstoff hat und Z eine Abgangsgruppe ist, hergestellt werden,
   oder
indem H)
   Verbindungen der Formel I, mit X gleich Sauerstoff, R¹, R², R³ und R⁴ wie unter 1) - 2) definiert und R⁵ C1 - C8-Alkyl, gegebenenfalls substituiert mit Fluor, Chlor, Brom, Jod, Hydroxy, C1 - C6-Acyloxy, Benzoyloxy, Phenoxy, C1 - C6-Alkoxy, C1 - C6-Alkylamino, Di(C1 - C6-alkyl)amino, C1 - C6-Alkylthio, Cyano, Carboxy, Carbamoyl, C3 - C8-Alkenyl, gegebenenfalls substituiert mit Fluor, Chlor, Brom, Jod, Hydroxy, C1 - C6-Acyloxy, Benzoyloxy, Phenoxy, C1 - C6-Alkoxy, C1 - C6-Alkylamino, Di(C1 - C6-alkyl)amino, C1 - C6-Alkylthio, Cyano, Carboxy, Carbamoyl, C3 - C8-Alkinyl, gegebenenfalls substituiert mit Fluor, Chlor, Brom, Jod, Hydroxy, C1 - C6-Acyloxy, Benzoyloxy, Phenoxy, C1 - C6-Alkoxy, C1 - C6-Alkylamino, Di(C1 - C6-alkyl)amino, C1 - C6-Alkylthio, Cyano, Carboxy, Carbamoyl, C4 - C8-Cycloalkyl, gegebenenfalls substituiert mit Fluor, Chlor, Brom, Jod, Hydroxy, C1 - C6-Acyloxy, Benzoyloxy, Phenoxy, C1 - C6-Alkoxy, C1 - C6-Alkylamino, Di(C1 - C6-alkyl)-amino, C1 - C6-Alkylthio, Cyano, Carboxy, Carbamoyl, C5 - C8-Cycloalkenyl, gegebenenfalls substituiert mit Fluor, Chlor, Brom, Jod, Hydroxy, C1 -C6-Acyloxy, Benzoyloxy, Phenoxy, C1-C6-Alkoxy, C1 - C6-Alkylamino, C1 - C6-Dialkylamino, C1 - C6-Alkylthio, Cyano, carboxy, Carbamoyl, (C1 - C6-Alkoxy)-(C1 - C6-alkyl), Di(C1 - C6-alkylamino)-(C1 - C6-alkyl) oder (C3 - C6-Cycloalkyl)alkyl, (C6 - C8-Cycloalkenyl)alkyl, mit bis zu fünf voneinander unabhängigen Resten R⁶ substituiertes Arylalkyl, Naphthylalkyl oder Heteroarylalkyl, wobei der Alkylrest jeweils 1 bis 3 C-Atome enthalten kann,
   durch reduktive Alkylierung einer Verbindung der Formel I, wobei R⁵ Wasserstoff und X Sauerstoff sind und für R¹, R², R³ und R⁴ die unter 1) - 2) genannten Definitionen gelten mit einer Carbonylverbindung der Formel XVI,

   R''-C(=O)-R''' (XVI),

   wobei R" und R''' gleich oder verschieden, unabhängig voneinander Wasserstoff, C1 - C7-Alkyl, gegebenenfalls substituiert mit Fluor, Chlor, Brom, Jod, Hydroxy, C1 - C6-Acyloxy, Benzoyloxy, Phenoxy, C1 - C6-Alkoxy, C1 - C6-Alkylamino, Di(C1 - C6-alkyl)amino, C1 - C6-Alkylthio, Cyano, Carboxy, Carbamoyl, C3 - C7-Alkenyl, gegebenenfalls substituiert mit Fluor, Chlor, Brom, Jod, Hydroxy, C1 - C6-Acyloxy, Benzoyloxy, Phenoxy, C1 - C6-Alkoxy, C1 - C6-Alkylamino, Di(C1 - C6-alkyl)amino, C1 - C6-Alkylthio, Cyano, Carboxy, Carbamoyl, C3 - C7-Alkinyl, gegebenenfalls substituiert mit Fluor, Chlor, Brom, Jod, Hydroxy, C1 - C6-Acyloxy, Benzoyloxy, Phenoxy, C1 - C6-Alkoxy, C1 - C6-Alkylamino, Di(C1 - C6-alkyl)amino, C1 - C6-Alkylthio, Cyano, Carboxy, Carbamoyl, C4 - C8-Cycloalkyl, gegebenenfalls substituiert mit Fluor, Chlor, Brom, Jod, Hydroxy, C1 - C6-Acyloxy, Benzoyloxy, Phenoxy, C1 - C6-Alkoxy, C1 - C6-Alkylamino, Di(C1 - C6-alkyl)amino, C1 - C6-Alkylthio, Cyano, Carboxy, Carbamoyl, C6-Cycloalkenyl, gegebenenfalls substituiert mit Fluor, Chlor, Brom, Jod, Hydroxy, C1 - C6-Acyloxy, Benzoyloxy, Phenoxy, C1 - C6-Alkoxy, C1 - C6-Alkylamino, Di(C1 - C6-alkyl)amino, C1 - C6-Alkylthio, Cyano, Carboxy, Carbamoyl, (C1 - C6-Alkoxy)-(C1 - C5-alkyl), [Di(C1 - C6-alkyl)amino]-(C1 - C5-alkyl) oder (C4 - C6-Cycloalkyl)alkyl, (C6-Cycloalkenyl)alkyl, mit bis zu fünf voneinander unabhängigen Resten R⁶ substituiertes Arylalkyl, Naphthylalkyl oder Heteroarylalkyl, wobei der Alkylrest jeweils 0 bis 2 C-Atome enthalten kann, sein können,
   und wobei R" und R''' unter Bildung eines 4- bis 8-gliedrigen Ringes miteinander verknüpft sein können, hergestellt werden
oder I)
   Verbindungen der Formel I, mit X gleich Sauerstoff und R¹, R², R³ und R⁴ wie unter 1) - 2) definiert und R⁵ C1 - C8-Alkyloxycarbonyl, C1 - C8-Alkylthiocarbonyl, C2 - C8-Alkenyloxycarbonyl, C2 - C8-Alkenylthiocarbonyl, C2 - C8-Alkinyloxycarbonyl, C1 - C6-Alkylaminocarbonyl, C3 - C6-Alkenylaminocarbonyl, Di(C1 - C6-alkyl)aminocarbonyl, Pyrrolidin-1-yl, Morpholino-, Piperidino-, Piperazinyl-, 4-Methylpiperazin-1-yl-carbonyl, gegebenenfalls substituiert mit Fluor, Chlor, Brom, Jod, Cyano, Amino, Mercapto, Hydroxy, C1 - C6-Acyloxy, Benzoyloxy, Benzyloxy, Phenoxy, C1 - C6-Alkoxy, C1 - C6-Alkylamino, Di(C1 - C6-alkyl)amino, C1 - C6-Alkylthio, C1 - C6-Alkylsulfonyl, Phenylsulfonyl, Oxo, Thioxo, Carboxy, Carbamoyl;
   oder mit bis zu fünf voneinander unabhängigen Resten R⁶ substituiertes Aryloxycarbonyl, Arylthio(carbonyl), Arylaminocarbonyl, Heteroaryloxycarbonyl, Heteroarylthiocarbonyl, Heteroarylaminocarbonyl, Arylalkyloxycarbonyl, (Arylalkylthio)carbonyl, Aryalkylaminocarbonyl, Heteroalkyloxycarbonyl, (Heteroalkylthio)carbonyl, Heteroalkylaminocarbonyl, wobei der Alkylrest jeweils 1 bis 3 C-Atome enthalten kann, hergestellt werden, indem man eine Verbindung der Formel XVII, 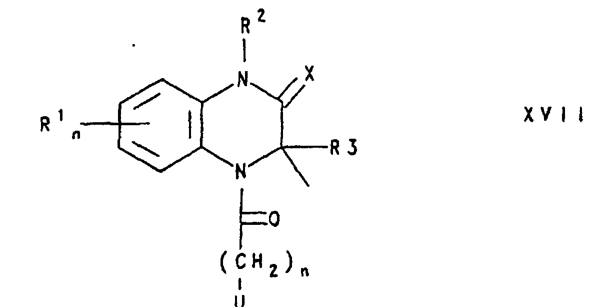 wobei für R¹, R², R³ und R⁴ die unter 1) - 2) genannten Definitionen gelten, n gleich 0, 1, 2 oder 3 ist, X gleich Sauerstoff und U eine Abgangsgruppe ist, mit einer Verbindung der Formel XVIII,

   Nu - H (XVIII),

   wobei Nu C1 - C8-Alkoxy, C2 - C8-Alkenyloxy, C2 - C8-Alkinyloxy, C1 - C8-Alkylthio, C2 - C8-Alkenylthio, C1 - C8-Alkylamino- und Di(C1 - C8-alkyl)amino, C2 - C8-Alkenylamino- und Di(C1 - C6-alkyl)amino, gegebenenfalls substituiert durch Fluor, Chlor, Brom, Hydroxy, C1 - C4-Alkoxy, C1 - C4-Alkylamino, Di(C1 - C4-alkyl)amino, C1 - C4-Alkylthio, Pyrrolidin-1-yl, Morpholino-, Piperidino-, Piperazinyl- oder 4-Methylpiperazin-1-yl-, gegebenenfalls substituiert durch C1 - C4-Alkyl, C2 - C6-Alkenyl, C1 - C4-Acyl, Oxo, Thioxo, Carboxy oder Phenyl, oder mit bis zu fünf voneinander unabhängigen Resten R⁶ (R⁶ ist wie eingangs definiert) substituiertes Aryloxy, Arylthio, Arylamino, Arylalkyloxy, Arylalkylthio, Aryalkylamino, Heteroaryloxy, Heteroarylthio, Heteroarylamino, Heteroarylalkyloxy, Heteroarylalkylthio, Heteroarylalkylamino, wobei der Alkylrest jeweils 1 bis 3 C-Atome enthalten kann, sein kann, zur Reaktion bringt.

Die obengenannte Methode A läuft vorzugsweise unter folgenden Bedingungen ab:

Der Substituent Z in der Formel III ist eine geeignete Abgangsgruppe, wie z.B. Chlor, Brom oder Jod, ein geeignetes Radikal der Schwefelsäure, ein aliphatischer oder aromatischer Sulfonsäureester oder ggf. halogeniertes Acyloxy.

Die Umsetzung wird zweckmäßigerweise in einem inerten Lösungsmittel durchgeführt. Geeignet sind z.B. aromatische Kohlenwasserstoffe wie Toluol oder Xylol, niedere Akohole wie Methanol, Ethanol oder 1-Butanol, Ether wie Tetrahydrofuran oder Glycoldimethylether, dipolar aprotische Lösungsmittel wie N,N-Dimethylformamid, N-Methyl-2-pyrrolidon, Acetonitril, Nitrobenzol, Dimethylsulfoxid oder Gemische dieser Lösungsmittel. Auch Zweiphasensysteme mit wäßrigen Lösungen von Basen in Gegenwart eines Phasentransferkatalysators, wie z.B. Benzyltriethylammoniumchlorid, sind möglich.
Die Anwesenheit einer geeigneten Base z.B. eines Alkali- oder Erdalkalimetallcarbonats oder -hydrogencarbonats wie Natriumcarbonat, Calciumcarbonat oder Natriumbicarbonat, eines Alkali- oder Erdalkalihydroxids wie Kaliumhydroxid oder Bariumhydroxid, eines Alkoholats wie Natriumethanolat oder Kalium-tert.-butylat, einer lithiumorganischen Verbindung wie Butyllithium oder Lithiumdiisopropylamin, eines Alkali- oder Erdalkalihydrids wie Natriumhydrid oder Calciumhydrid, ein Alkalifluorid wie Kaliumfluorid oder einer organischen Base wie Triethylamin oder Pyridin zum Auffangen der bei der Reaktion freiwerdenden Säure kann nützlich sein.

In manchen Fällen ist der Zusatz eines Jodsalzes, z.B. Kaliumjodid, angebracht. Die Reaktion wird gewöhnlich bei Temperaturen zwischen - 10 und 160 °C durchgeführt, vorzugsweise bei Raumtemperatur.

Für diese Umsetzung müssen etwaige nucleophile Substituenten wie z.B. Hydroxy-, Mercapto- oder Aminogruppen mit Ausnahme der 1- und/oder 4-Position in Verbindungen der Formel II oder III, vor Durchführung der Reaktion in geeigneter Weise derivatisiert oder mit wieder abspaltbaren gebräuchlichen Schutzgruppen wie z.B. Acetyl oder Benzyl versehen werden.

Für die Umsetzung wie zuvor unter B) beschrieben wird vorzugsweise als Schwefelungsreagenz 2,4- Bis(4-methoxyphenyl)-1,3-dithia-2,4-diphosphetan-2,4-disulfid (Lawessons Reagenz), Bis(tricyclohexylzinn)sulfid, Bis(tri-nbutylzinn)sulfid, Bis(triphenylzinn)sulfid, Bis(trimethylsilyl)sulfid oder Phosphorpentasulfid verwendet.
Die Reaktion wird zweckmäßigerweise in einem organischen Lösungsmittel oder einem Lösungsmittelgemisch, bei Raumtemperatur oder höher, bevorzugt bei der Siedetemperatur des Reaktionsgemisches und möglichst unter wasserfreien Bedingungen durchgeführt. Geeignet sind z.B. Schwefelkohlenstoff, Toluol, Xylol, Pyridin, 1,2-Dichlorethan. Bei Verwendung der erwähnten Zinn- oder Silylsulfide ist es angebracht, die Schwefelungsreaktion in Gegenwart einer Lewissäure wie Bortrichlorid durchzuführen.

In Gegenwart anderer Carbonylgruppen in einer Verbindung der Formel I, z.B. in einer Verbindung, wo X gleich Sauerstoff und einer oder mehrere Reste R¹ bis R⁶ gleich Acyl sind, ist das Carbonyl vor der Schwefelungsreaktion nach bekannten Methoden durch eine geeignete Schutzgruppe, z.B. durch Acetalisierung, zu schützen; anschließende Schutzgruppenabspaltung führt zur gewünschten Verbindung.

Für die oben unter C beschriebene Umsetzung ist der Substituent Z eine geeignete Abgangsgruppe, vorzugsweise Chlor, Brom oder Jod, ein geeignetes Radikal der Schwefelsäure, ein aliphatischer oder aromatischer Sulfonsäureester oder ggf. halogeniertes Acyloxy.

Die Reaktionsbedingungen für diese Umsetzung entsprechen denjenigen der Methode A.

Die unter D) beschriebene Cyclisierung findet in einem geeigneten Lösungsmittel statt wie Methanol, Ethanol, N,N-Dimethylformamid oder N-Methylpyrrolidon in Gegenwart einer Base; geeignet sind Alkali- oder Erdalkalimetallcarbonate oder -hydrogencarbonate wie Natriumcarbonat, Calciumcarbonat oder Natriumbicarbonat, Alkali- oder Erdalkalihydroxide wie Kaliumhydroxid oder Bariumhydroxid, Alkoholate wie Natriumethanolat oder Kalium- tert.-butylat, lithiumorganische Verbindungen wie Butyllithium oder Lithiumdiisopropylamin, Alkali- oder Erdalkalihydride wie Natriumhydrid oder Calciumhydrid oder eine organische Base wie Triethylamin oder Pyridin - letztere können auch als Lösungsmittel verwendet werden, oder organische oder anorganische Säuren wie Eisessig, Trifluoressigsäure, Salzsäure oder Phosphorsäure. Die Reaktion wird vorzugsweise bei Temperaturen zwischen 20 und 120 °C, besonders bevorzugt bei Raumtemperatur durchgeführt.

Die Verbindungen der allgemeinen Formel V, wobei R¹ bis R⁵ und Y wie unter 1) - 2) definiert, können aus Verbindungen der Formel VI, 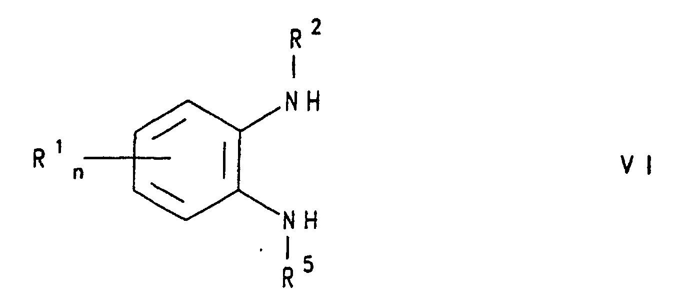 wobei R¹, R² und R⁵ wie unter 1) - 2) definiert, durch Alkylierung mit einer Verbindung der Formel VII, 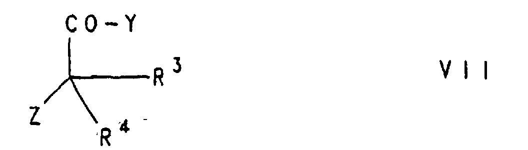 wobei R³, R⁴, Y wie unter 1) - 2) und Z wie unter A) definiert, erhalten werden. Die Reaktionsbedingungen für diese Alkylierung entsprechen den zur Methode A gegebenen.
Unter geeigneten Bedingungen findet dabei gleichzeitig der Ringschluß zum Dihydrochinoxalin der allgemeinen Formel I statt.

Verbindungen der allgemeinen Formel V, in denen R¹, R³ bis R⁵ und Y wie unter 1) - 2) definiert sind und R² Wasserstoff bedeutet können außerdem auch aus Verbindungen der Formel VIII, 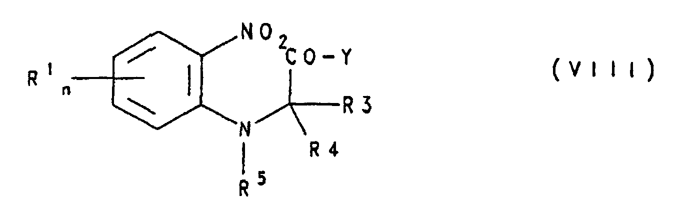 wobei R¹, R³ bis R⁵ und Y wie unter 1) - 2) definiert sind, hergestellt werden indem man die Nitrogruppe nach bekannten Verfahren zur Aminogruppe reduziert.
Unter geeigneten Bedingungen, z.B. bei Reduktion in Anwesenheit von Säure findet dabei gleichzeitig der Ringschluß zum Dihydrochinoxalin der allgemeinen Formel I statt.
Die Reduktion wird nach Standardmethoden (s. z.B. Methoden der Organischen Chemie (Houben-Weyl), E. Müller (Herausgeber); G. Thieme Verlag, Stuttgart, 1957; Bd. XI/1, S. 360 - 490) z.B. mit Zinn(II)chlorid in Eisessig, TiCl₃ in Salzsäure, oder durch katalytische Hydrierung durchgeführt, wobei die Wahl des Reagenzes durch die chemische Stabilität der verschiedenen Substituenten R¹, R³ bis R⁵ bestimmt wird; ist z.B. einer der Reste Alkenyl wird man die erste Methode wählen, um die Doppelbindung zu erhalten.

Die als Ausgangsmaterialien für die beschriebenen Synthesen benötigten Phenylendiamine der allgemeinen Formel VI sind literaturbekannt oder käuflich oder können nach literaturbekannten Methoden synthetisiert werden.

N-Ortho-Nitrophenylaminosäurederivate der generellen Formel VIII, wobei R¹ₙ und R³ bis R⁵ wie unter 1) - 2) definiert sind und Y gleich OR⁷ ist, worin R⁷ für Wasserstoff, C1 - C6-Alkyl, ggf. jeweils z.B. Halogen-substituiertes Phenyl, Benzyl oder 9- Fluorenylmethyl steht, bedeutet, können z.B. durch Aminierung von ortho-Halogennitroaromaten der allgemeinen Formel IX, 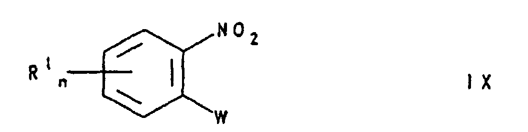 wobei R¹ wie unter 1) - 2) definiert ist und W Fluor, Chlor, Brom oder Jod bedeutet, mit Aminosäuren oder ihren Estern der allgemeinen Formel X, 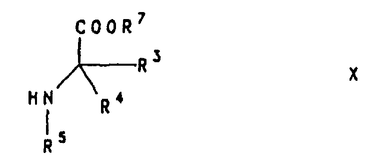 wobei R³, R⁴, R⁵ und R⁷ wie unter 1) - 2) definiert sind, erhalten werden.
Die Reaktion kann in Gegenwart einer anorganischen oder organischen Hilfsbase, wie z.B. Natrium- oder Kaliumcarbonat, Natriumhydroxid oder Triethylamin durchgeführt werden. Günstig ist die Verwendung eines inerten Lösungsmittels bei Temperaturen zwischen 0 und 150 °C, vorzugsweise bei Rückflußtemperatur. Geeignete Lösungsmittel sind offenkettige oder cyclische Ether, z.B. Tetrahydrofuran oder Glycoldimethylether, aromatische Kohlenwasserstoffe, z.B. Toluol oder Chlorbenzol, Alkohole, z.B. Ethanol, Isopropanol oder Glycolmonomethylether, dipolar aprotische Lösungsmittel, z.B. N,N-Dimethylformamid, N-Methyl-2-pyrrolidon oder 1,3- Dimethyl-tetrahydro-2(1H)-pyrimidinon.

Die N-ortho-Nitrophenylaminosäuren der Formel VIII mit Y gleich Hydroxy lassen sich falls gewünscht oder erforderlich nach wohlbekannten Standardmethoden in die Säurederivate der Formel VIII mit Y gleich Hydroxy, C1 - C4-Alkoxy, ggf. halogeniertes C1 - C4-Acyloxy, Chlor, Brom oder Jod umwandeln.

Ortho-Halogennitroaromaten der allgemeinen Formel IX und Aminosäuren der allgemeinen Formel X sind literaturbekannt und käuflich oder lassen sich nach literaturbekannten Methoden herstellen.

Die oben unter E) beschriebene Umsetzung erfolgt vorzugsweise durch katalytische Hydrierung (mit Wasserstoff) oder Hydrosilylierung (mit Alkylsilanen, z.B. Diphenylsilan) in Gegenwart eines Hydrierkatalysators, z.B. Raney-Nickel oder Palladium auf Kohle, bei einem Wasserstoffdruck von 1 bis 5 bar oder mittels eines Reduktionsmittels aus der Klasse der komplexen Metallhydride wie Natriumborhydrid oder Natriumcyanoborhydrid oder mit Metallen bzw. Metallsalzen und Säure wie z.B. Zink/Eisessig oder SnCl₂/HCl. Zweckmäßigerweise wird die Reaktion in einem inerten Lösungsmittel wie niederen Alkoholen, z.B. Methanol oder Isopropanol, Ethern wie Tetrahydrofuran oder Glycoldimethylether, dipolar aprotische Lösungsmitteln wie N,N-Dimethylformamid, aromatischen Kohlenwasserstoffen wie Toluol oder Xylol oder Gemischen dieser Lösungsmittel bei Temperaturen zwischen -20 und 100 °C, vorzugsweise bei Raumtemperatur durchgeführt.

Wird bei der beschriebenen Umsetzung ein chiraler Hydrierkatalysator, z.B. Di-µchloro-bis[(cycloocta-1c,5c- dien)-rhodium(I)]/(+) oder (-)-4,5-Bis-(diphenylphosphino- methyl)-2,2-dimethyl-1,3-dioxolan, oder ein chirales komplexes Metallhydrid, z.B. Natrium-tris-(N-benzyloxycarbonyl-L- prolinoyloxy)-borhydrid, verwendet, so lassen sich selektiv die einzelnen Enantiomeren erzeugen.

In Gegenwart von Substituenten in Verbindungen der Formel XI, welche unter den beschriebenen Bedingungen hydriert bzw. reduziert werden können, z.B. Oxo, ist die Verwendung eines Intermediates der Formel XI, mit Substituenten, die nicht angegriffen werden, welche aber zu der benötigten Gruppe derivatisiert werden können, z.B. Hydroxy, nötig. Die Substituenten können auch mit einer gebräuchlichen Schutzgruppe, z.B. einer Acetalschutzgruppe, versehen sein, die nach der oben beschriebenen Umsetzung wieder entfernt werden kann.

Chinoxalinone der allgemeinen Formel XI mit R¹ bis R³ wie unter 1) -2) definiert können nach bekannten Verfahren durch Kondensation eines Phenylendiamins der Formel VI, wobei R¹ und R² wie unter 1) - 2) definiert sind und R⁵ gleich Wasserstoff ist, mit einer alpha-Ketocarbonsäure der allgemeinen Formel XII,

R³-CO-COOH (XII)

wobei R³ wie unter 1) - 2) definiert ist, erhalten werden.
Zweckmäßigerweise wird die Reaktion in einem inerten Lösungsmittel in einem Temperaturbereich zwischen 0 und 150 °C durchgeführt; geeignete Lösungsmittel sind z.B. Alkohole, z.B. Ethanol oder Isopropanol, offenkettige oder cyclische Ether, z.B. Glycoldimethylether oder Tetrahydrofuran oder dipolar aprotische Lösungsmittel, z.B. N,N-Dimethylformamid oder Acetonitril.

Die oben unter F) beschriebene Umsetzung wird zweckmäßigerweise in einem Zweiphasensystem, bestehend aus einem organischen, nicht wassermischbaren Lösungsmittel oder Lösungsmittelgemisch, bestehend aus z.B. halogenierten Kohlenwasserstoffen, z.B. Dichlormethan oder 1,2-Dichlorethan oder aromatischen Kohlenwasserstoffen, z.B. Toluol oder Xylol und einer konzentrierten wäßrigen Lösung eines Alkali- oder Erdalkalimetallhydroxids, z.B. Natrium- oder Bariumhydroxid durchgeführt. Vorteilhaft ist die Anwesenheit eines Phasentransferkatalysators, wie z.B. Benzyltriethylammoniumchlorid oder Tetrabutylammoniumbromid.

Die Reaktion wird gewöhnlich bei Temperaturen zwischen 0 und 50 °C durchgeführt, vorzugsweise bei Raumtemperatur.

Substituenten in Verbindungen der Formeln VI und XIII, bzw. XIV die unter den Reaktionsbedingungen nicht stabil sind, müssen durch solche ersetzt werden, die zu der benötigten Gruppe derivatisiert werden können. Die Substituenten können auch mit einer gebräuchlichen Schutzgruppe versehen sein, die nach der oben beschriebenen Umsetzung wieder entfernt werden kann.

Bei der oben unter G) beschriebenen Reaktion ist Z in Formel XV eine geeignete Abgangsgruppe, wie z.B. Chlor, Brom oder Jod, ein geeignetes Radikal der Schwefelsäure, ein aliphatischer oder aromatischer Sulfonsäureester oder ggf. halogeniertes Acyloxy.

Die Reaktionsbedingungen für diese Umsetzung entsprechen denjenigen der Methode A.

Die unter H) beschriebene Reaktion findet vorzugsweise durch katalytische Hydrierung (mit Wasserstoff) in Gegenwart eines Hydrierkatalysators, z.B. Palladium auf Kohle, bei einem Wasserstoffdruck von 1 bis 5 bar, oder mittels eines Reduktionsmittels aus der Klasse der komplexen Metallhydride, wie Natriumborhydrid, Natriumtriacetoxyborhydrid oder Natriumcyanoborhydrid statt.

Zweckmäßigerweise wird die Umsetzung in einem inerten Lösungsmittel, wie niederen Alkoholen, z.B. Methanol oder Isopropanol, Ethern, z.B. Tetrahydrofuran oder Glycoldimethylether, halogenierten Kohlenwasserstoffen, z.B. Dichlormethan oder Dichlorethan, bei Temperaturen zwischen -20 und 100 °C, vorzugsweise bei Raumtemperatur durchgeführt. Die Anwesenheit einer Säure, wie z.B. Essigsäure oder Trifluoressigsäure, oder einer Lewissäure, wie z.B. Titantetrachlorid, ist vorteilhaft. In Gegenwart von Substituenten in Verbindungen der Formeln I und XVI, welche unter den beschriebenen Bedingungen hydriert bzw. reduziert werden können, z.B. Oxo, ist die Verwendung eines Intermediates der Formeln I und XVI, mit Substituenten, die nicht angegriffen werden, welche aber zu der benötigten Gruppe derivatisiert werden könne, z.B. Hydroxy, nötig. Säurelabile Gruppen, wie z.B. Acetale oder unter den Reaktionsbedingungen reagierende Gruppen, wie z.B. primäre Amine, sind ebenfalls zu vermeiden bzw. mit einer gebräuchlichen Schutzgruppe zu versehen.

Die unter I) beschriebene Umsetzung wird zweckmäßigerweise in einem inerten Lösungsmittel durchgeführt. Geeignet sind z.B. aromatische Kohlenwasserstoffe wie Toluol oder Xylol, niedere Alkohole wie Methanol, Ethanol oder 1-Butanol, Ether wie Tetrahydrofuran oder Glycoldimethylether, dipolar aprotische Lösungsmittel wie N,N-Dimethylformamid, N-Methyl-2-pyrrolidon, Acetonitril, Nitrobenzol, Dimethylsulfoxid oder Gemische dieser Lösungsmittel. Auch Zweiphasensysteme mit wäßrigen Lösungen von Basen in Gegenwart eines Phasentransferkatalysators, wie z.B. Benzyltriethylammoniumchlorid, sind möglich.
Die Anwesenheit einer geeigneten Base z.B. eines Alkali- oder Erdalkalihydroxids wie Kaliumhydroxid oder Bariumhydroxid, eines Alkoholats wie Natriumethanolat oder Kalium-tert.-butylat, einer lithiumorganischen Verbindung wie Butyllithium oder Lithiumdiisopropylamid, eines Alkali- oder Erdalkalihydrids wie Natriumhydrid oder Calciumhydrid, ein Alkalifluorid wie Kaliumfluorid oder einer organischen Base wie Triethylamin oder Pyridin kann nützlich sein. Die Reaktion wird gewöhnlich bei Temperaturen zwischen -10 und 160 °C durchgeführt, vorzugsweise bei Raumtemperatur.

Für diese Umsetzung sind etwaige nucleophile Substituenten wie z.B. Hydroxy-, Mercapto- oder Aminogruppen in den Verbindungen XVII und XVIII, die nicht an der Reaktion beteiligt sind, in geeigneter Weise zu derivatisieren oder mit wieder abspaltbaren gebräuchlichen Schutzgruppen wie z.B. Acetyl oder Benzyl zu versehen.

Die für die genannte Reaktion benötigten Verbindungen XVII, wobei für R¹, R², R³ und R⁴ die unter 1) - 2) beschriebenen Definitionen gelten, n gleich 0, 1, 2 oder 3 ist, X gleich Sauerstoff und U eine geeignete Abgangsgruppe, Halogen, wie z.B. Chlor, Brom, Jod, ein halogeniertes aliphatischen oder aromatisches Alkoholat wie z.B. 2,2,2-Trichlorethoxy, Chlorphenoxy oder ein über Stickstoff verknüpfter Heterocyclus wie z.B. Imidazolyl, Triazolyl, Benztriazolyl ist, werden hergestellt, indem man eine Verbindung der Formel I, wobei R⁵ Wasserstoff und X Sauerstoff sind und für R¹, R², R³ und R⁴ die unter 1) - 2) beschriebenen Definitionen gelten mit einem geeigneten Kohlensäurederivat, z.B. Phosgen, Diphosgen, Triphosgen, Chlorameisensäuretrichlorethylester oder Carbonyldiimidazol beziehungsweise mit einem geeigneten Halogencarbonsäurehalogenid, z.B. Bromacetylchlorid umsetzt.

Die Umsetzung wird zweckmäßigerweise in einem inerten Lösungsmittel durchgeführt. Geeignet sind z.B. aromatische Kohlenwasserstoffe wie Toluol oder Xylol, Ether wie Tetrahydrofuran oder Glycoldimethylether oder halogenierte Kohlenwasserstoffe wie Dichlormethan oder Dichlorethan.

Die Anwesenheit einer geeigneten Base z.B. eines Alkali- oder Erdalkalihydroxids wie Kaliumhydroxid oder Bariumhydroxid oder einer ogranischen Base wie Triethylamin oder Pyridin kann nützlich sein.

Die Reaktion wird gewöhnlich bei Temperaturen zwischen -30 und 160 °C durchgeführt, vorzugsweise bei Raumtemperatur.

Weiterhin gehören zum Gegenstand der vorliegenden Erfindung die Verbindungen wie unter 1) bis 2) beschrieben in Kombination mit einem Nukleosid als Arzneimittel, vorzugsweise zur Behandlung von Viruserkrankungen.

Als Indikationsgebiete in der Humanmedizin können beispielsweise genannt werden:
1) Die Behandlung und Prophylaxe von menschlichen Retrovirusinfektionen
2) Für die Behandlung oder die Prophylaxe von HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenophathie-Syndrom) sowie der durch dieses Virus verursachten Immunschwäche und Encephalopathie.
3) Für die Behandlung oder die Prophylaxe einer HTLV-I oder HTLV-II Infektion
4) Für die Behandlung oder die Prophylaxe des AIDS-carrier Zustandes (AIS-Überträger-Zustand)

Als Indikationen in der Tiermedizin können beispielsweise genannt werden: Infektionen mit
a) Maedivisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegerziekte Virus (bei Schafen)
e) infektiösem Virus der Anämie (des Pferdes)
f) Infektionen verursacht durch das Katzenleukemievirus
g) Infektionen verursacht durch das Virus der Katzen-Immundefizienz Virus.

Eine besondere Bedeutung besitzen die erfindungsgemäßen Kombinationen zur Bekämpfung von HIV und HIV verursachten Erkrankungen.

Des weiteren betrifft die vorliegende Erfindung Arzneimittel mit einem Gehalt an mindestens einer erfindungsgemäßen Kombination von Verbindungen, sowie die Verwendung der genannten Verbindungen zur Herstellung von Arzneimitteln, vorzugsweise zur Behandlung von Viruserkrankungen, insbesondere zur Behandlung von Krankheiten, hervorgerufen durch das HIV.

Weiterhin gehört zum Gegenstand der vorliegenden Erfindung die Verwendung von Kombinationen der obengenannten Verbindungen zur Herstellung von Arzneimitteln zur Behandlung von Viruserkrankungen.

Die erfindungsgemäßen Arzneimittel können enteral (oral), parenteral (intravenös), rektal, subcutan, intramuskulär oder lokal (topisch) angewendet werden.

Sie können in Form von Lösungen, Pulvern (Tabletten, Kapseln einschließlich Microkapseln), Salben (Cremes oder Gele) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Als zweckmäßige Dosierung werden 0.1 - 10, vorzugsweise 0.2 - 8 mg/kg Körpergewicht ein oder mehrmals täglich verabreicht. Die verwendeten Dosierungseinheiten richten sich zweckmäßigerweise nach der jeweiligen Pharmakokinetik der verwendeten Substanz bzw. der verwendeten galenischen Zubereitung.

Die verwendete Dosierungseinheit der erfindungsgemäßen Verbindungen beträgt z.B. 1 - 1500 mg, vorzugsweise 50 - 500 mg.

Die Zusammensetzung der erfindungsgemäßen Kombinationspräparate kann weite Bereiche überstreichen und mittels Methoden des Standes der Technik optimiert werden. Eine geeignete Zusammensetzung liegt z.B. zwischen 1:1000 und 1000:1, vorzugsweise zwischen 1:100 und 100:1.

### Testbeschreibung

### HIV-Infektion in Zellkultur

Der HIV-Test wurde mit Modifikationen nach der Methode von Pauwels et al. (vgl. Journal of Virological Methods 20, (1988), 309-321) durchgeführt.
Normale menschliche Blutlymphozyten (PBL's) wurden über Ficoll-Hypaque angereichert und im RPMI 1640, 20 % fötales Kälberserum mit Phythaemagglutinin (90 µg/ml) und Interleukin-2 (40 U/ml) stimuliert. Zur Infektion mit dem infektiösen HIV wurden PBL's pelletiert, und das Zellpellet wurde anschließend in 1 ml HIV-Viruslösung zur Adsorption suspendiert und 1 Stunde bei 37 °C inkubiert.
Die Virusadsorptionslösung wurde zentrifugiert und das infizierte Zellpellet in Wachstumsmedium aufgenommen, so daß 1 x 10⁵ Zellen pro ml eingestellt waren. Die derart infizierten Zellen wurden zu 1 x 10⁴ Zellen/Napf in die Näpfe von 96er Mikrotiterplatten pipettiert.
Alternativ wurden anstelle der PBL's H9 Zellen für die antiviralen Tests eingesetzt.
Die Testung der kombinatorischen Wirkung der Prüfverbindungen wurde mittels Schachbrett-Titration (Chequerboardtitration) durchgeführt.
die erste vertikale Reihe der Mikrotiterplatte enthielt nur Wachstumsmedium und Zellen, die nicht infiziert, aber ansonsten genau wie oben beschrieben, behandelt worden waren (Zellkontrolle). Die zweite vertikale Reihe der Mikortitreplatte enthielt nur HIV-infizierte Zellen (Viruskontrolle) in Wachstumsmedium. Die übrigen Näpfe enthielten die erfindungsgemäßen Verbindungen - alleine oder in entsprechenden Kombinationen - in unterschiedlichen Konzentrationen, ausgehend von den Näpfen der 3. vertikalen Reihe der Mikrotiterplatte, von der die Prüfsubstanzen in 2er Schritten weiter verdünnt wurden (100 µl Volumen pro Napf). Für die Kombination wurden Verdünnungen der 2. Substanz auf einer separaten 96er Mikrotiterplatte hergestellt und anschließend auf die vorbereitete erste Platte zupipettiert. Dazu wurden jeweils 100 µl der vorbereiteten HIV-infizierten Zellen gegeben (s.o.).

Damit waren Testkonzentrationen im Bereich ca. 10 - 50fach ober- und unterhalb der IC-50 Konzentration abgedeckt.
Die Testansätze wurden solange bei 37 °C inkubiert, bis in der unbehandelten Viruskontrolle die für das HIV typische Syncytienbildung an der Wirtszelle (zwischen Tag 3 und Tag 6 nach Infektion) mikroskopisch nachweisbar wurde.
In der unbehandelten Viruskontrolle resultierten unter diesen Testbedingungen etwa 20 - 50 Syncytien, während die unbehandelte Zellkontrolle keine Syncytien aufwies.
Die Überstände der 96er Platte wurden dann geerntet und in einem HIVspezifischen ELISA-Test auf HIV-spezifisches Antigen untersucht (Vironostika HIV Antigen, Organon Teknika).
Die Hemmwerte wurden entsprechend der Cut-Off-Werte aus entsprechenden Zell- bzw. Viruskontrollen bzw. interner Testkontrollen in Prozent (%)-Hemmwerte umgerechnet und die IC-50 Werte wurden als die Konzentration der behandelten und infizierten Zellen ermittelt, bei der 50 % des Virus-spezifischen Antigens durch die Behandlung mit den Verbindungen unterdrückt waren. Zur Analyse der syntergistischen Wirksamkeit der Verbindungen wurden die Differenzwerte von errechneten und gemessenen Hemmwerten jeder Kombination ermittelt (Prichard, M. N. et al., Antimicrob. Agents Chemoth. (1993), 37, 540-545).
Differenzwerte > Null bedeuten, daß eine synergistische Wirksamkeit beschrieben werden kann. Beispielsweise wurden mit der Verbindung der Formel A folgende Ergebnisse erzielt:
(Tab. 1a und Tab 1b)

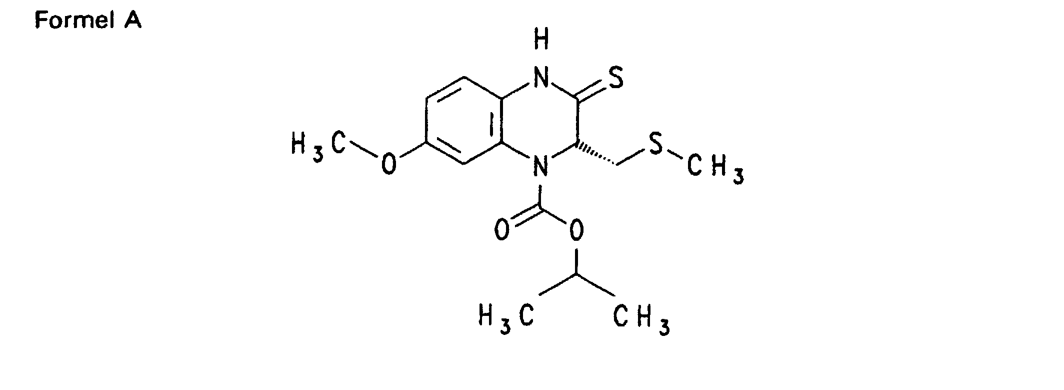

### Chemische Bezeichnung:

S-4-lsopropoxycarbonyl-6-methoxy-3-(methylthio-methyl)-3,4-dihydrochinoxalin-2(1H)-thion, Schmelzpunkt 101 °C.

**Tab. 1a**

| Differenztabelle zur berechneten und erwarteten Wirkung von AZT mit Beispiel der Formet (A) | | | | | | |
|---|---|---|---|---|---|---|
| Verb. der Formel (A) nM | 50 | 25 | 12 | 6 | 3 | 1 |
| AZT nM | | | | | | |
| 50 | 0 | 0 | 0 | 0 | 0 | 0 |
| 25 | 0 | 0 | 0 | 0 | -17 | -4 |
| 12 | 0 | 0 | 4 | 18 | 23 | 23 |
| 6 | 0 | 0 | 7 | 29 | 23 | -17 |
| 3 | 0 | 0 | 10 | 33 | 10 | -13 |
| 1,5 | 0 | 0 | -2 | 40 | 17 | 3 |
| 0,7 | -17 | -20 | -5 | 7 | 17 | 3 |

**Tab. 1 b**

| Differenztabelle zur berechneten und experimentell ermittelten Hemmdaten von der Verbindung Beispiel Formel (A) und 3'-TC | | | | | | |
|---|---|---|---|---|---|---|
| Verb. der Formel (A) nM | 50 | 25 | 12 | 6 | 3 | 1,5 |
| 3TC nM | | | | | | |
| 50 | 0 | 0 | 0 | 2 | -7 | -60 |
| 25 | 0 | 0 | 0 | 40 | 70 | 30 |
| 12 | 0 | 0 | 0 | 47 | 60 | 20 |
| 6 | 0 | 0 | 0 | 30 | 40 | 22 |
| 3 | 0 | 0 | 0 | 34 | 17 | 0 |

Im Konzentrationsfenster 0,7 - 12 nM AZT mit 1 - 6 nM des Chinoxalins resultiert eine synergistische Wirksamkeit der Kombinationen. Ebenfalls synergistisch wirksam ist das Chinoxalin und 3TC im Konzentrationsfenster 1,5 - 6 nM bzw. 3 - 25 nM. Zur Messung einer synergistischen Toxizität der Verbindungen wurden Substanzkonzentrationen um den Tox-50 Wert der Einzelverbindungen geprüft und in gleicher Weise ausgewertet. Keine der untersuchten Kombinationen wies eine synergistische Toxizität auf.

Durch die nachfolgenden Beispiele sowie durch den Inhalt der Patentansprüche wird die vorliegende Erfindung näher erläutert. Die folgenden mit * gekennzeichneden Beispiele sind nur illustrativ, aber nicht vom Gegenstand der Ansprüche umfasst.

### * Beispiel I

### (3S)-6-Chlor-3-methyl-3,4-dihydro-chinoxalin-2(1 H)-on

### A) (S)-N-(3-Chlor-6-nitrophenyl)-alanin

2,4-Dichlornitrobenzol (21.0 g, 0.109 mol) und 23.0 g (0.258 mol) L-Alanin wurden in 400 ml 2-Methoxyethanol unter Zusatz von 120 ml 2N Natronlauge für 48 h unter Rückfluß erhitzt. Anschließend wurde im Vakuum eingeengt und der Rückstand in wäßriger Natriumhydrogencarbonatlösung aufgenommen. Es wurde dreimal mit Essigsäureethylester extrahiert, dann mit 6N Salzsäure angesäuert und das gelbe Produkt mit Essigsäureethylester extrahiert. Die organische Phase wurde einmal mit gesättigter wäßriger Natriumchloridlösung gewaschen, getrocknet (Magnesiumsulfat) und das Lösungsmittel unter vermindertem Druck entfernt. Zurück blieben 14.7 g (55 %) gelber Feststoff vom Schmelzpunkt 167 - 169 °C (nach Kristallisation aus Essigsäureethylester).
¹H-NMR (270 MHz, d₆-DMSO): δ = 1.47 (d, J = 7 Hz, 3 H), 4.57 (quintett, J = 7 Hz, 1 H), 6.77 (dd, J = 9, 2 Hz, 1 H), 7.11 (d, J = 2 Hz, 1 H), 8.12 (d, J = 9 Hz, 2 H), 8.41 (br. d, J = 7 Hz, 1 H), 13.2 ppm (br., 1 H).
MS: (M + H)⁺ = 245

### B) (3S)-6-Chlor-3-methyl-3,4-dihydro-chinoxalin-2(1H)-on

Das Produkt des Beispiels IA (14.0 g, 0.057 mol) wurde in 400 ml Methanol gelöst und unter Raney-Nickel-Katalyse mit 1 atm Wasserstoff bei Raumtemperatur hydriert. Nach Aufnahme der berechneten Menge Wasserstoff wurde der Katalysator abgesaugt und die Reaktionslösung im Vakuum eingeengt. Der Rückstand wurde durch Chromatographie an Kieselgel mit Essigsäureethylester / Heptan = 1 : 2 und 1 : 1 als Elutionsmittel gereinigt. Die Ausbeute betrug 6.0 g (53 %) eines bräunlichen Feststoffs mit Schmelzpunkt 122 - 123 °C (nach Umkristallisation aus Isopropanol / Heptan).
¹H-NMR (60 MHz, d₆-DMSO): δ = 1.23 (d, J = 11 Hz, 3 H), 3.81 (dq, J = 11, 4 Hz, 1 H), 6.27 (br., 1 H), 6.3 - 6.9 (m, 3 H), 10.3 ppm (br., 1 H).
MS: (M + H)⁺ = 197
[*α*]_{D}²³ = + 77.3° (c = 1, MeOH)

### C) (3R)-6-Chlor-3-methyl-3,4-dihydro-chinoxalin-2(1H)-on

Die Verbindung wurde nach den unter Beispiel IA und IB beschriebenen Methoden ausgehend von D-Alanin hergestellt. Schmp. 123 - 124 °C (nach Umkristallisation aus Isopropanol / Heptan)
Die NMR-Daten stimmten mit denen der in Beispiel IB beschriebenen Verbindung überein.
[α]_{D}²³ = - 81.0° (c = 1, MeOH)

### D) (3RS)-6-Chlor-3-methyl-3,4-dihydro-chinoxalin-2(1H)-on

Die Verbindung wurde nach den in Beispiel IA und IB beschriebenen Methoden ausgehend von D,L-Alanin hergestellt. Schmp. 110 °C (nach Umkristallisation aus Isopropanol / Heptan)
Die NMR-Daten stimmten mit denen der in Beispiel IB beschriebenen Verbindung überein.

Die folgenden Verbindungen der Formel I wurden unter Verwendung der entsprechenden Halogenaromaten und Aminosäurederivate in analoger Weise synthetisiert:

### * Beispiel II

### (3S)-3-Benzyl-7-chlor-3,4-dihydrochinoxalin-2(1H)-on

### A) (S)-N-(4-Chlor-2-nitrophenyl)-phenylalanin

L-Phenylalanin (8,3 g, 0,05 mol) und 4,8 g (0,025 mol) 2,5-Dichlornitrobenzol wurden in 40 ml wasserfreiem Dimethylsulfoxid (DMSO) gelöst und unter Rühren in einer Argonatmosphäre auf 80 °C erwärmt. Kalium-tert.-butylat (4,2 g, 0,025 mol), gelöst in 30 ml DMSO wurden innerhalb von 40 min zugetropft. Man rührte 3 h bei 80 bis 90 °C weiter, ließ abkühlen, saugte unumgesetztes Phenylalanin ab und wusch mit Wasser nach. Die gesammelten alkalischen Filtrate wurden zweimal mit Diethylether extrahiert, zur Entfernung von nicht umgesetzten Dichlornitrobenzol. Anschließend wurde mit Eisessig angesäuert, mehrmals mit Essigsäureethylester extrahiert, über Magnesiumsulfat getrocknet und eingedampft.

Das Produkt fiel als rotes Öl (6,7 g, 84 %) an, das ohne Reinigung weiter umgesetzt wurde.

### B) (3S)-3-Benzyl-7-chlor-3,4-dihydrochinoxalin-2(1H)-on

Das Produkt aus Beispiel IIA (12 g) wurde in 300 ml wasserfreiem Methanol gelöst und unter Palladium/Kohle-Katalyse mit 1 atm Wasserstoff bei Raumtemperatur hydriert. Nach beendeter Reaktion wurde abgesaugt, eingeengt und an Kieselgel mit Diisopropylether als Elutionsmittel chromatographiert. Dabei erhielt man 1,32 g des gewünschten Produkts, das aus Isopropanol kristallisierte, Schmp. 185 °.
¹H-NMR (270 MHz, d₆-DMSO): δ = 2.9 (m, 2 H), 4.08 (m, 1 H), 6.09 (d, 1 H), 6.7 (m, 2 H), 6.78 (m, 1 H), 7.2 (m, 5 H), 10.34 ppm (br. s, 1 H).
MS: (M + H)⁺ = 273, (M - 92)⁺ = 181.

Die Verbindungen der Tabelle 2 wurden wie in den vorausgehenden Beispielen beschrieben hergestellt.

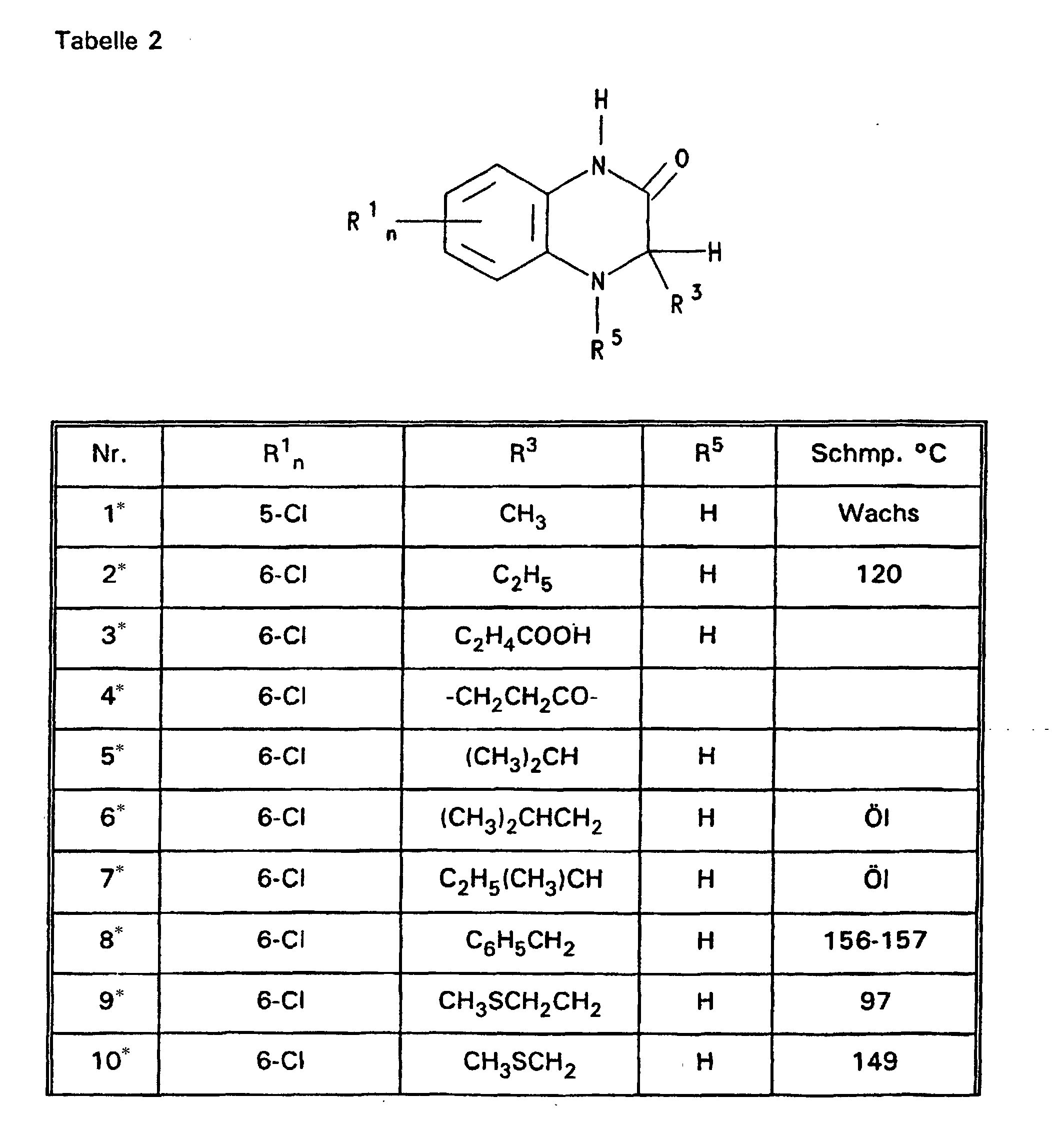

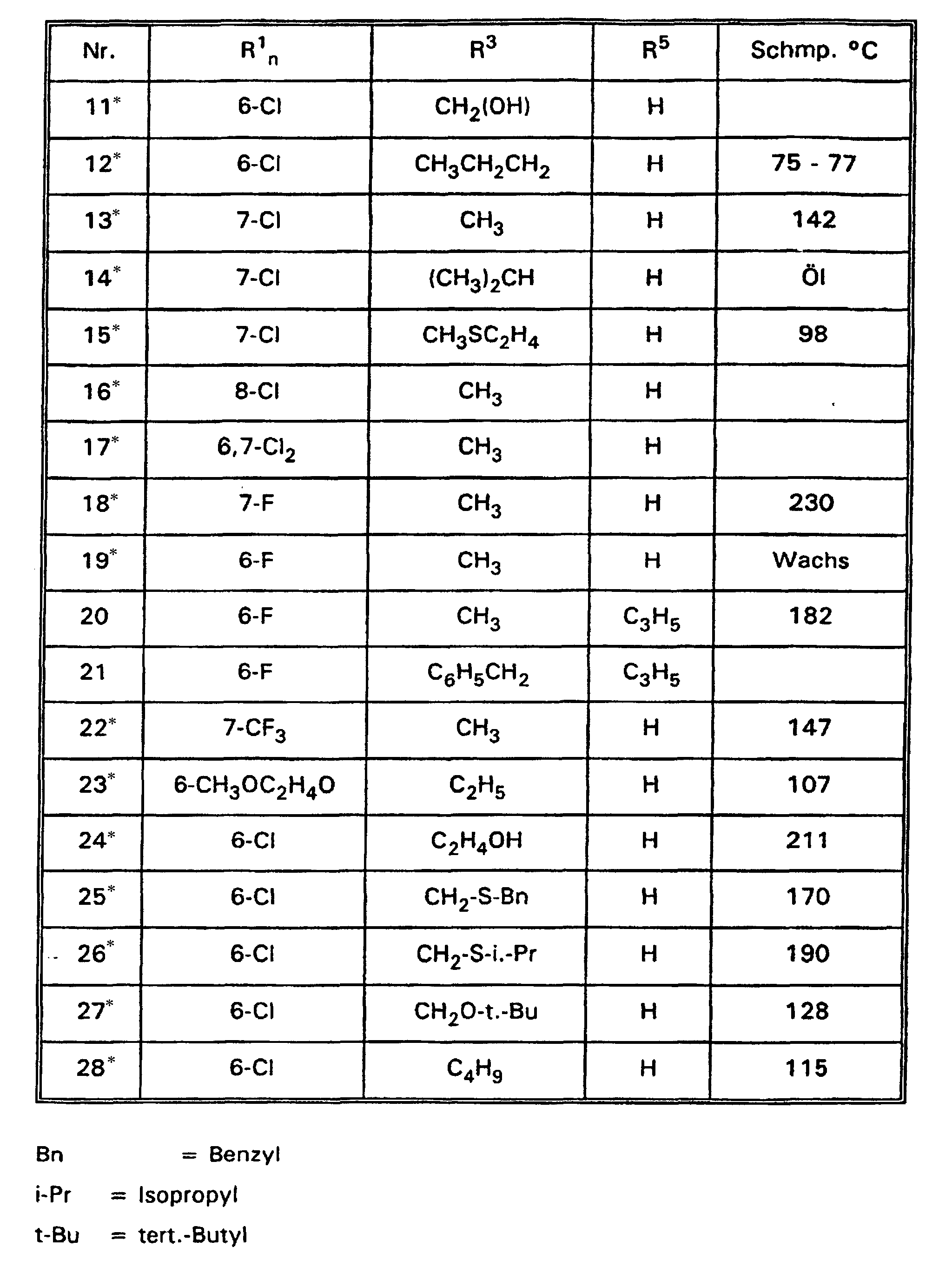

### Beispiel III

### (3S)-4-N-(Benzyloxycarbonyl)-6-chlor-3-methyl-3,4-dihydro-chinoxalin-2(1H)-on

Die Verbindung des Beispiels IB (1.0 g, 5.1 mmol) wurde in 20 ml Dichlormethan gelöst. Es wurden 10 ml 2N wäßrige Natriumhydrogencarbonatlösung zugegeben und unter Eiskühlung und kräftigem Rühren 0.9 ml (90 %; 5.7 mmol) Chlorameisensäurebenzylester zugetropft. Das Zweiphasensystem wurde anschließend bei Raumtemperatur für 60 h gerührt. Nach 30 h wurden nochmals 0.2 ml (1.3 mmol) Chlorameisensäurebenzylester zugegeben. Nach vollständiger Umsetzung wurden die Phasen getrennt, die organische Phase einmal mit Wasser gewaschen, getrocknet (Magnesiumsulfat) und im Vakuum das Lösungsmittel entfernt. Das Produkt wurde durch Chromatographie an Kieselgel mit Methyl-tert.-butylether / Heptan = 1 : 1 als Elutionsmittel gereinigt. Man erhielt 1.65 g (98 %) weißes schaumartiges Produkt.
¹H-NMR (270 MHz, d₆-DMSO): δ = 1.15 (d, J = 7 Hz, 3 H), 4.85 (q, J = 7 Hz, 1 H), 5.20 (d, J = 12 Hz, 1 H), 5.27 (d, J = 12 Hz, 1 H), 6.97 (d, J = 7 Hz, 1 H), 7.19 (dd, J = 8, 2 Hz, 1 H), 7.3 - 7.45 (m, 5 H), 7.67 (d, J = 2 Hz, 1 H), 10.81 ppm (br. s, 1 H).
MS: (M + H)⁺ = 381

### Beispiel IV

### (3S)-4-N-(Benzyloxycarbonyl)-6-chlor-3-methyl-8-nitro-3,4- dihydro-chinoxalin-2(1H)-on

Die Verbindung des Beispiels III (1.5 g, 4.5 mmol) wurde in Eisessig (15 ml) nitriert. Insgesamt 5 ml (124.3 mmol) rauchende Salpetersäure wurden innerhalb von 4 h bei 0 °C bis Raumtemperatur zugetropft. Anschließend wurde auf 100 ml Eiswasser gegossen und das als gelber Feststoff anfallende Produkt abgesaugt, gründlich mit Wasser gewaschen und getrocknet. Schmp. 85 °C (subl.)
¹H-NMR (270 MHz, d₆-DMSO): δ = 1.22 (d, J = 8 Hz, 3 H), 4.89 (q, J = 8 Hz, 1 H), 5.24 (d, J = 12 Hz, 1 H), 5.31 (d, J = 12 Hz, 1 H), 7.35 - 7.5 (m, 5 H), 7.69 (s, 1 H), 8.00 (s, 1 H), 11.11 ppm (br. s, 1 H).
MS: (M + H)⁺ = 376

### Beispiel V

### (3S)-8-Amino-4-N-(benzyloxycarbonyl)-6-chlor-3-methyl-3,4- dihydro-chinoxalin-2(1H)-on

Die Verbindung des Beispiels IV (1.5 g, 4.0 mmol) wurde in 150 ml Methanol gelöst und unter Raney-Nickel-Katalyse mit 1 atm Wasserstoff bei Raumtemperatur hydriert. Nach Aufnahme der berechneten Menge Wasserstoff wurde vom Katalysator abgesaugt und im Vakuum eingeent. Das Produkt wurde durch Chromatographie an Kieselgel mit Essigsäureethylester / Heptan = 2 : 1 als Elutionsmittel gereinigt. Die Ausbeute betrug 0.68 g (49 %) bräunlicher Feststoff vom Schmelzpunkt 152 - 154 °C.
¹H-NMR (270 MHz, d₆-DMSO): δ = 1.11 (d, J = 8 Hz, 3 H), 4.79 (q, J = 8 Hz, 1 H), 5.15 (d, J = 12 Hz, 1 H), 5.24 (d, J = 12 Hz, 1 H), 5.38 (br. s, 2 H), 6.42 (s, 1 H), 7.3 - 7.4 (m, 6 H), 10.59 ppm (br. s, 1 H).
MS: (M + H)⁺ = 346

### Beispiel VI

### A) (3S)-6-Chlor-3-methyl-4-N-(3-methyl-2-buten-1-yl)-3,4- dihydro-chinoxalin-2(1H)-on

Die Verbindung des Beispiels IB (1.0 g, 5.0 mmol) wurde in 20 ml Acetonitril gelöst und in Gegenwart von 1.0 g (7.0 mmol) gepulvertem Kaliumcarbonat mit 3-Methyl-2-buten-1-ylbromid (90 %-ig; 0.92 ml, 7.0 mmol) bei Raumtemperatur alkyliert. Nach 7 h war die Reaktion beendet. Es wurde abgesaugt, im Vakuum eingeent und das Produkt durch Chromatographie an Kieselgel mit Essigsäureethylester / Heptan = 1 : 2 als Elutionsmittel gereinigt. Die Ausbeute betrug 0.97 g (72 %) bräunlicher Feststoff vom Schmelzpunkt 117 - 118 °C (nach Kristallisation aus Methyl-tert.-butylether / Heptan).
¹H-NMR (270 MHz, d₆-DMSO): δ = 1.02 (d J = 8 Hz, 3 H), 1.74 (s, 6 H), 3.69 (dd, J = 14, 8 Hz, 1 H), 3.85 - 3.9 (m, 2 H), 5.19 (m, 1 H), 6.65 - 6.8 (m, 3 H), 10.47 ppm (br. s, 1 H).
MS: (M + H)⁺ = 265
[α]_{D}²³ = + 168.0° (c = 1, MeOH)

### B) (3R)-6-Chlor-3-methyl-4-N-(3-methyl-2-buten-1-yl)-3,4- dihydro-chinoxalin-2(1H)-on

Die Verbindung wurde nach der in Beispiel VIA beschriebenen Methode ausgehend von der Verbindung des Beispiels IC hergestellt. Schmp. 115 - 117 °C (nach Umkristallisation aus Isopropanol / Diethylether)
Die NMR-Daten stimmten mit denen der in Beispiel VIA beschriebenen Verbindung überein.
[α]_{D}²³ = - 172° (c = 1, MeOH)

### C) (3RS)-6-Chlor-3-methyl-4-N-(3-methyl-2-buten-1-yl)-3,4- dihydro-chinoxalin-2(1 H)-on

Die Verbindung wurde nach der in Beispiel VIA beschriebenen Methode ausgehend von der Verbindung des Beispiels ID hergestellt. Schmp. 148 - 149 °C (nach Umkristallisation aus Isopropanol / Diethylether)
Die NMR-Daten stimmten mit denen der in Beispiel VIA beschriebenen Verbindung überein.

### Beispiel VII

### (3S)-6-Chlor-3-methyl-4-N-(2-buten-1-yl)-3,4-dihydro-chinoxalin- 2(1H)-on

Die Substanz wurde analog zu der in Beispiel VIA beschriebenen Verbindung hergestellt, jedoch mit 2-Buten-1-ylbromid als Alkylierungsmittel. Schmp. 87 - 88 °C (nach Kristallisation aus Diethylether / Heptan)
¹H-NMR (270 MHz, d₆-DMSO): δ = 1.01 (d, J = 8 Hz, 3 H), 1.70 (dd, J = 8, 1 Hz, 3 H), 3.63 (dd, J = 16, 6 Hz, 1 H), 3.85 - 4.0 (m, 2 H), 5.47 (m, 1 H), 5.75 (m, 1 H), 6.65 - 6.8 (m, 3 H), 10.48 ppm (br. s, 1 H).
MS: (M + H)⁺ = 251

### Beispiel VIII

### 4-N-(Isopropenyloxycarbonyl)-3,3,7-trimethyl-3,4-dihydrochinoxalin-2(1H)-on

3,3,7-Trimethyl-3,4-dihydrochinoxalin-2(1H)-on (0,4 g, 2,1 mmol) wurden in 10 ml wasserfreiem Pyridin gelöst und bei Raumtemperatur unter Rühren mit 0,24 ml (2,2 mmol) Chlorameisensäureisopropenylester versetzt. Man ließ 6 h bei Raumtemperatur rühren, versetzte mit Wasser, saugte die entstehende Fällung ab, wusch mit Wasser nach und trocknete. Es wurden 0,4 g (69 %) farblose Kristalle vom Schmelzpunkt 185 °C erhalten.
¹H-NMR (270 MHz, d₆-DMSO): δ = 1.5 (s, 6 H), 1.9 (s, 3 H), 2.25 (s, 3 H), 4.7 (m, 2 H), 6.7 - 6.9 (m, 2 H), 7.15 (d, J = 8 Hz, 1 H), 10.6 ppm (br. s, 1 H). MS: M⁺ = 274

### Beispiel IX

### (3S)-6-Chlor-4-N-(4-methoxyphenoxycarbonyl)-3-methyl-3,4- dihydro-chinoxalin-2(1H)-on

Die Verbindung des Beispiels IB (0.5 g, 2.55 mmol) wurde in 10 ml wasserfreiem N,N-Dimethylformamid gelöst und 0.41 ml (2.8 mmol) Triethylamin zugegeben. Unter Rühren wurden erst 0.42 ml (2.8 mmol) Chlorameisensäure-4-methoxyphenylester zugetropft und nach 2 h nochmals 0.21 ml (1.9 mmol). Nach vollständigem Umsatz (18 h) wurde das Lösungsmittel unter vermindertem Druck abgezogen, der Rückstand in Essigsäureethylester aufgenommen, mit Wasser gewaschen und getrocknet (Natriumsulfat). Nach dem Einengen blieben 0.48 g (54 %) eines weißen Feststoffs zurück. Schmp. 187 -190 °C (nach Umkristallisation aus Isopropanol)
¹H-NMR (270 MHz, d₆-DMSO): δ = 1.24 (d, J = 8 Hz, 3 H), 3.77 (s, 3 H), 4.94 (q, J = 8 Hz, 1 H), 6.97 (dd, J = 8, 2 Hz, 1 H), 7.03 (d, J = 8 Hz, 1 H), 7.2 - 7.3 (m, 3 H), 7.78 (s, 1 H), 10.89 ppm (br. s, 1 H).
MS: (M + H)⁺ = 347

### Beispiel X

### (3S)-6-Chlor-4-N-(4-fluorphenoxycarbonyl)-3-methyl-3,4-dihydro-chinoxalin-2(1H)-on

Die Verbindung wurde analog zu der in Beispiel VIA beschriebenen Verbindung hergestellt, außer daß Chlorameisensäure-4- fluorphenylester als Acylierungsmittel verwendet wurde. Schmp. 168 - 170 °C (nach Kristallisation aus Isopropanol)
¹H-NMR (270 MHz, d₆-DMSO): δ = 1.24 (d, J = 8 Hz, 3 H), 4.94 (q, J = 8 Hz, 1 H), 7.03 (d, 8 Hz, 1 H), 7.2 - 7.5 (m, 5 H), 7.83 (d, J = 2 Hz, 1 H), 10.90 ppm (br. s, 1 H).
MS: (M + H)⁺ = 335

### Beispiel XI

### (3S)-6-Chlor-4-N-(4-chlorphenoxycarbonyl)-3-methyl-3,4-dihydro-chinoxalin-2(1H)-on

Die Verbindung wurde analog zu der in Beispiel VIA beschriebenen Verbindung hergestellt, außer daß Chlorameisensäure-4- chlorphenylester zur Acylierung eingesetzt wurde. Schmp. 185 - 188 °C (nach Kristallisation aus Isopropanol/Diethylether)
¹H-NMR (270 MHz, d₆-DMSO): δ = 1.25 (d, J = 8 Hz, 3 H), 4.94 (q, J = 8 Hz, 1 H), 7.04 (d, 8 Hz, 1 H), 7.25 (dd, J = 8, 2 Hz, 1 H), 7.35 - 7.6 (m, 4 H), 7.80 (s, 1 H), 10.91 ppm (br. s, 1 H).
MS: (M + H)⁺ = 351

### Beispiel XII

### (3S)-4-N-(2-Bromethyloxycarbonyl)-6-chlor-3-methyl-3,4-dihydrochinoxalin-2(1H)-on

Die Verbindung wurde analog zu der in Beispiel VIA beschriebenen Verbindung hergestellt, außer daß Chlorameisensäure-2- bromethylester zur Acylierung eingesetzt wurde. Schmp. 133 - 136 °C (nach Kristallisation aus Isopropanol)
¹H-NMR (270 MHz, d₆-DMSO): δ = 1.16 (d, J = 8 Hz, 3 H), 3.7 - 3.8 (m, 2 H), 4.4 - 4.6 (m, 2 H), 4.86 (q, J = 8 Hz), 6.99 (d, 8 Hz, 1 H), 7.21 (dd, 8, 2 Hz, 1 H), 7.74 (d, J = 2 Hz, 1 H), 10.84 ppm (br. s, 1 H).
MS: (M + H)⁺ = 348

### Beispiel XIII

### (3S)-6-Chlor-N-(isopropenyloxycarbonyl)-3-methyl-3,4-dihydro-chinoxalin-2(1H)-on

Die Substanz wurde analog zu der in Beispiel VIA beschriebenen Verbindung hergestellt, außer daß Chlorameisensäure- isopropenylester zur Acylierung eingesetzt wurde. Schmp. 158 - 159 °C
¹H-NMR (270 MHz, CDCl₃): δ = 1.33 (d, J = 8 Hz, 3 H), 2.02 (s, 3 H), 4.79 (s, 1 H), 4.83 (s, 1 H), 5.17 (q, J = 8 Hz, 1 H), 6.86 (d, J = 8 Hz, 1 H), 7.12 (dd, J = 8 , 2 Hz, 1 H), 7.74 (br. s, 1 H), 9.28 ppm (br. s, 1 H).
MS: (M + H)⁺ = 281

### Beispiel XIV

### (3S)-6-Chlor-3-methyl-4-N-(vinyloxycarbonyl)-3,4-dihydrochinoxalin-2(1H)-on

Die Substanz wurde analog zu der in Beispiel VIA beschriebenen Verbindung hergestellt, außer daß Chlorameisensäurevinylester zur Acylierung eingesetzt wurde. Schmp. 177 - 179 °C
¹H-NMR (270 MHz, CDCl₃): δ = 1,33 (d, J = 8 Hz, 3 H), 4.96 (dd, J = 14, 2 Hz, 1 H), 5.20 (q, J = 8 Hz, 1 H), 6.83 (d, J = 8 Hz, 1 H), 7.12 (dd, J = 8, 2 Hz, 1 H), 7.2 - 7.3 (m, 2 H), 7.71 (br. s, 1 H), 9.42 ppm (br. s, 1 H).
MS: (M + H)⁺ = 267

### Beispiel XV und Beispiel XVI

6-Chlor-3,4-dihydro-chinoxalin-2(1H)-on wurde analog zu dem in Beispiel VIA beschriebenen Verfahren mit 3-Methyl-2-buten-1-ylbromid umgesetzt. Mittels Chromatographie an Kieselgel ließen sich zwei Produkte isolieren.
6-Chlor-4-N-(3-methyl-2-buten-1-yl)-3,4-dihydro-chinoxalin- 2(1H)-on Schmp. 150 - 151 °C (nach Umkristallisation aus Essigsäureethylester)
¹H-NMR (270 MHz, d₆-DMSO): δ = 1.72 (s, 6 H), 3.67 (s, 2 H), 3.80 (d, J = 7 Hz, 2 H), 5.20 (m, 1 H), 6.7 - 6.8 (m, 3 H), 10.49 ppm (br. s, 1 H).
MS: (M + H)⁺ = 251

6-Chlor-4-N-(3-methyl-2-buten-1-yl)-3-(1,1-dimethyl-2-propen-1-yl)-3,4-dihydrochinoxalin-2(1 H)-on
Schmp. 110 - 112 °C (nach Kristallisation aus Heptan)
¹H-NMR (270 MHz, d₆-DMSO): δ = 0.94 (s, 3 H), 0.97 (s, 3 H), 1.65 (s, 3 H), 1.66 (s, 3 H), 3.77 (dd, J = 16, 7 Hz, 1 H), 4.23 (dd, J = 16, 7 Hz, 1 H), 4.8 - 4.9 (m, 2 H), 5.02 (m, 1 H), 5.75 (dd, J = 17, 11 Hz, 1 H), 6.6 - 6.7 (m, 3 H), 10.49 ppm (br. s, 1 H).
MS: (M + H)⁺ = 319

Die folgenden Verbindungen der Formel I wurden aus den entsprechenden unsubstituierten Chinoxalinonen in analoger Weise synthetisiert und gegebenenfalls weiter derivatisiert:

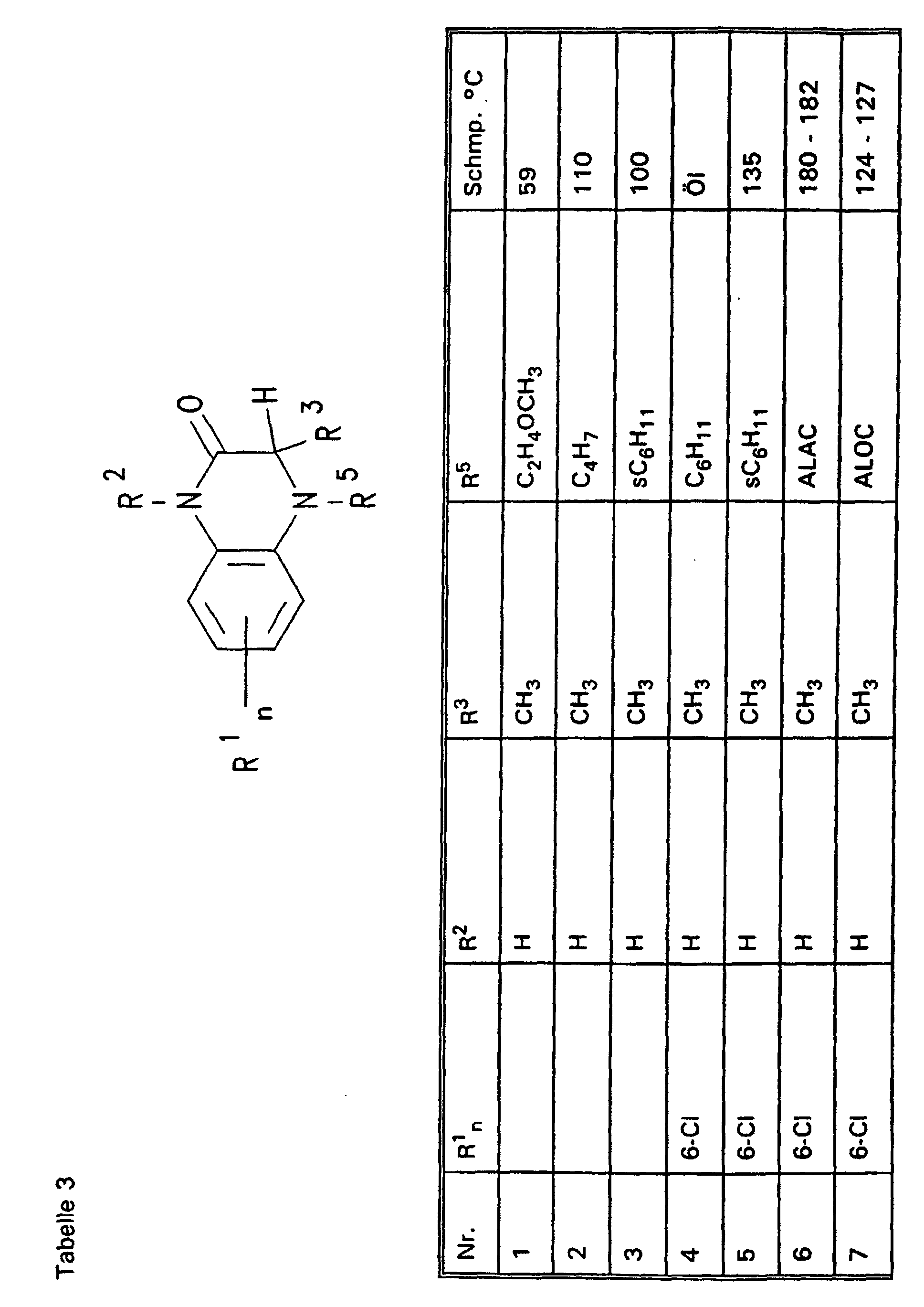

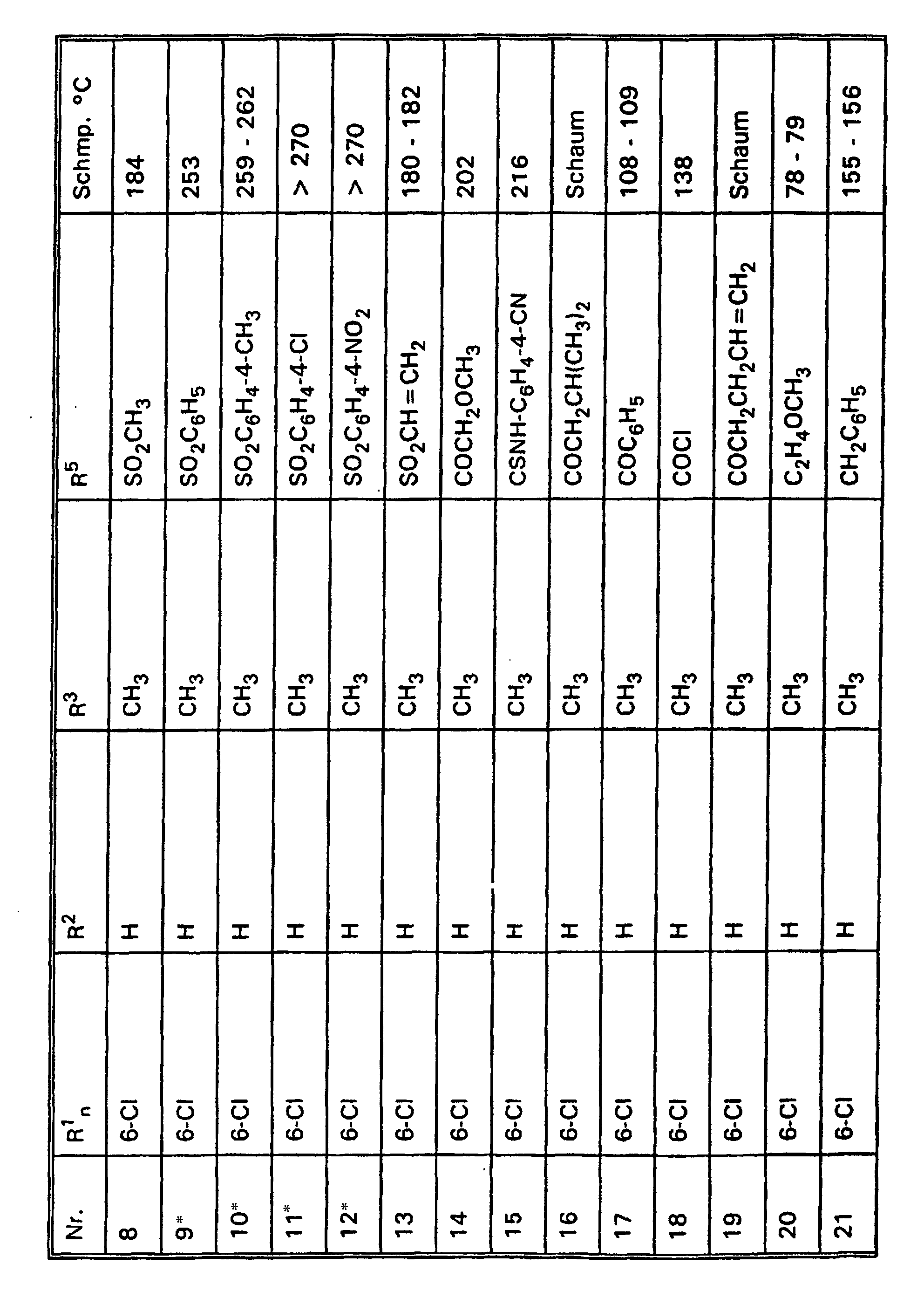

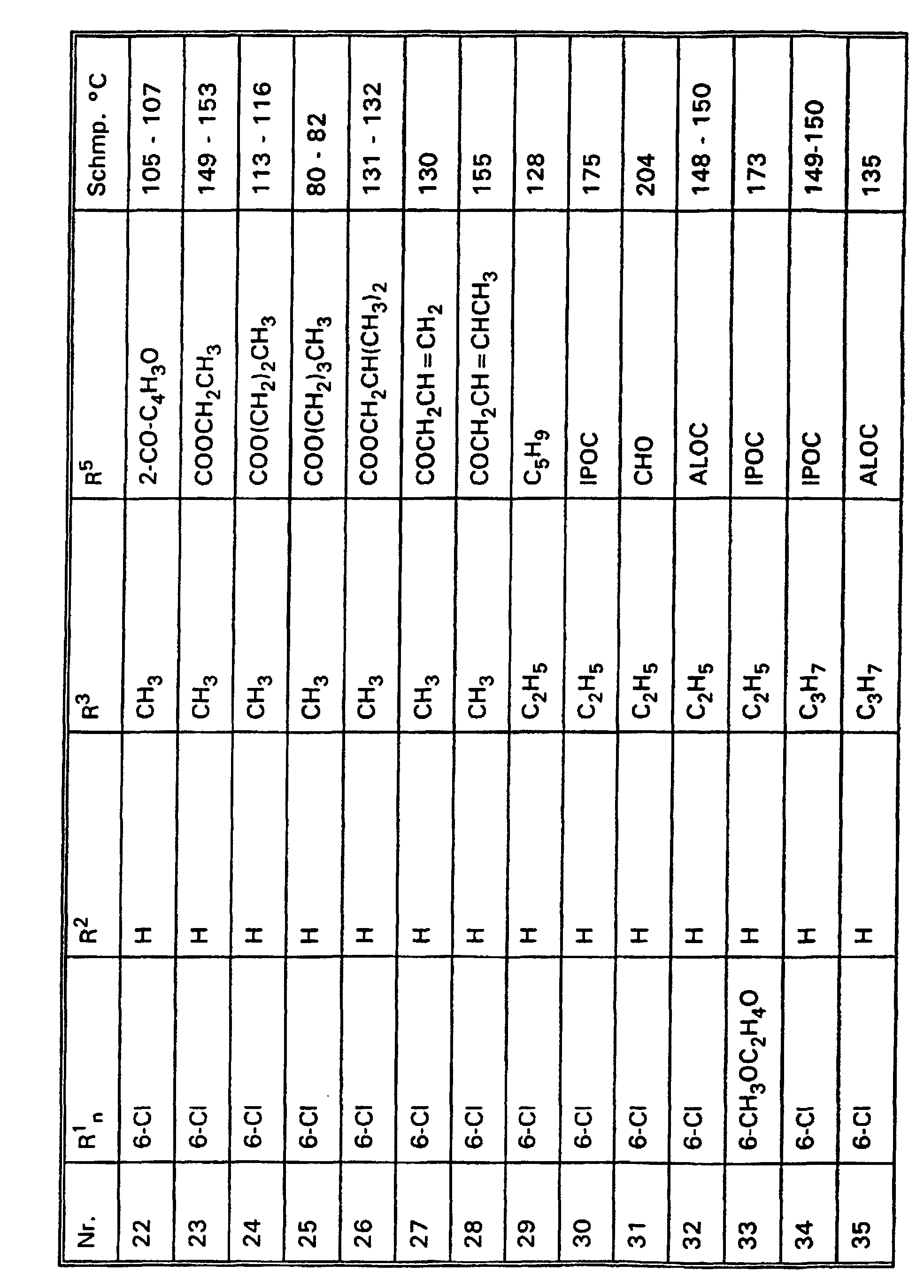

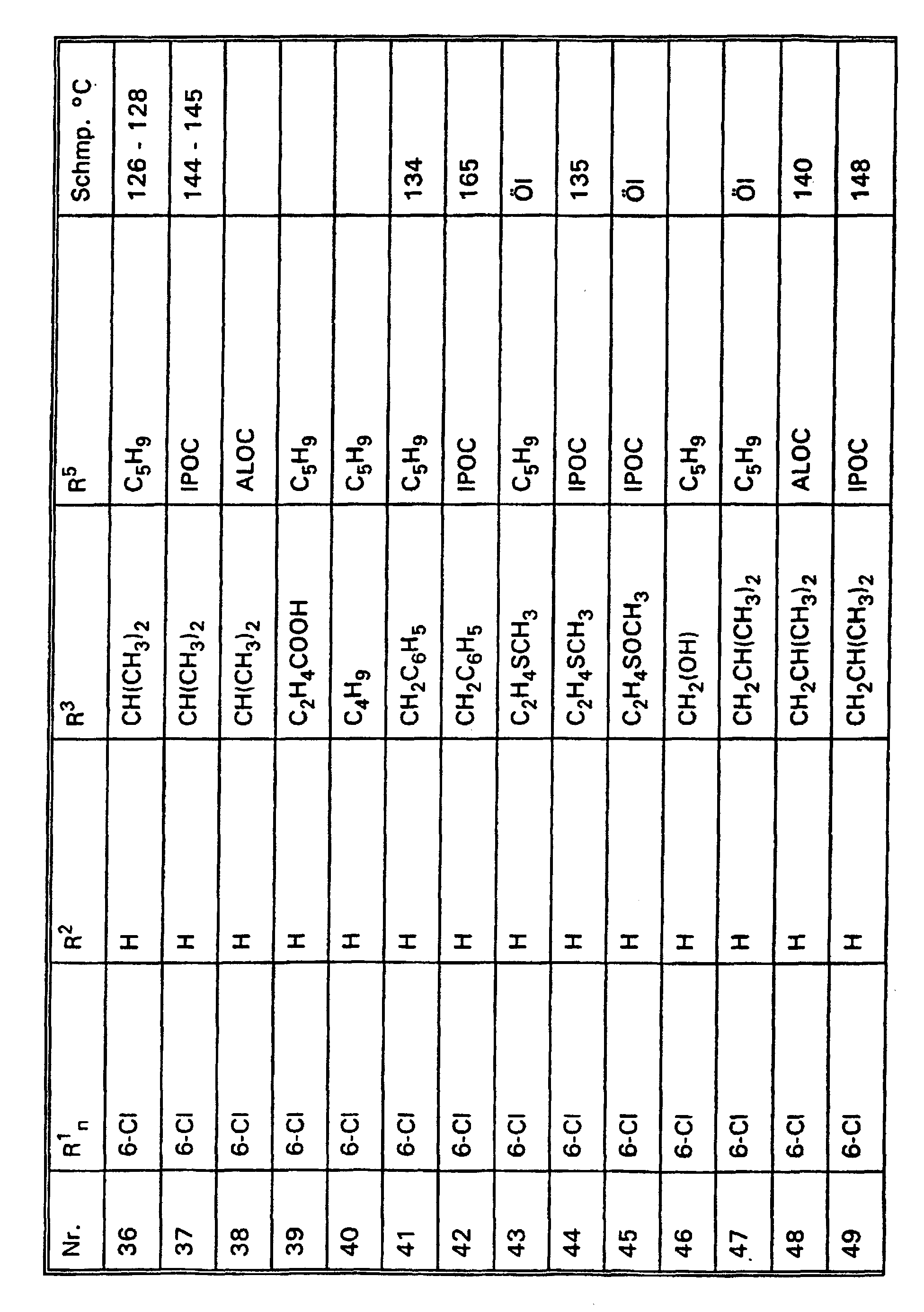

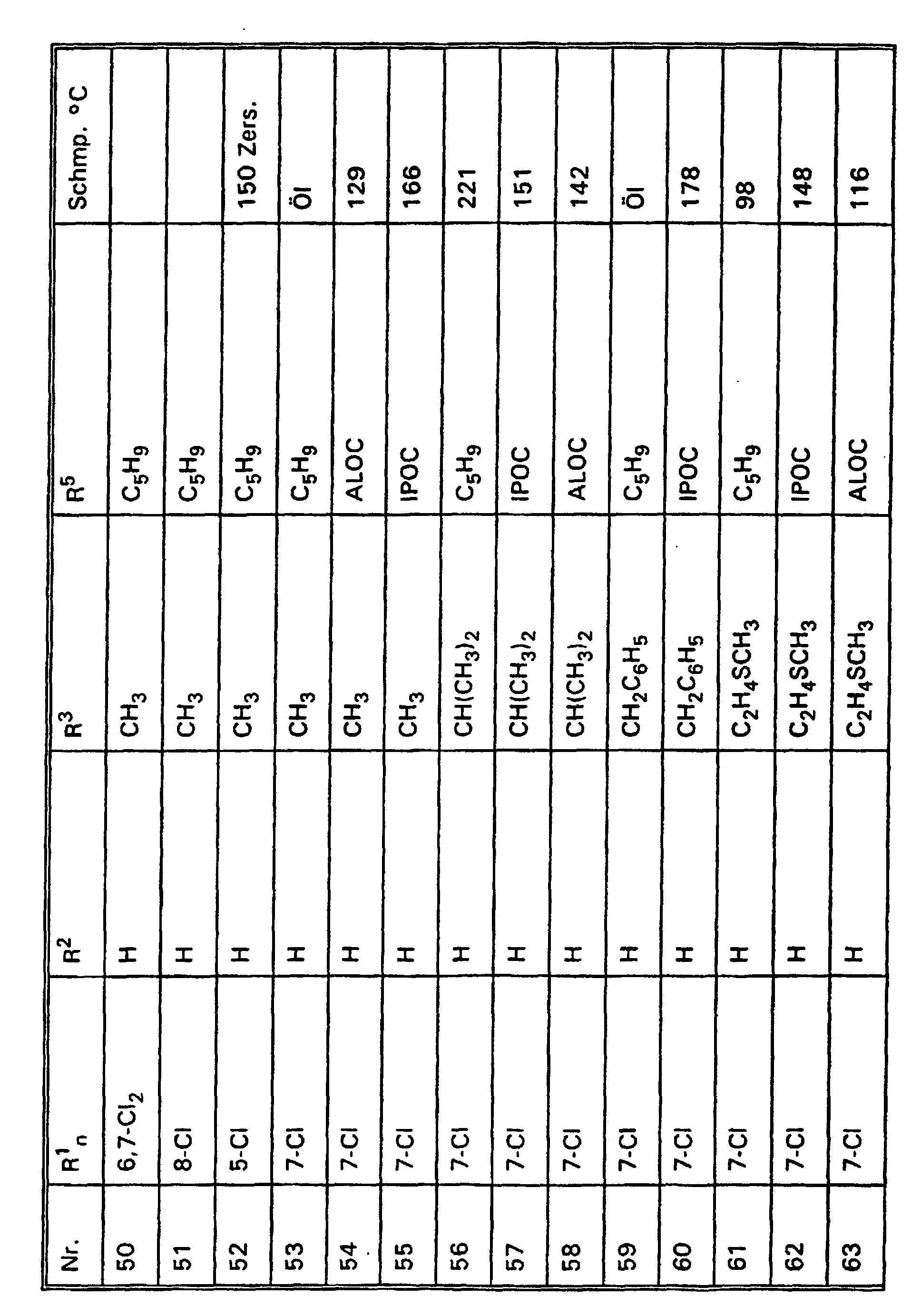

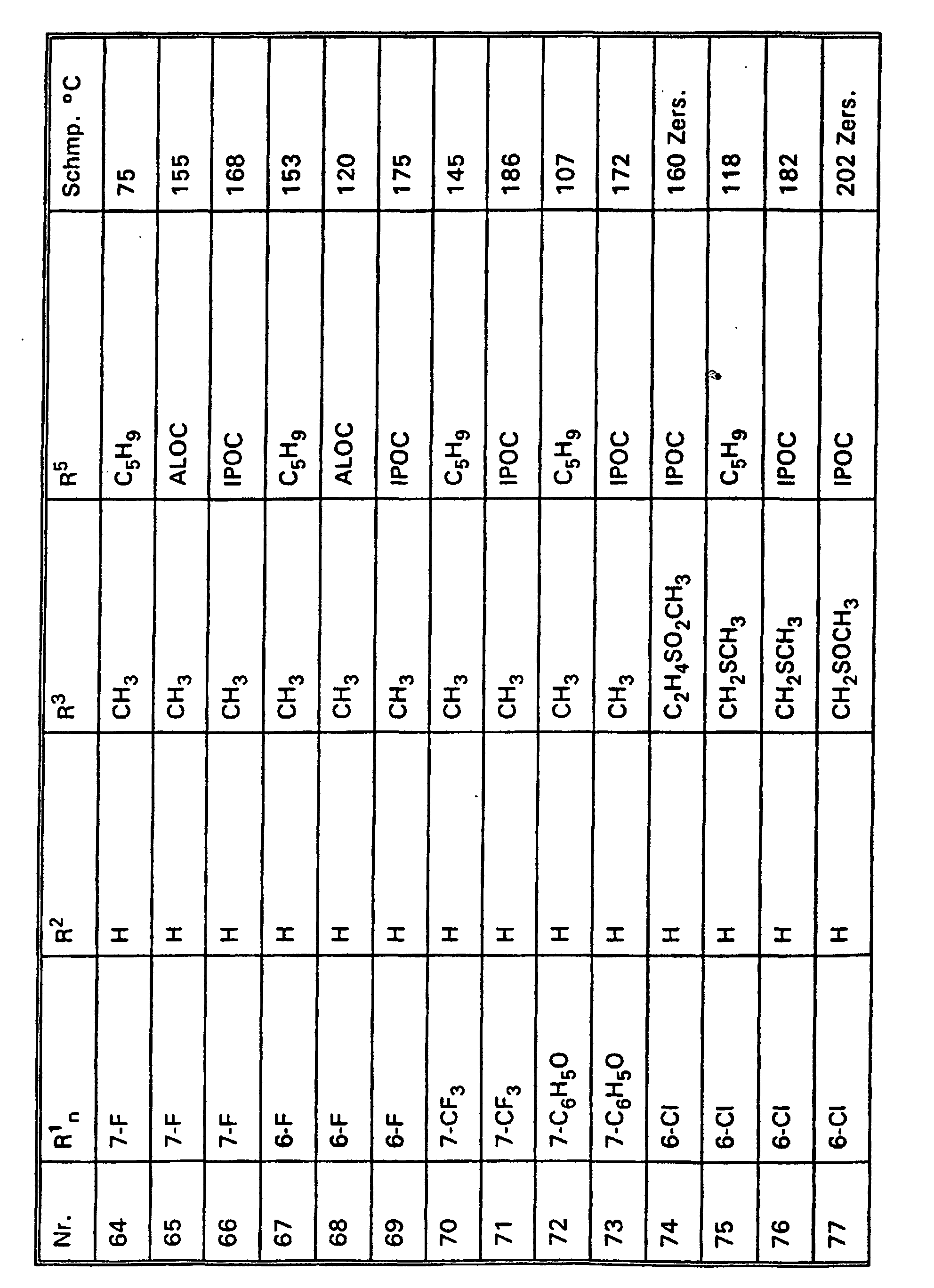

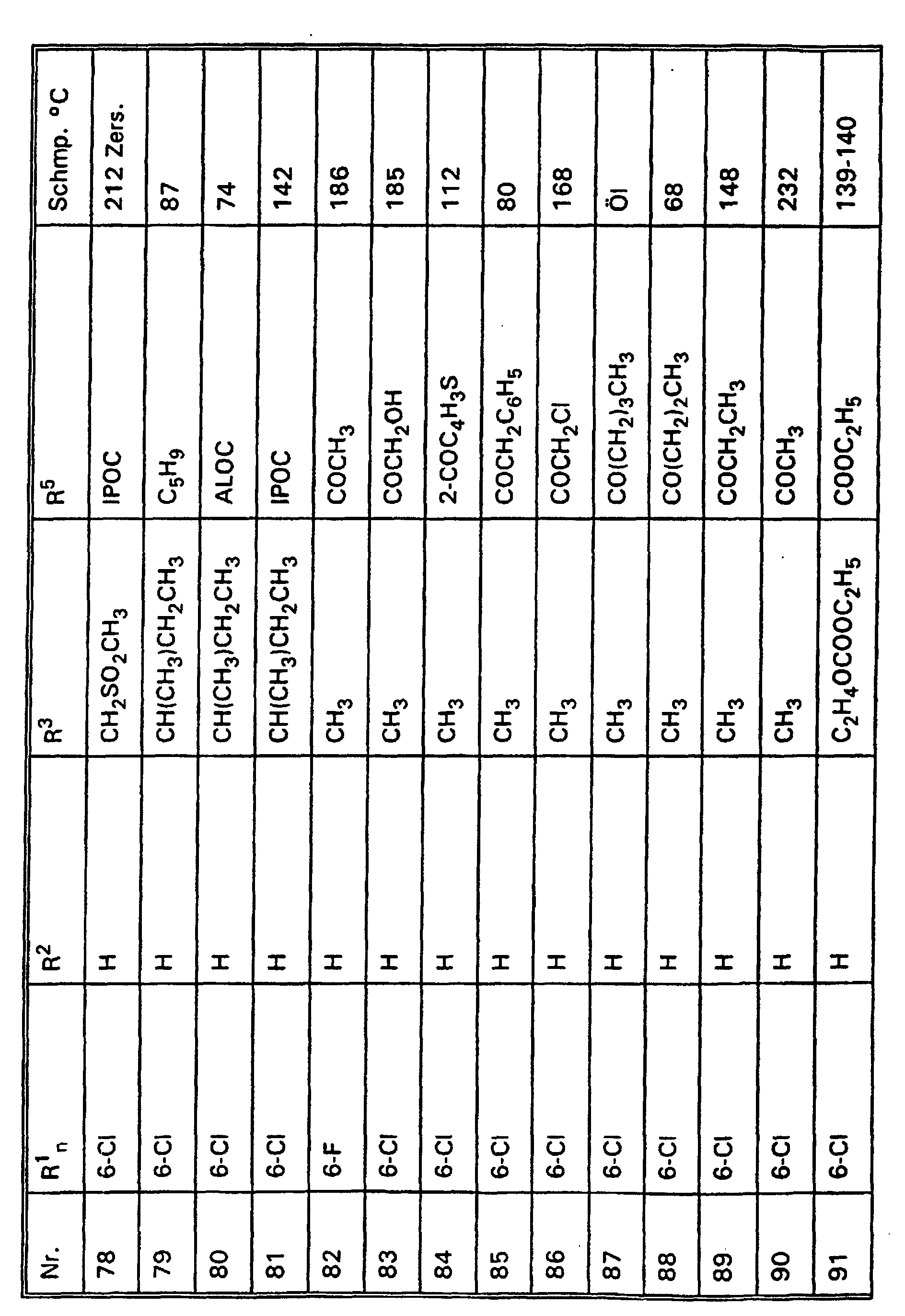

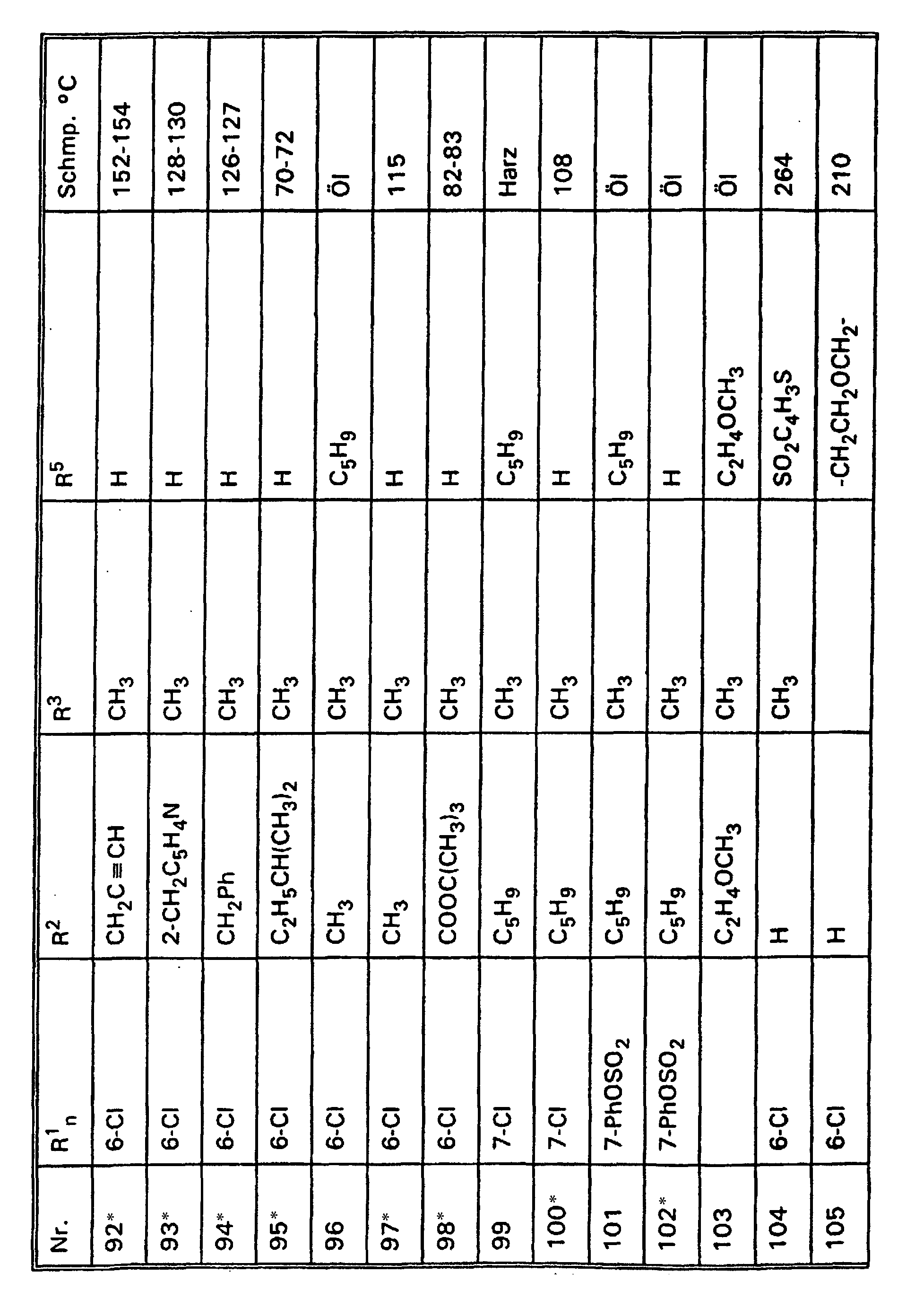

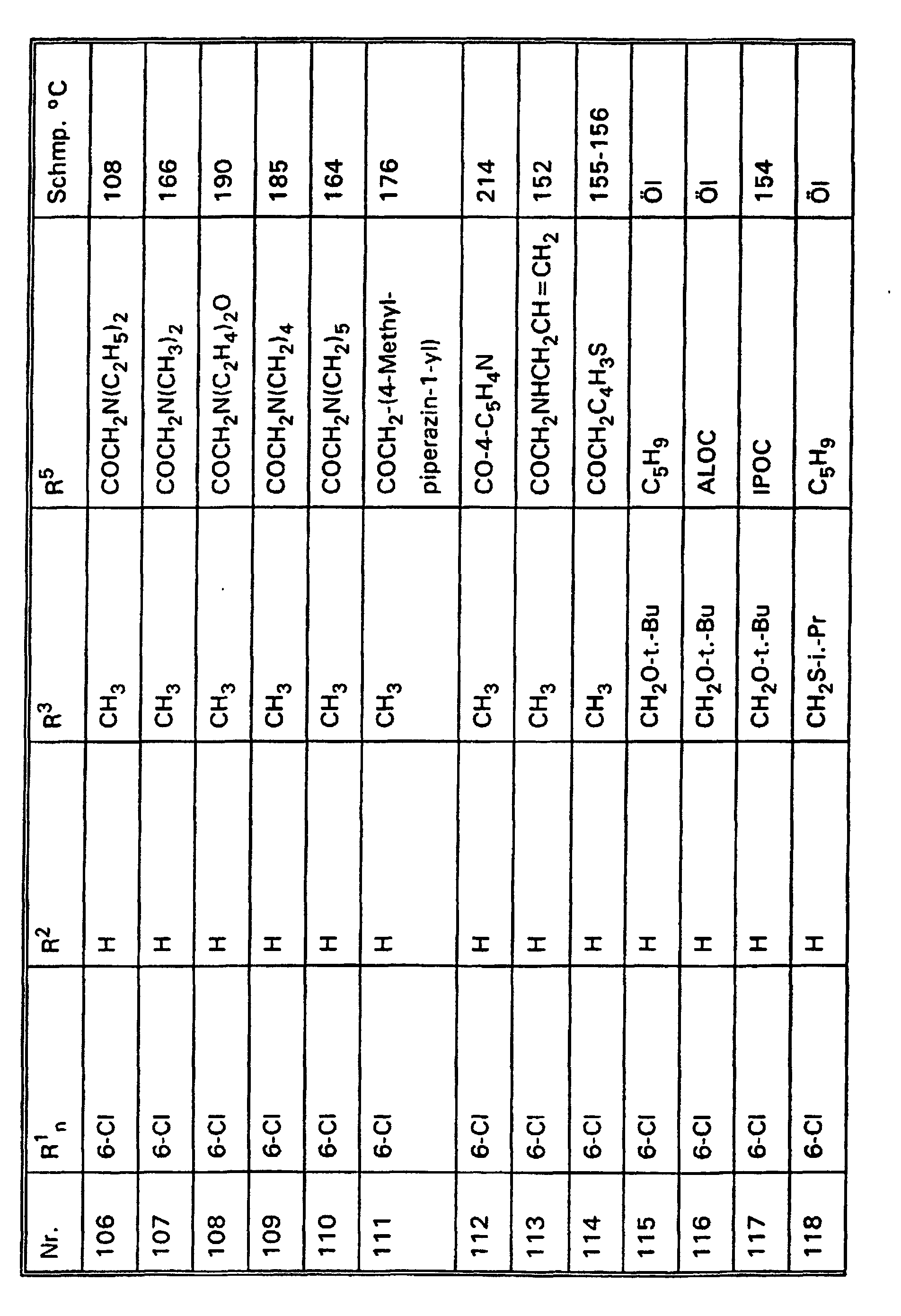

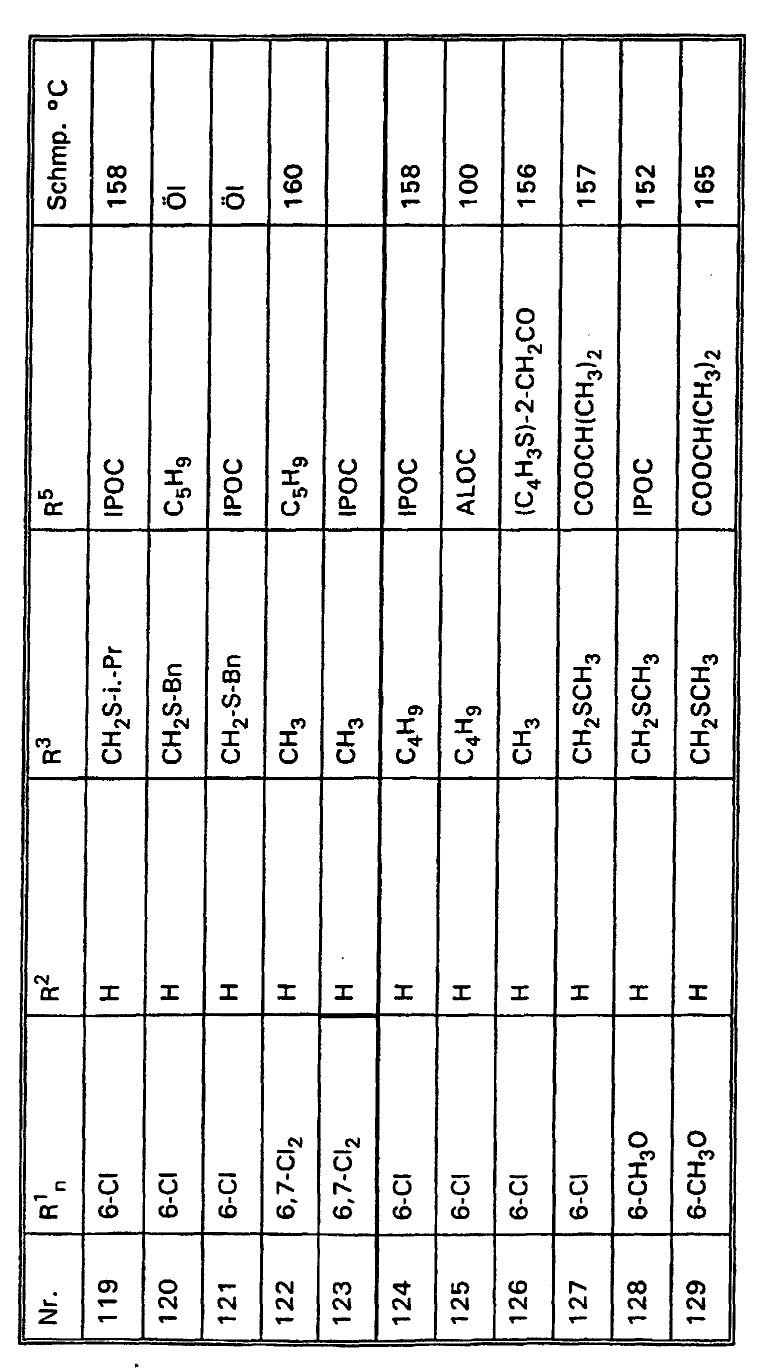

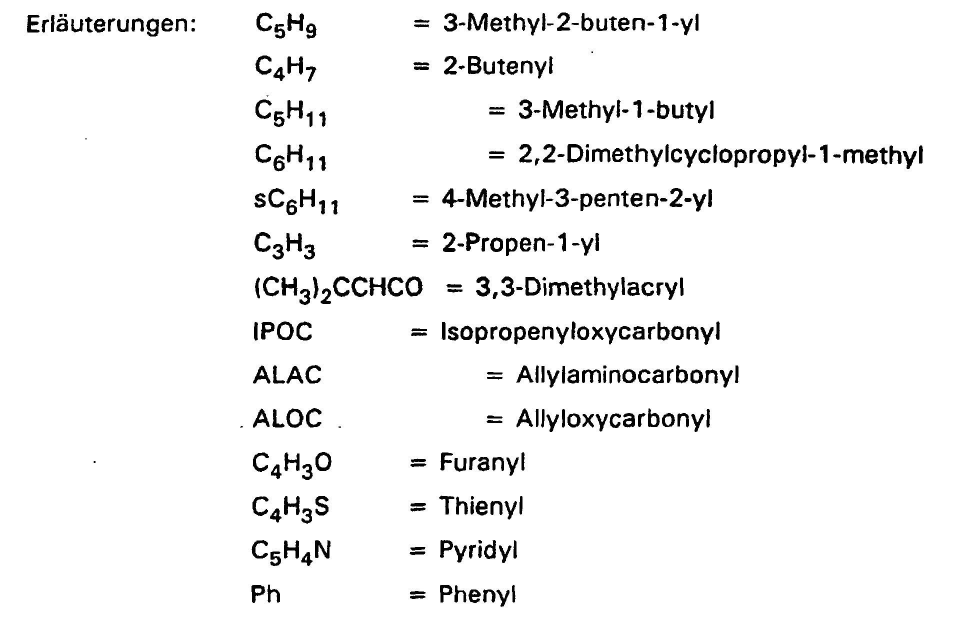

### Beispiel XVII

### 6,7-Dimethoxy-3-methyl-3,4-dihydro-chinoxalin-2(1H)-on

4,5-Dimethoxy-1,2-dinitrobenzol (34.2 g, 0.15 mol) wurden in 500 ml Methanol unter Raney-Nickel-Katalyse mit 1 atm Wasserstoff hydriert. Nach Aufnahme der berechneten Menge Wasserstoff wurde abgebrochen, vom Katalysator abgesaugt und das Lösungsmittel im Vakuum abgezogen. Um alles Wasser zu entfernen, wurde noch zweimal in Methanol aufgenommen und wieder eingeengt. Das als braunes Öl verbleibende 4,5-Dimethoxy-1,2-phenylendiamin (24.0 g) wurde in 200 ml Ethanol (96 %) unter Zusatz von 21.0 ml (0.15 mol) Triethylamin mit 17.1 ml (0.15 mol) 2-Chlorpropionsäuremethylester für 48 h unter Rückfluß erhitzt. Die tiefdunkle Lösung wurde eingeengt, in Essigsäureethylester aufgenommen, zweimal mit Wasser gewaschen, getrocknet (Natriumsulfat) und das Lösungsmittel im Vakuum abgezogen.
Das Rohprodukt wurde durch Anrühren mit Diethylether kristallisiert (6.2 g, 19 %). Eine analysenreine Probe mit Schmelzpunkt 151 °C erhielt man durch Chromatographie an Kieselgel mit Essigsäureethylester als Elutionsmittel.
¹H-NMR (60 MHz d₆-DMSO): δ = 1.22 (d, J = 7 Hz, 3 H), 3.63 (s, 3 H), 3.67 (s, 1 H), 3.6 - 3.7 (m, 1 H), 5.62 (br. s, 1 H), 6.40 (s, 1 H), 6.45 (s, 1H), 9.90 ppm (br. s, 1 H).
MS: M⁺ = 222

Die folgenden Verbindungen der Formel I wurden in analoger Weise synthetisiert und gegebenenfalls weiter derivatisiert:

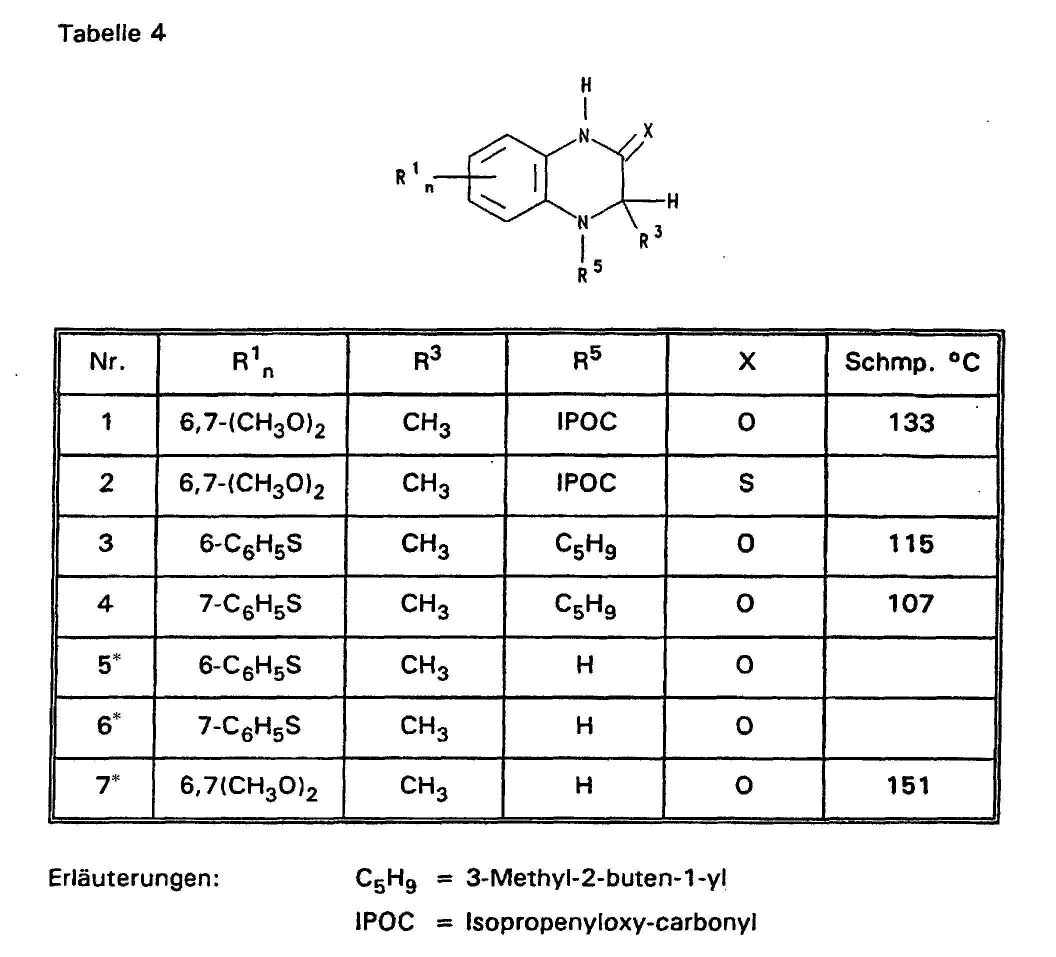

### Beispiel XVIII

### (3RS)-6-Chlor-4-N-(cyclopropyl)-3-methyl-3,4-dihydro-chinoxalin- 2(1H)-on

### A) (2RS)-N-(4-Chlor-2-cyclopropylaminophenyl)-(2-brom-propionsäureamid)

4-Chlor-2-cyclopropylamino-nitrobenzol (2.10 g, 0.01 mol) wurden in 100 ml Methanol unter Raney-Nickel-Katalyse mit 1 atm Wasserstoff hydriert. Nach Aufnahme der berechneten Menge Wasserstoff wurde abgebrochen, vom Katalysator abgesaugt und das Lösungsmittel im Vakuum abgezogen. Um alles Wasser zu entfernen, wurde noch zweimal in Methanol aufgenommen und wieder eingeengt. Das als braunes Öl verbleibende 4-Chlor-2- cyclopropylaminoanilin (1.80 g) wurde in 50 ml wasserfreiem 1,2-Dimethoxyethan gelöst und unter Rühren auf -60 °C abgekühlt. Eine Lösung von 1.1 ml (0.01 mol) 2-Brompropionsäurechlorid in 5 ml wasserfreiem 1,2-Dimethoxyethan wurde langsam zugetropft und die Reaktionsmischung 2 h bei -60 - -70 °C nachgerührt. Dann ließ man auf ca. -20 °C erwärmen und goß auf 150 ml eiskalte gesättigte wäßrige Natriumhydrogencarbonatlösung. Es wurde zweimal mit Essigsäureethylester extrahiert, die organische Phase einmal mit Wasser gewaschen, getrocknet (Natriumsulfat) und im Vakuum eingeengt. Nach Kristallisation mit Diethylether / Pentan verblieben 2.51 g (79 %) des gewünschten Produkts mit Schmp. 130 °C.
¹H-NMR (270 MHz, d₆-DMSO): δ = 0.4 - 0.5 (m, 2 H), 0.7 - 0.8 (m, 2 H), 1.75 (d, J = 7 Hz, 3 H), 2.39 (m, 1 H), 4.72 (q, J = 7 Hz, 1 H), 5.6 (br. s, 1 H), 6.66 (dd, J = 8, 2 Hz, 1 H), 6.96 (d, J = 2 Hz, 1 H), 7.21 (d, J = 8 Hz, 1 H), 9.36 ppm (br. s, 1 H).
MS: (M + H)⁺ = 319, 317

### B) (3RS)-6-Chlor-4-N-(cyclopropyl)-3-methyl-3,4-dihydro- chinoxalin-2(1 H)-on

Die Verbindung des Beispiels XVIIIA (318 mg, 1.0 mmol) wurde in 20 ml Ethanol (96 %) gelöst und nach Zugabe von 0.28 ml (2.0 mmol) Triethylamin 18 h unter Rückfluß erhitzt. Das Lösungsmittel wurde unter vermindertem Druck entfernt und das Reaktionsprodukt durch Chromatographie an Kieselgel mit Essigsäureethylester / Heptan = 1 : 2 als Elutionsmittel gereinigt. Die Ausbeute betrug 200 mg (85 %) weiße Kristalle vom Schmp. 167 °C (nach Kristallisation aus Pentan).
¹H-NMR (270 MHz, d₆-DMSO): δ = 0.40 (m, 1 H), 0.63 (m, 1 H), 0.76 (m, 1 H), 0.98 (m, 1 H), 1.12 (d, J = 7 Hz, 3 H), 2.47 (m, 1 H), 3.87 (q, J = 7 Hz, 1 H), 6.78 (s, 2 H), 7.0 (s, 1 H), 10.46 ppm (br. s, 1 H).
MS: (M + H)⁺ = 237

Die folgenden Verbindungen der Formel I wurden analog der in Beispiel XVIII beschriebenen Reaktionsführung unter Verwendung der entsprechend substituierten ortho-Nitroaniline und 2- Halogencarbonsäurederivate synthetisiert und gegebenenfalls weiter derivatisiert:

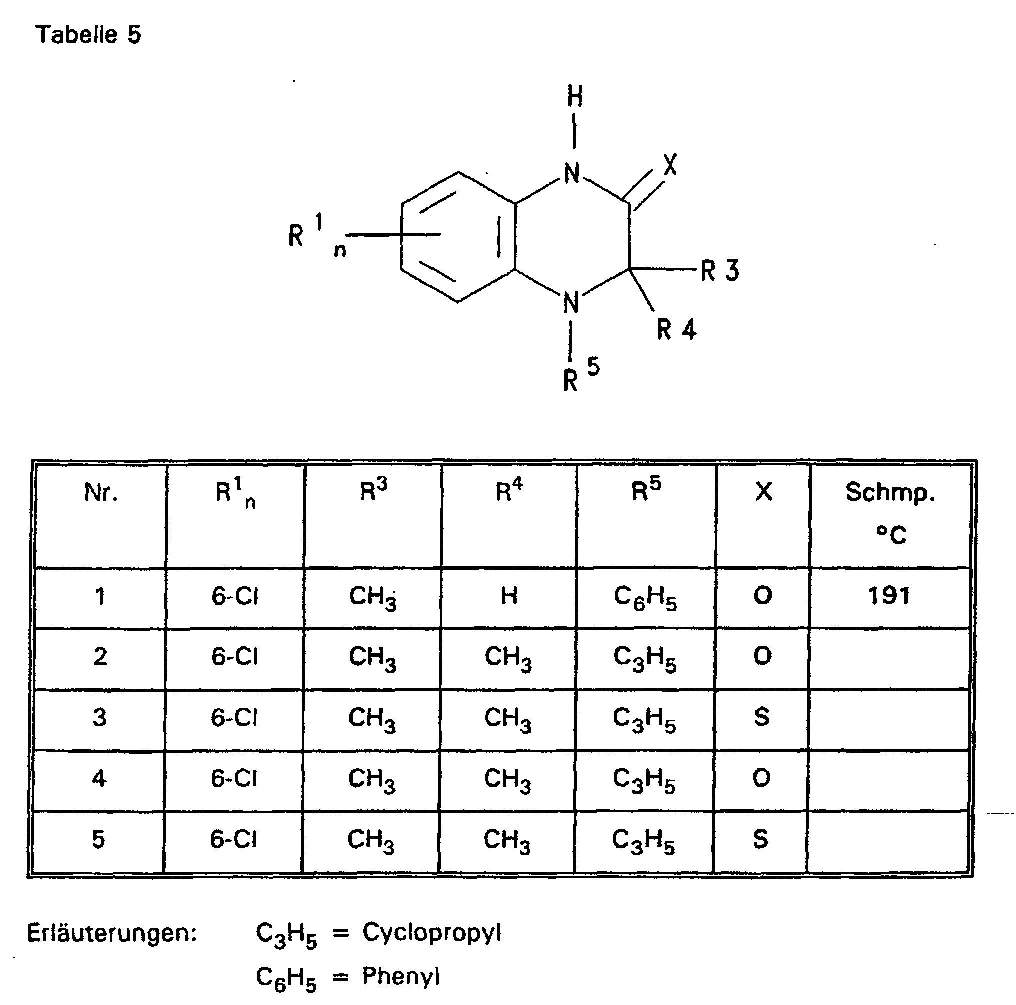

### Beispiel XIX

### 7-Chlor-1-N-(cyctopropyl)-3,3-dimethyl-3,4-dihydro-chinoxalin-2(1H)-on

4-Chlor-2-cyclopropylamino-nitrobenzol (2.0 g, 9.4 mmol) wurde wie unter Beispiel XVIIIA beschrieben hydriert. Das entstandene 4-Chlor-2-cyclopropylamino-anilin (1.70 g) wurde in 20 ml Dichlormethan aufgenommen. Es wurden 1.6 ml (2.01 mmol) Chloroform, 1.8 ml (2.45 mmol) Aceton und 0.10 g (0.4 mmol) Benzyltriethylammoniumchlorid zugesetzt und die Reaktionslösung auf 10 °C gekühlt. Langsam wurden unter kräftigem Rühren 4 ml 50 %-ige Natronlauge zugetropft, wobei die Reaktionstemperatur 10 °C nicht übersteigen sollte. Nach 5 h Rühren bei 10 °C wurden die Phasen verdünnt und getrennt. Die organische Phase wurde einmal mit Wasser gewaschen, getrocknet (Magnesiumsulfat) und im Vakuum eingedampft. Das Rohprodukt wurde durch Chromatographie an Kieselgel mit Essigsäureethylester / Heptan = 1 : 2 als Elutionsmittel gereinigt. Die Ausbeute betrug 1.0 g (42 %) weiße Kristalle vom Schmelzpunkt 132 - 133 °C (nach Umkristallisation aus Toluol / Heptan).
¹H-NMR (270 MHz, d₆-DMSO): δ = 0.45 - 0.55 (m, 2 H), 1.05 - 1.1 (m, 2 H), 1.19 (s, 6 H), 2.71 (m, 1 H), 6.09 (br. s, 1 H), 6.71 (d, J = 8 Hz, 1 H), 6.88 (dd, J = 8, 2 Hz, 1 H), 7.19 ppm (d, J = 2 Hz, 1 H).
MS: (M + H)⁺ = 251

Die folgenden Verbindungen der Formel I wurden in analoger Weise synthetisiert und gegebenenfalls weiter derivatisiert:

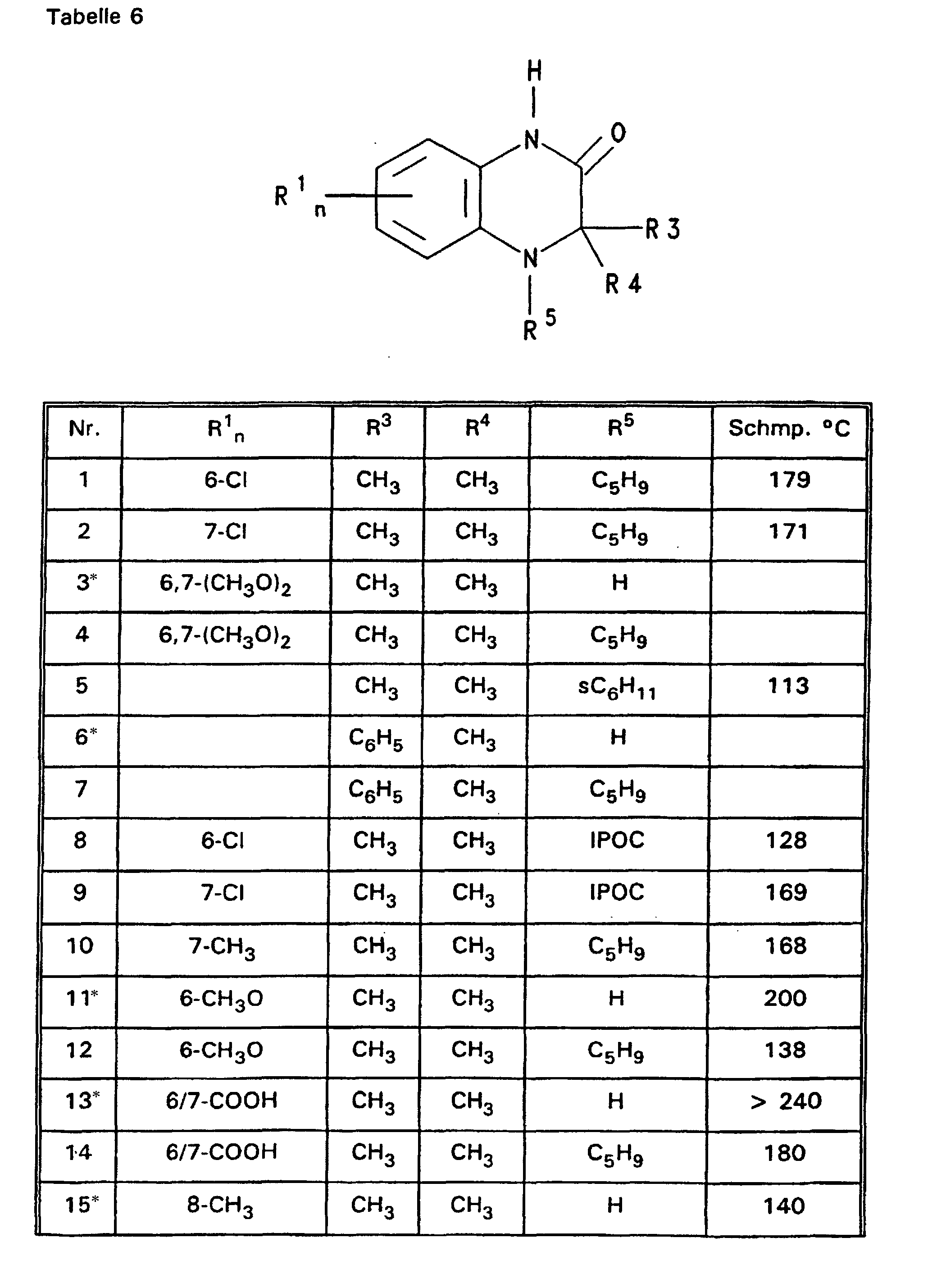

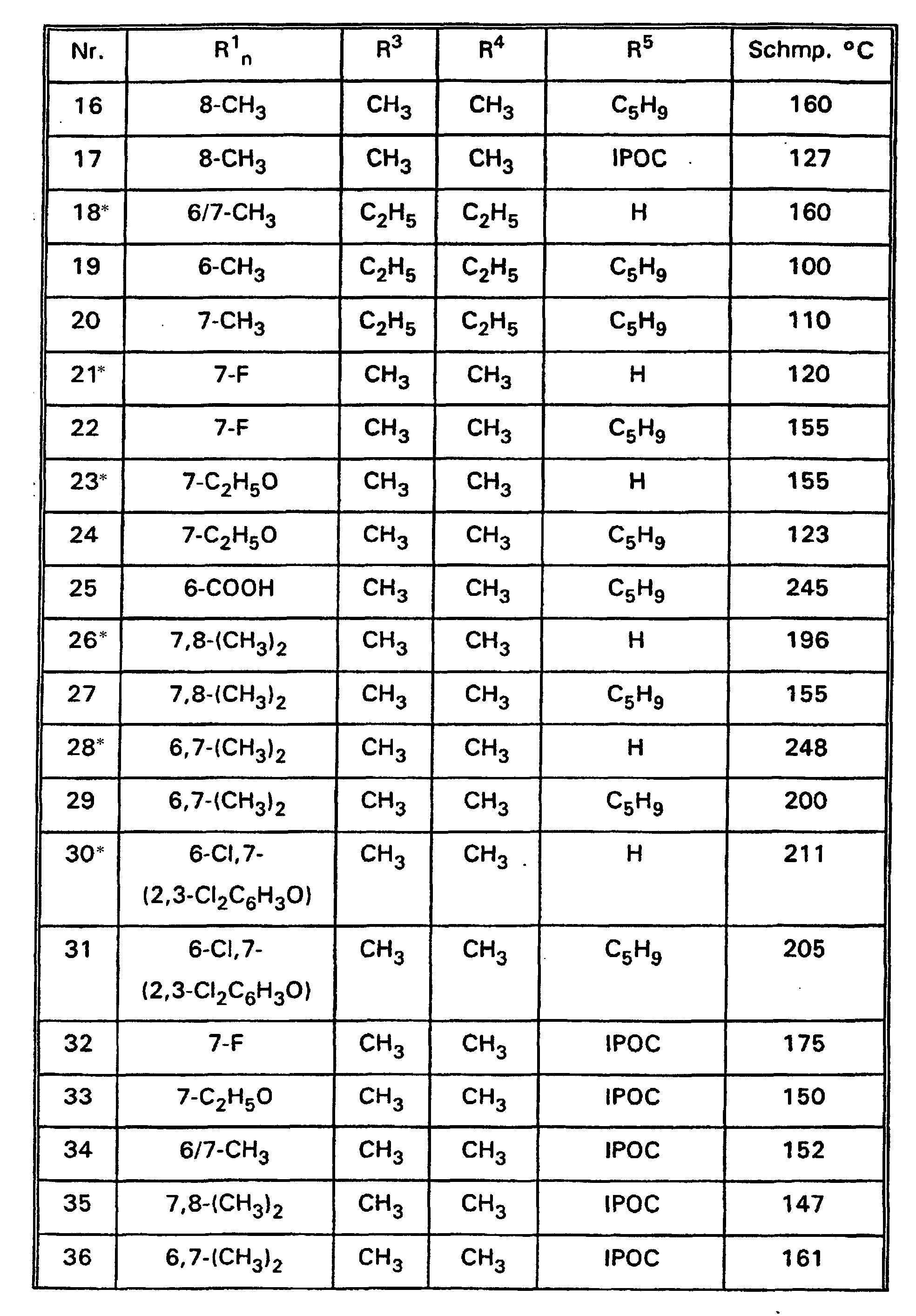

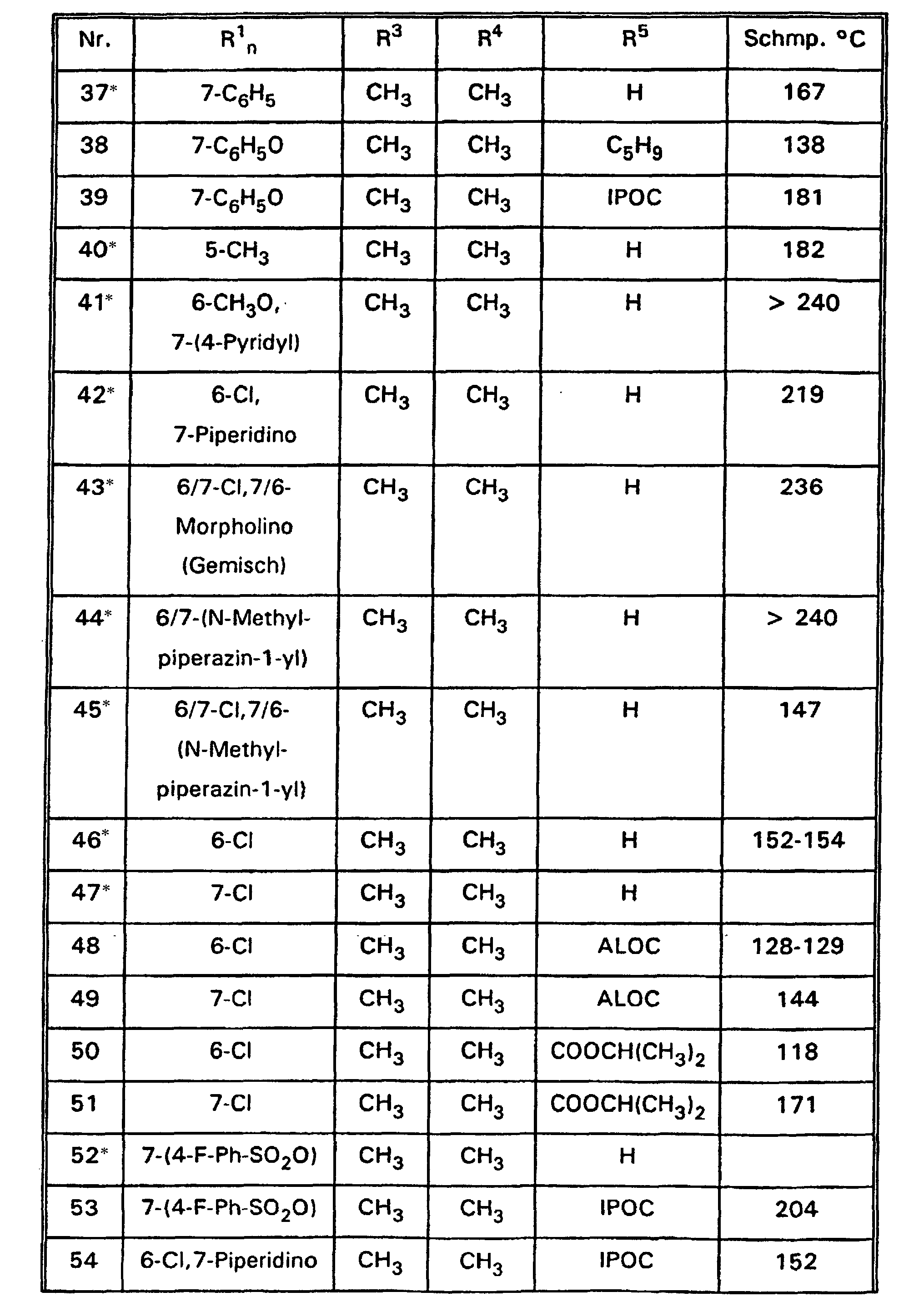

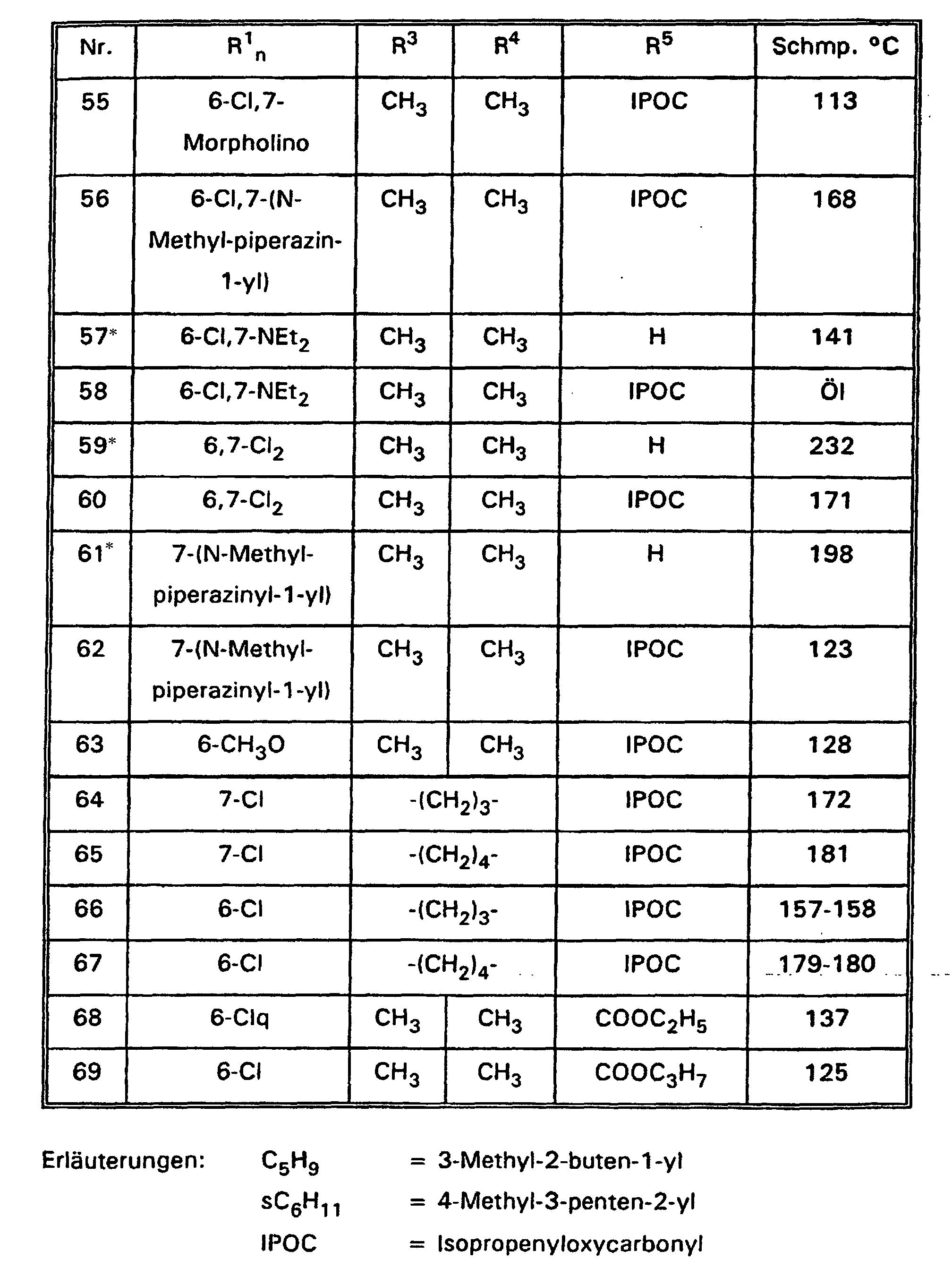

### Beispiel XX

### 3,3-Dimethyl-4-N-(3-methyl-2-buten-1-yl)-3,4-dihydro-chinoxalin- 2(1 H)-on

Die Verbindung wurde analog zu der in Beispiel VIA beschriebenen Verbindung ausgehend von 3,3-Dimethyl-3,4-dihydro-chinoxalin- 2(1H)-on (J. T. Lai, Synthesis 1982, 71) hergestellt. Schmp. 146 - 147 °C (nach Kristallisation aus Methyl-tert.-butylether / Heptan)
¹H-NMR (270 MHz, d₆-DMSO): δ = 1.27 (s, 3 H), 1.68 (s, 3 H), 1.72 (s, 3 H), 3.88 (d, J = 7 Hz, 1 H), 5.15 (m, 1 H), 6.60 (d, J = 7 Hz, 1 H), 6.67 (t, J = 7 Hz, 1 H), 6.78 (d, J = 7 Hz, 1 H), 6.87 (t, J = 7 Hz, 1 H), 10.33 ppm (br. s, 1 H). MS: (M + H)⁺ = 245

### Beispiel XXI

### 4-N-(3-Methyl-2-buten-1-yl)-3,4-dihydro-chinoxalin-2(1H)-on-3-spiro-1'cyclohexan

Die Verbindung wurde analog zu der in Beispiel VIA beschriebenen Verbindung ausgehend von Spiro[cyclohexan-1,3'-(3',4'-dihydro- chinoxalin-(1'H)-on)] (J. T. Lai, Synthesis 1982, 71) hergestellt. Schmp. 82 - 83 °C (nach Kristallisation aus Heptan)
¹H-NMR (270 MHz, d₆-DMSO): δ = 1.25 - 1.75 (m, 10 H), 3.75 (d, J = 6 Hz, 2 H), 5.07 (m, 1 H), 6.7 - 7.0 (m, 4 H), 10.15 ppm (br. s, 1 H).
MS: (M + H)⁺ = 285

### Beispiel XXII

### 4-N-(3-Methyl-2-buten-1-yl)-3,4-dihydro-chinoxalin-2(1H)-thion-3-spiro-1'cyclohexan

Die Verbindung des Beispiels XXI (500 mg, 1.8 mmol) wurde unter Argon zusammen mit 370 mg (0.9 mmol) 2,4-Bis-(4-methoxyphenyl)- 1,3-dithia-2,4-diphosphetan-2,4-disulfid (Lawesson's Reagenz) in 10 ml wasserfreiem Toluol für 1.5 h unter Rückfluß erhitzt. Anschließend wurde im Vakuum eingeengt und die Produkte durch Chromatographie an Kieselgel mit Methyl-tert. -butylether / Heptan = 10 : 1 als Elutionsmittel isoliert. Die Ausbeute betrug 50 mg (9 %) gelbe Kristalle vom Schmp. 125 °C.
¹H-NMR (270 MHz, d6-DMSO): δ = 1.1 - 1.9 (m, 16 H), 3.64 (d, J = 7 Hz, 2 H), 4.99 (m, 1 H), 6.95 - 7.1 (m, 3 H), 7.18 (d, J = 7 Hz, 1 H), 12.2 ppm (br. s, 1 H).
MS: (M + H)⁺ = 301

Als weiteres Produkt wurde 3,4-Dihydro-chinoxalin-2(1H)-thion-3-spiro-1'cyclohexan mit einer Ausbeute von 110 mg (26 %) isoliert; gelbe Kristalle vom Schmp. 178 °C.
¹H-NMR (270 MHz, CDCl₃): δ = 1.25 - 2.2 (m, 10 H), 4.18 (br. s, 1 H), 6.7-6.8 (m, 3 H), 6.97 (m, 1 H), 9.42 ppm (br. s, 1 H).
MS: (M + H)⁺ = 233.

### Beispiel XXIII

### (35)-6-Chlor-4-N-(isopropenyloxycarbonyl)-3-methyl-3,4-dihydrochinoxalin-2(1H)-thion

Die Verbindung des Beispiels XIII (0.5 g, 1.78 mmol) gelöst in 10 ml wasserfreiem Pyridin wurde mit 0.47 g (2.12 mmol) Phosphorpentasulfid für 4 h unter Rückfluß erhitzt. Es wurde im Vakuum eingeengt und an Kieselgel mit Essigsäureethylester/Heptan = 1 : 1 als Elutionsmittel chromatographiert. Man erhielt 0.25 g (47 %) eines gelb kristallinen Feststoffs vom Schmelzpunkt 148 - 150 °C (nach Umkristallisation aus Essigsäureethylester/Heptan).
¹H-NMR (270 MHz, d₆-DMSO): δ = 1.24 (d, J = 7 Hz, 3 H), 1.96 (s, 3 H), 4.8 - 4.9 (m, 2 H), 5.28 (q, J = 7 Hz, 1 H), 7.22 (d, J = 8 Hz, 1 H), 7.30 (dd, J = 8, 2 Hz, 1 H), 7.72 (br. s, 1 H), 12.84 ppm (br. s, 1 H).
MS: (M + H)⁺ = 297.

Die folgenden Verbindungen der Formel I wurden aus den entsprechenden 3,4-Dihydrochinoxalin-2(1H)-onen in analoger Weise synthetisiert:

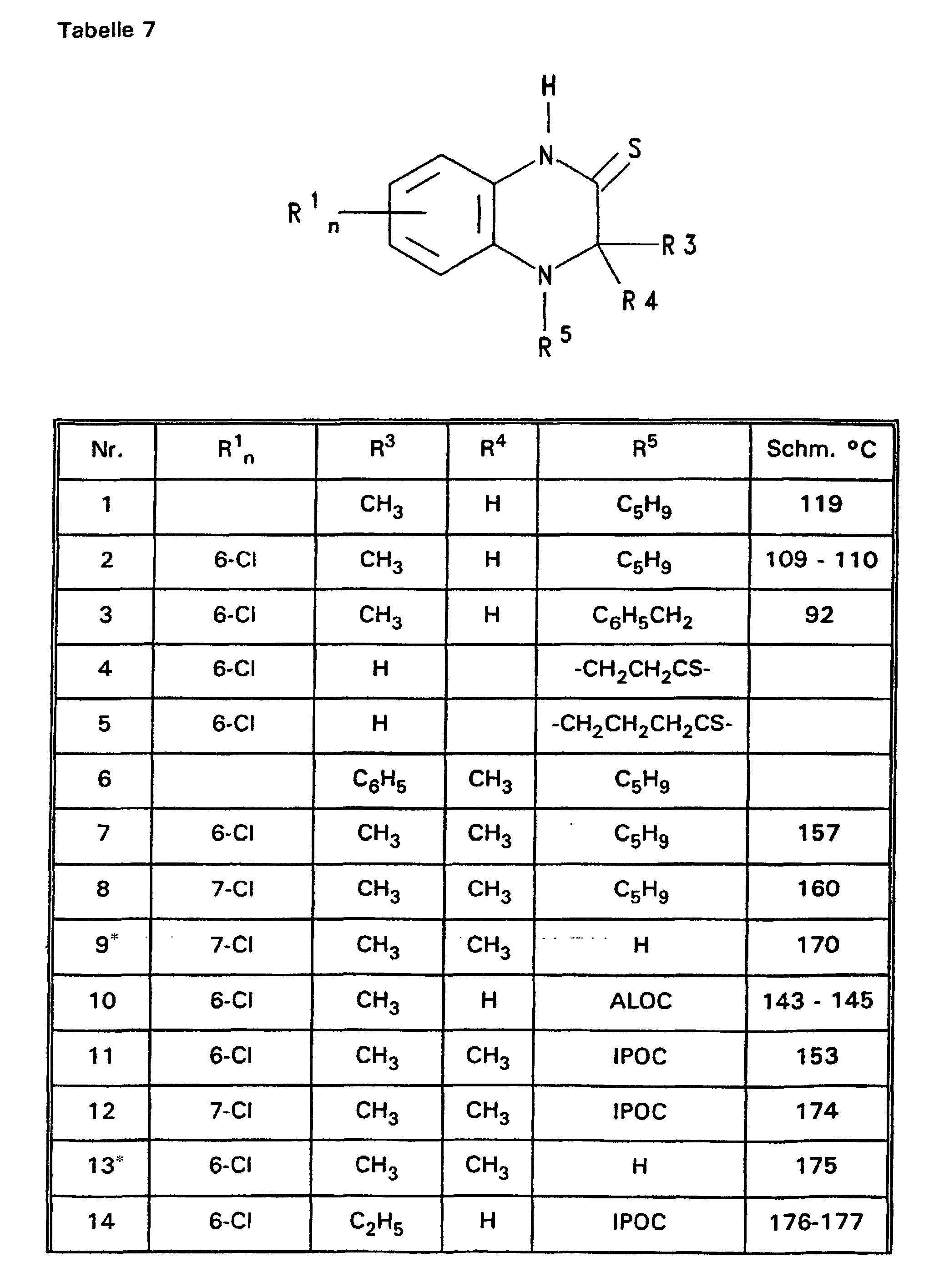

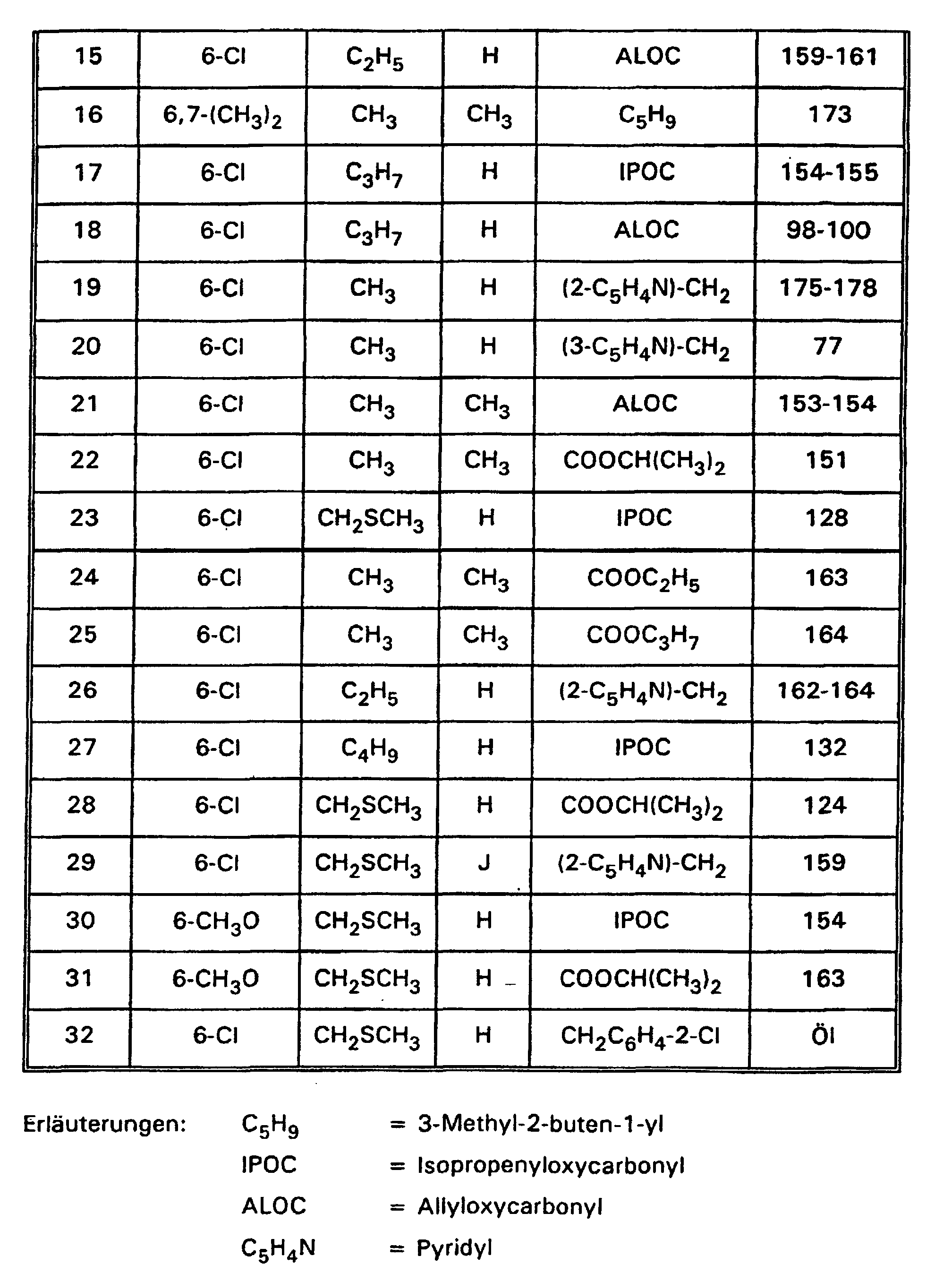

### Beispiel XXIV

### (3RS)-3-Methyl-4-N-(3-methyl-2-buten-1-yl)-2-methylthio-3,4-dihydro-chinoxalin

(3RS)-3-Methyl-4-N-(3-methyl-2-buten-1-yl)-3,4-dihydro-chinoxalin-2(1H)-thion (Tabelle 7, Nr. 1) (0.49 g, 2.0 mmol) wurde in 20 ml Ethanol (96 %) gelöst und mit 5.1 ml (2.2 mmol) einer 1 %igen Natriumethanolat-Lösung versetzt. Nach 15 min Rühren bei Raumtemperatur wurden 0.14 ml (2.2 mmol) Methyljodid zugetropft und es wurde für weitere 2 h bei Raumtemperatur gerührt. Die Reaktionslösung wurde eingeengt und der Rückstand an Kieselgel chromatographiert. Mit Essigsäureethylester / Heptan = 1 : 6 wurden 500 mg (96 %) eines gelben Öls isoliert.
¹H-NMR (d₆-DMSO): δ = 0.96 (d, J = 7 Hz, 3 H), 1.72 (s, 6 H), 2.44 (s, 3 H), 3.71 (dd, J = 15, 6 Hz, 1 H), 3.89 (dd, J = 15, 6 Hz, 1 H), 4.00 (q, J = 7 Hz, 1 H), 5.20 (m, 1 H), 6.65 - 6.75 (m, 2 H), 7.02 (t, J = 8 Hz, 1 H), 7.11 ppm (d, J = 8 Hz, 1 H).
MS: (M + H)⁺ = 261

Auf gleiche Weise wurde die folgende Verbindung der Formel I synthetisiert:

4-lsopropenyloxycarbonyl-2-(isopropenyloxycarbonyl)-thio-3,3,7,8-tetramethyl-3,4-dihydrochinoxalin.
Schmp.: 115 °C

### Beispiel XXV

### (3RS)-3-Methyl-4-N-(3-methyl-2-buten-1-yl)-3,4-dihydrochinoxalin-2(1H)-on

(3RS)-3-Methyl-3,4-dihydro-chinoxalin-2(1H)-on (4.86 g, 0.03 mol) gelöst in 50 ml N,N-Dimethylformamid wurde mit 4.2 ml (0.033 mol) 3-Methyl-2-buten-1-ylbromid (90 %-ig) in Gegenwart von 4.60 g (0.033 mol) gepulvertem Kaliumcarbonat alkyliert. Das Reaktionsgemisch wurde bei Raumtemperatur gerührt bis alles Edukt umgesetzt war. Dann wurde das Lösungsmittel im Vakuum abgezogen, der Rückstand in Essigsäureethylester und Wasser aufgenommen, die Phasen getrennt, die wäßrige zweimal mit Essigsäureethylester extrahiert und die vereinigten organischen Extrakte zweimal mit Wasser gewaschen. Trocknen über Natriumsulfat, Einengen im Vakuum und Kristallisieren aus Pentan ergab 5.80 g (84 %) weiß kristallines Produkt vom Schmp. 92 - 93 °C.
¹H-NMR (270 MHz, d₆-DMSO): δ = 0.99 (d, J = 7 Hz, 3 H), 1.72 (s, 6 H), 3.67 (dd, J = 15, 7 Hz, 1 H), 3.86 (q, J = 7 Hz, 1 H), 3.88 (dd, J = 15, 7 Hz, 1 H), 5.21 (m, 1 H), 6.65 - 6.9 (m, 4 H), 10.31 ppm (br. s, 1 H).
MS: (M + H)⁺ = 231

### * Beispiel XXVI

### 3,3a-Dihydropyrrolo[1,2-a]chinoxalin-1,4(2H,5H)-dion

Man erhitzte 2-Fluornitrobenzol (14.1 g, 0.1 mol) und L- Glutaminsäure (45.0 g, 0.3 Mol) in 100 ml 2-Methoxyethanol unter Rühren auf 95 °C und tropfte 300 ml 2N Natronlauge zu. Anschließend wurde noch 3 h bei dieser Temperatur weitergerührt.
Nach dem Abkühlen wurde die Lösung mit 400 ml Methanol versetzt und mit Raney-Nickel als Katalysator bei Normaldruck hydriert.
Nach beendeter Wasserstoffaufnahme saugte man den Katalysator ab und engte die Lösung unter vermindertem Druck ein.
Der Rückstand wurde mit 250 ml 2N Salzsäure angesäuert und ca. 30 Minuten auf dem Dampfbad erwärmt. Die dabei entstehende Fällung wurde abgesaugt, mit Wasser und Alkohol gewaschen und anschließend getrocknet, Schmp. 255 °C Zers..
¹H-NMR (60 MHz, d₆-DMSO): δ = 1.9 - 2.7 (m, 4 H), 4.5 (t, J = 8 Hz, 1 H), 6.8 - 7.3 (m, 3 H), 7.8 -8.2 (m, 1 H), 10.7 ppm (br. s, 1 H).
MS: M⁺ = 202

### * Beispiel XXVII

### 7-Phenoxysulfonyl-3,3a-dihydropyrrolo[1,2-a]chinoxalin-1,4(2H,5H)-dion

Die Verbindung wurde in analoger Weise durch Umsetzung von 4- Chlor-3-nitrobenzolsulfonsäurephenylester mit L-Glutaminsäure erhalten, Schmp. 140 °C (Zers.).
¹H-NMR (60 MHz, d₆-DMSO): δ = 1.6 - 2.5 (m, 4 H), 4.07 (t, J = 6 Hz, 1 H), 6.7 - 7.6 (m, 8 H), 10.57 ppm (br. s, 1 H).
MS: M⁺ = 358

### * Beispiel XXVIII

### 3-Carboxymethyl-3,4-dihydro-chinoxalin-2(1H)-on

Man erhitzte 2-Fluornitrobenzol (14.1 g, 0.1 mol) und L- Asparaginsäure (40.0 g, 0.3 Mol) in 100 ml 2-Methoxyethanol unter Rühren auf 95 °C und tropfte 300 ml 2N Natronlauge zu. Anschließend wurde noch 1 h bei dieser Temperatur weitergerührt.
Nach dem Abkühlen wurde die Lösung mit 500 ml Methanol versetzt und mit Raney-Nickel als Katalysator bei Normaldruck hydriert.
Nach beendeter Wasserstoffaufnahme saugte man den Katalysator ab und engte die Lösung unter vermindertem Druck ein.
Der Rückstand wurde mit 500 ml 2N Salzsäure angesäuert, anschließend eingeengt, mit Natriumacetat abgestumpft und mit Essigsäureethylester extrahiert. Nach Trocknen mit Natriumsulfat und Abziehen des Lösungsmittels erhielt man einen zunächst öligen Rückstand, der beim Verrühren mit Wasser kristallisierte, Schmp. 152 -154 °C.
¹H-NMR (60 MHz, d₆-DMSO): δ = 2.5 - 2.7 (dd tw. verdeckt, 2 H), 4.1 (td, J = 6, 2 Hz, 1 H), 5.98 (br. s, 1 H), 6.5 - 6.9 (m, 4 H), 10.30 (br. s, 1 H), 12.37 ppm (br. s, 1 H).
MS: M⁺ = 206

| | | | | |
|---|---|---|---|---|
| CHN-Analyse | berechnet | C 58.2; | H 4.8; | N 13.6 %, |
| | gefunden | C 58.4; | H 4.7; | N 13.7 % |

### * Beispiel XXIX

### 7-Phenoxysulfonyl-3,4-dihydro-chinoxalin-2(1H)-on

### A) N-[(2-Nitro-4-phenoxysulfonyl)phenyl]glycinmethylester

4-Chlor-3-nitrobenzolsulfonsäurephenylester (62.7 g, 0.2 mol) und Glycinmethylester Hydrochlorid (100.4 g, 0.8 mol), gelöst in 250 ml Methanol, wurden mit 200 ml Triethylamin versetzt und 15 min unter Rückfluß erhitzt. Nach dem Abkühlen wurde mit 1 I 2N Essigsäure versetzt, abgesaugt und mit Wasser gewaschen. Der Rückstand wurde aus Essigsäureethylester umkristallisiert und mit Methanol und Diisopropylether gewaschen, Schmp. 120 -123 °C.

### B) 7-Phenoxysulfonyl-3,4-dihydro-chinoxalin-2(1H)-on

N-[(2-Nitro-4-phenoxysulfonyl)phenyl]glycinmethylester (36.6 g, 0.1 mol) wurde in einem Gemisch aus 250 ml N,N- Dimethylformamid und 250 ml Methanol mit Raney-Nickel als Katalysator unter Normaldruck hydriert. Nach Beendigung der Wasserstoffaufnahme wurde der Katalysator abgesaugt und die Lösung im Vakuum vom Lösungsmittel befreit. Der Rückstand wurde in 40 ml 2-Methoxyethanol gelöst und eine Stunde auf dem Dampfbad erwärmt. Die entstehende Fällung wurde abgesaugt und mit Methanol gewaschen, Schmp. 253 - 254 °C.
¹H-NMR (60 MHz, d₆-DMSO): δ = 4.0 (d, J = 4 Hz, 2 H), 6.6 - 7.6 (m, 9 H), 10.43 ppm (br. s, 1 H).
MS: (M+H)⁺ = 305

### * Beispiel XXX

### 4-(3-Methyl-2-buten-1-yl)-7-phenoxysulfonyl-3,4-dihydro-chinoxalin-2(1H)-on

7-Phenoxysulfonyl-3,4-dihydro-chinoxalin-2(1H)-on (1.52 g, 5.0 mmol) wurde in 20 ml N,N-Dimethylacetamid mit 2 ml 3-Methyl-2- buten-1-ylbromid 8 h bei 100 °C gerührt. Nach dem Abkühlen wurde mit Wasser versetzt und mit Essigsäureethylester extrahiert. Nach Trocknen mit Magnesiumsulfat wurde die Lösung eingeengt und über eine Kieselgelsäule mit Essigsäureethylester / Heptan = 1 : 1 chromatographiert. Die substanzhaltigen Fraktionen wurden einrotiert, anschließend mit Pentan verrührt und abgesaugt, Schmp. 132 °C.
¹H-NMR (270 MHz, d₆-DMSO): δ = 1.73 (s, 6 H), 3.90 (s, 2 H), 3.93 (tw. verdecktes d, J = 6 Hz, 2 H), 5.20 (br. t, J = 6 Hz, 1 H), 6.75 - 7.45 (m, 8 H), 10.66 ppm (s, 1 H).
MS: (M+H)⁺ = 373

Die folgenden Verbindungen der Formel I wurden unter Verwendung der entsprechenden Halogenaromaten und Aminosäurederivate in analoger Weise synthetisiert und gegebenenfalls am Stickstoffatom-4 weiter derivatisiert:

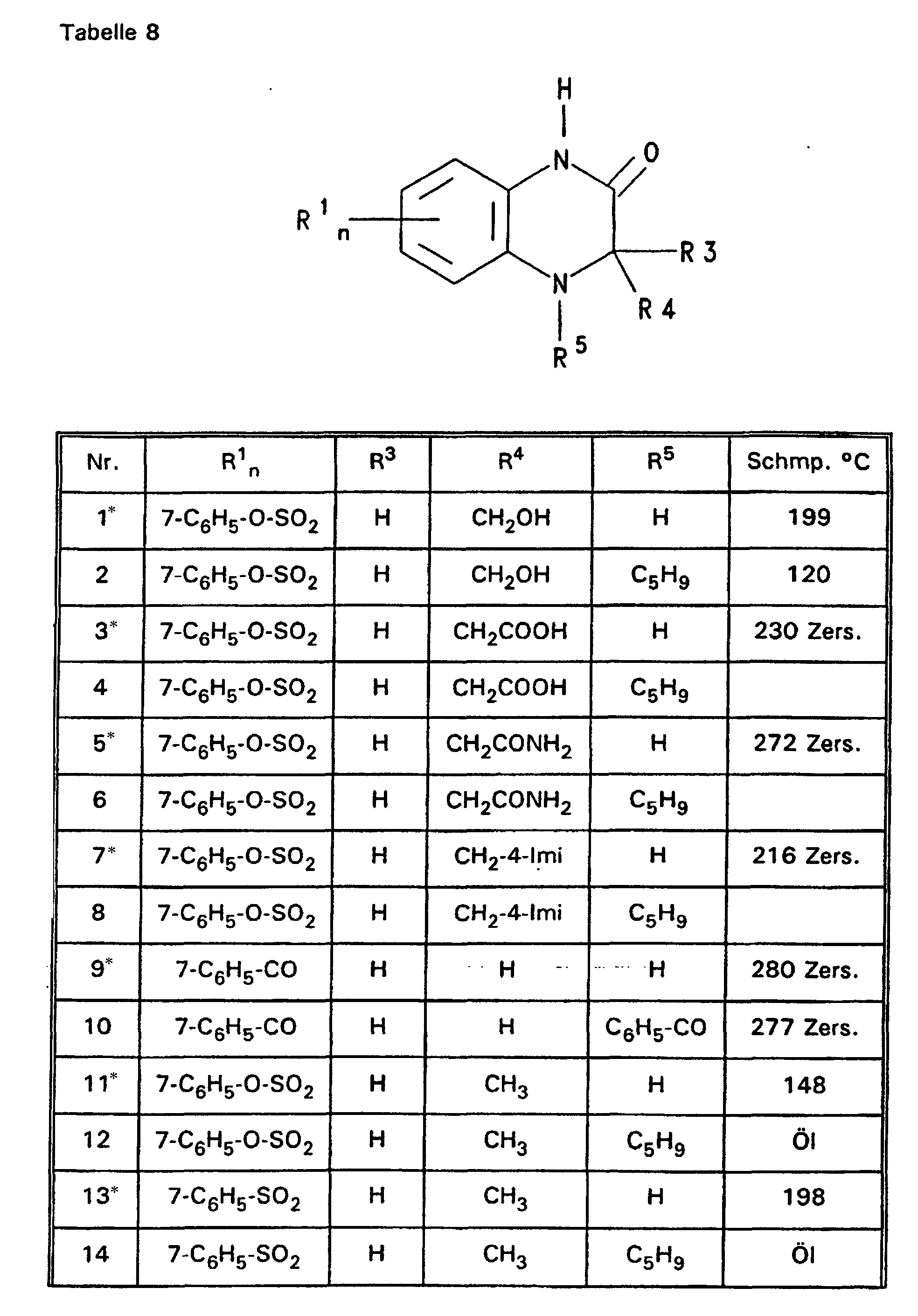

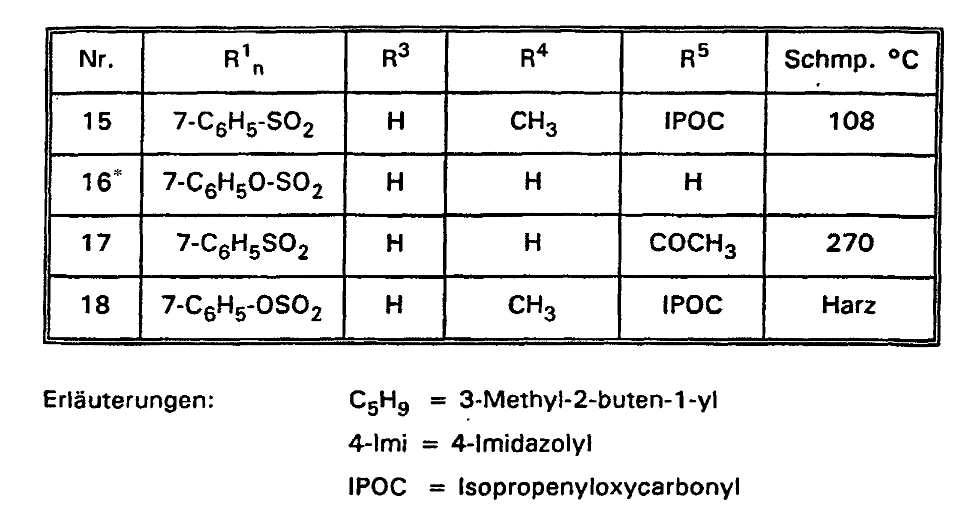

### * Beispiel XXXI

### 6-Chlor-7-phenoxysulfonyl-1,2,3,3a-tetrahydropyrrolo[2,1-c]-chinoxalin-4(5H)-on

### A) 2,4-Dichlor-3-nitrobenzolsulfonsäurephenylester

2,6-Dichlornitrobenzol wurde mit einem Überschuß Chlorsulfonsäure 7 h bei 130 °C gerührt. Nach dem Abkühlen goß man auf Eis, saugte das Sulfochlorid ab, wusch neutral und trocknete über Natriumhydroxid, Schmp. 91 °C. Das erhaltene Sulfochlorid (29.05 g, 0.1 mol) und Phenol (11.5 g, 0.12 mol) wurden in 150 ml Aceton gelöst und bei 10 °C mit 14 ml Triethylamin versetzt. Man rührte 1 h unter Kühlung und weitere 4 h bei Raumtemperatur nach, versetzte dann mit 200 ml Wasser, saugte die entstandene Fällung bei 10 °C ab, wusch mit Wasser und trocknete im Vakuum bei 80 °C, Schmp. 102 °C.

### B) N-[(3-Chlor-2-nitro-4-phenoxysulfonyl)phenyl]prolin

2,4-Dichlor-3-nitrobenzolsulfonsäurephenylester (34.8 g, 0.1 mol), 69.0 g (0.6 mol) L-Prolin, 200 ml 2N Natronlauge und 200 ml 2-Methoxyethanol wurden 10 min bei 80 °C gerührt. Die klare Lösung wurde bei 50 °C mit konzentrierter Salzsäure angesäuert und auf Eis gegossen. Die Fällung wurde abgesaugt, mit Wasser neutral gewaschen und bei 80 °C getrocknet. Schmp. 148 °C (nach Umkristallisation aus Methanol)

### C) 6-Chlor-7-phenoxysulfonyl-1,2,3,3a-tetrahydropyrrolo[2,1-c]-chinoxalin-4(5H)-on

N-[(3-Chlor-2-nitro-4-phenoxysulfonyl)phenyl]prolin (38.0 g, 0.075 mol) wurde in 500 ml Methanol und 25 ml conc. Ammoniaklösung mit Raney-Nickel als Katalysator unter Normaldruck hydriert.
Nach beendeter Wasserstoffaufnahme wurde der Katalysator abgesaugt und die Lösung eingeengt, mit 2N Salzsäure etwa 30 min auf dem Dampfbad erwärmt, abgekühlt, abgesaugt und mit Wasser neutral gewaschen. Schmp. 197 °C (nach Umkristallisation aus Eisessig)

### * Beispiel XXXII

### 8-(4-Methyl-1-piperazinyl)-3-(2-methylpropyl)-5-phenoxysulfonyl-3,4-dihydrochinoxalin-2(1 H)-on

### A) 2-Chlor-4-(4-methyl-1-piperazinyl)-3-nitrobenzolsulfonsäure- phenylester

2,4-Dichlor-3-nitrobenzolsulfonsäurephenylester (17.4 g, 0.05 mol) und 25 ml Methylpiperazin wurden in 100 ml Isopropanol 10 min unter Rückfluß erhitzt und anschließend eingeengt. Der Rückstand wurde mit 50 ml 50 %igem Methanol verrührt, abgesaugt, mit 50 %igem Methanol und zuletzt mit Wasser gewaschen. Schmp. 94 -95 °C (nach Umkristallisation aus Cyclohexan)

### B) N-[(3-(4-Methyl-1-piperazinyl)-2-nitro-6-phenoxysulfonyl)-phenyl]leucin Hydrochlorid

2-Chlor-4-(4-methyl-1-piperazinyl)-3-nitrobenzolsulfonsäure- phenylester (41.1 g, 0.1 mol) und L-Leucin (39.3 g, 0.3 mol) wurden in einem Gemisch aus 100 ml N,N-Dimethylformamid, 50 ml 2-Methoxyethanol und 100 ml 2N Natronlauge 8 h bei 95 °C gerührt. Das erkaltete Reaktionsgemisch wurde mit conc. Salzsäure angesäuert. Die Fällung wurde in Essigsäureethylester aufgenommen, mit Natriumsulfat getrocknet und im Vakuum vom Lösungsmittel befreit. Es resultierte ein orangefarbenes Öl.

### C) 8-(4-Methyl-1-piperazinyl)-3-(2-methylpropyl)-5-phenoxysulfonyl-3,4-dihydrochinoxalin-2(1H)-on Hydrochlorid

N-[(3-(4-Methyl-1-piperazinyl)-2-nitro-6-phenoxysulfonyl)- phenyl]leucin Hydrochlorid (25.3 g, 0.05 mol) wurde in 250 ml Methanol und 25 ml Eisessig mit Raney-Nickel als Katalysator unter Normaldruck hydriert.
Nach beendeter Wasserstoffaufnahme wurde der Katalysator abgesaugt und die Lösung eingeengt, mit 2N Salzsäure etwa 10 min auf dem Dampfbad erwärmt und dann im Vakuum eingeengt. Der Rückstand wurde in Wasser gelöst, mit Ammoniak alkalisch gestellt und in Essigsäureethylester aufgenommen. Das nach dem Einengen verbleibende Öl wurde in 400 ml Diisopropylether gelöst und mit ethanolischer Salzsäure neutralisiert. Die Fällung wurde abgesaugt, mit Diisopropylether gewaschen und getrocknet, Schmp. ab 90 °C (Zers.).
MS: M⁺ = 458

Die folgenden Verbindungen der Formel I wurden unter Verwendung der entsprechenden Halogenaromaten und Aminosäurederivate in analoger Weise synthetisiert und gegebenenfalls am Stickstoffatom-4 weiter derivatisiert:

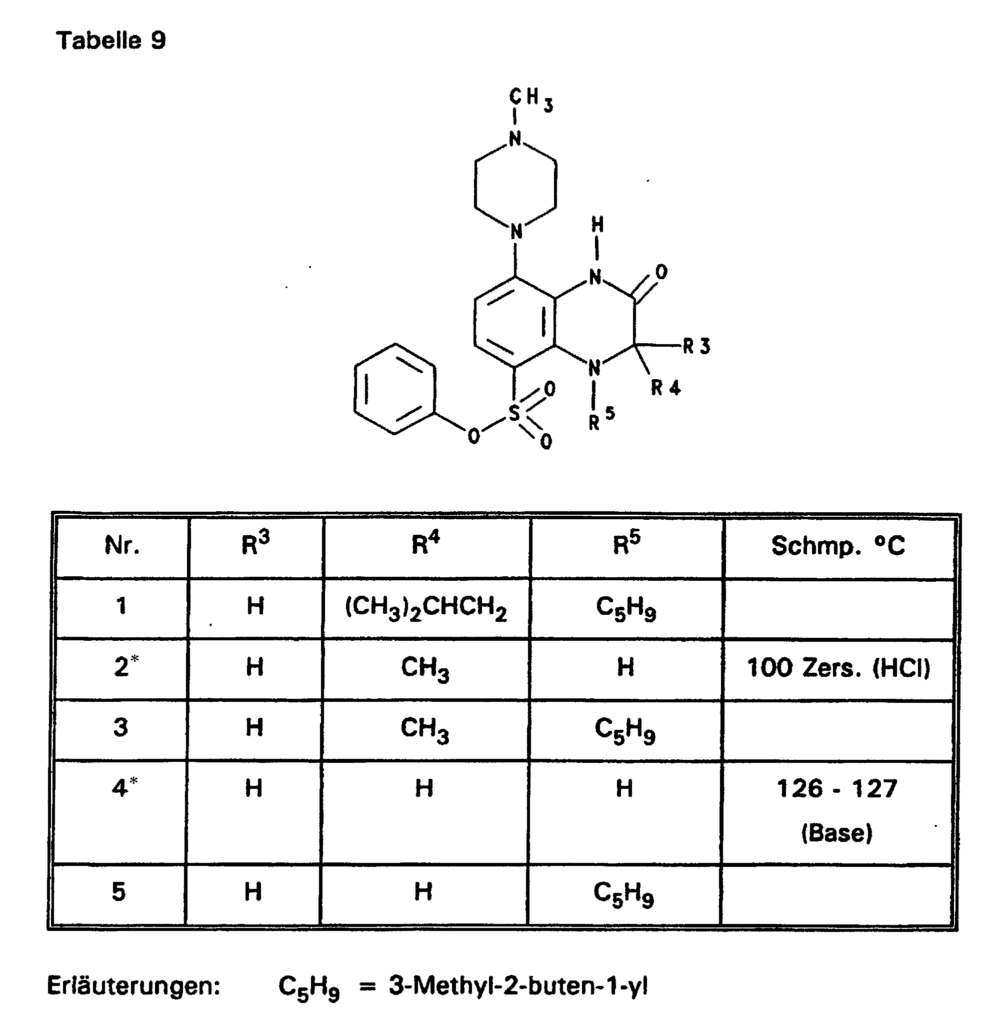

### Beispiel XXXIII

### (3RS)-4-N-Cyclohexyl-3-methyl-3,4-dihydrochinoxalin-2(H)-on

(3RS)-3-Methyl-3,4-dihydrochinoxalin-2(1H)-on (0,81 g, 0,005 mol) und 1 ml (0,1 mol) Cyclohexanon wurden in 20 ml 1,2-Dichlorethan vorgelegt.
Trifluoressigsäure (1,9 ml, 0,025 mol) wurde zugetropft, wobei unter leichtem Erwärmen eine klare Lösung entstand. Nach Zugabe von 2,1 g (0,01 mol) Natriumtriacetoxyborhydrid ließ man die exotherme Reaktion 30 min rühren und quenchte dann durch Zugabe von gesättigter wäßriger Natriumhydrogencarbonatlösung. Die Phasen wurden getrennt, die organische Phase mit gesättigter wäßriger Natriumchloridlösung nachgewaschen, getrocknet (Magnesiumsulfat) und eingeengt. Das Rohprodukt wurde an Kieselgel mit Essigsäureethylester/Heptan = 1 : 1 chromatrographiert. Man erhielt 1,15 g (94 %) des gewünschten Produkts, Schmp. 131 - 132 °C (Toluol/Heptan).
¹H-NMR (270 MHz, d₆-DMSO): δ = 0.97 (d, J = 7 Hz, 3 H), 1.0 - 2.0 (m, 10 H), 3.39 (m, 1 H), 3.91 (q, J = 7 Hz, 1 H), 6.68 - 6.94 (m, 4 H), 10.27 ppm (br. s, 1 H).
MS: (M+H)⁺ = 245.

Die folgenden Verbindungen der Formel I wurden in analoger Weise synthetisiert.

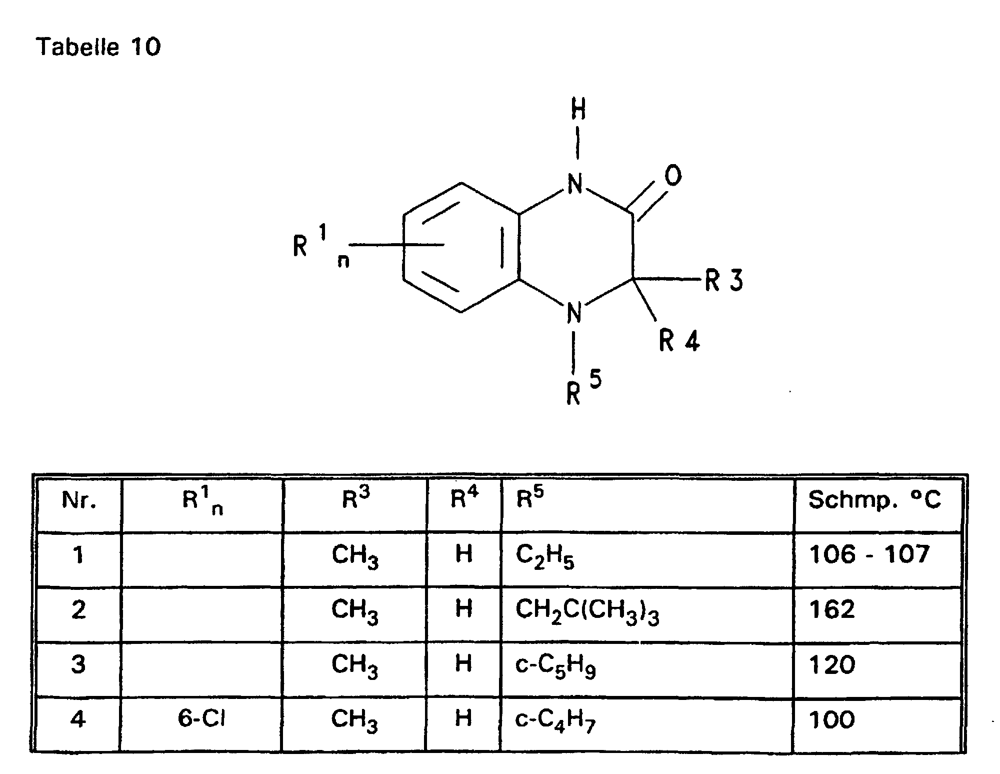

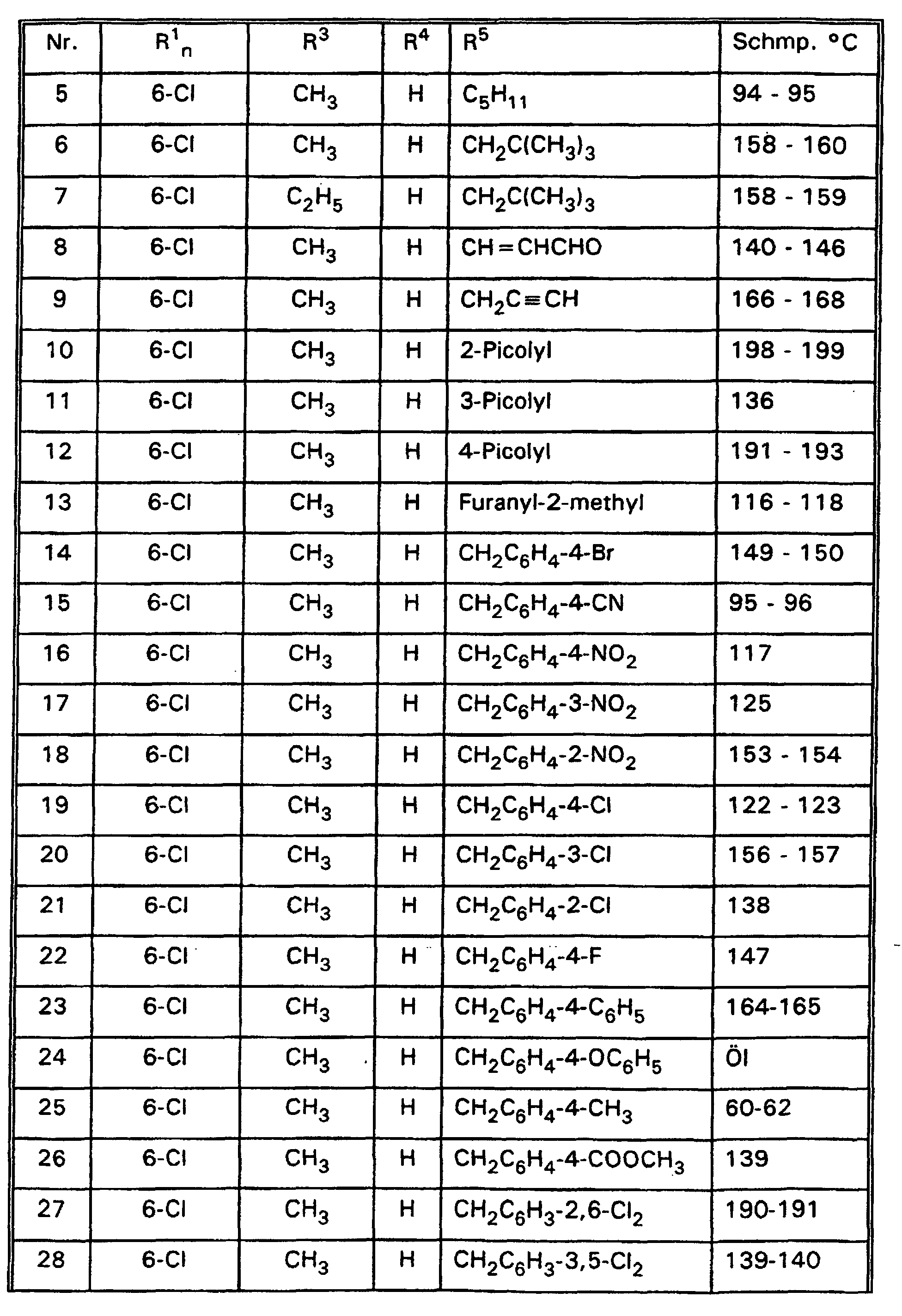

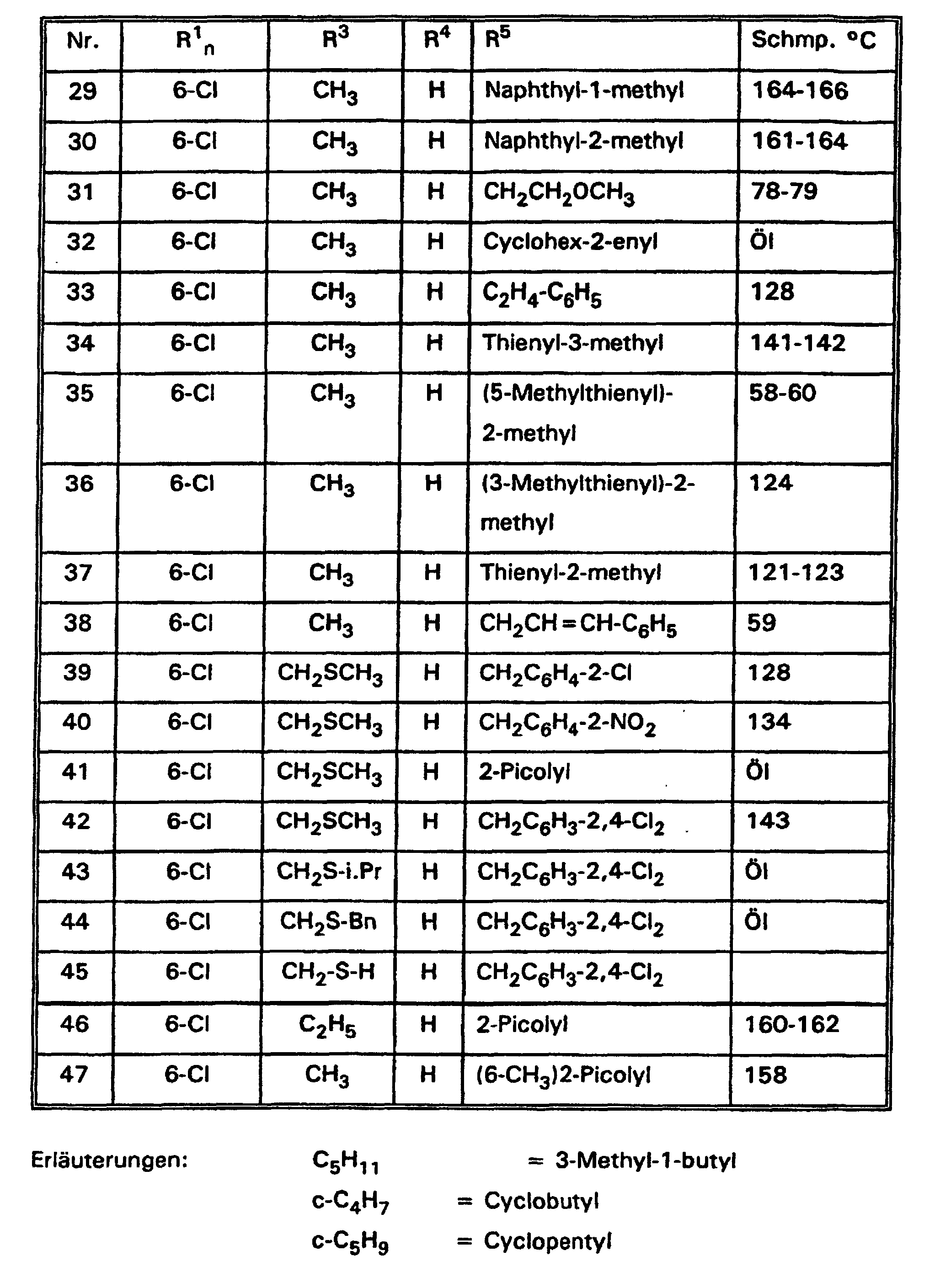

### Beispiel XXXIV

### (3RS)-3-Methyl-4-N-(3-oxo-1-butyl)-3,4-dihydrochinoxalin-2(1H)-on

3-Methyl-3,4-dihydrochinoxalin-2(1H)-on (0,5 g, 3,1 mmol) wurde zusammen mit 0,35 ml (4,3 mmol) Methylvinylketon und einer katalytischen Menge Triethylamin in 20 ml wasserfreiem Ethanol für 20 h bei Raumtemperatur gerührt. Nach Chromatographie an Kieselgel mit Methyl-tert.-butylether/Heptan = 2:1 erhielt man 620 mg (87 %) des gewünschten Produkts, Schmp. 108-109 °C (Methyl-tert.-butylether/Heptan).
¹H.NMR (270 MHz, d₆-DMSO): δ = 1,03 (d, J = 7 Hz, 3 H), 2,11 (s, 3H), 2,77 (t, J = 6 Hz, 2 H), 3,30 (m, 1 H), 3,50 (m, 1 H), 3,88 (q, J = 7 Hz, 1 H), 6,68 (m, 1 H), 6,78 (m, 1 H), 6,88 (m, 1 H), 10,31 ppm (br. s, 1 H).
MS : (M + H)⁺ = 233, M⁺ = 232

### Beispiel XXXV

### (3S)-6-Chlor-4-N-Chlorcarbonyl-3-methyl-3,4-dihydrochinoxalin-2(1H)-on

Die Verbindung des Beispiels IB (2,0 g, 0,01 mol) wurde in 100 ml wasserfreiem Toluol in Gegenwart von 2 ml (0,014 mol) Triethylamin mit Bis-(trichlormethyl)-carbonat (Triphosgen) (1,5 g, 0,005 mol) für 1 h auf 80 °C erhitzt. nach dem Abkühlen wurde mit Wasser und gesättigter wäßriger Natriumchloridlösung gewaschen, getrocknet (Magnesiumsulfat) und das Lösungsmittel unter vermindertem Druck entfernt. Der Rückstand (2,5 g) kristallisierte nach Anrühren mit Heptan und war für präparative Zwecke rein genug.
Eine analysenreine Probe erhielt man durch Chromatographie an Kieselgel mit Essigsäureethylester/Heptan = 1 : 1 als Elutionsmittel. Schmp. 142-144 °C.
¹H-NMR (270 MHz, d₆-DMSO): 1,25 (d, J = 7 Hz, 3 H), 3,83 (q, J = 7 Hz, 1 H), 6,61 (dd, J = 6, 2 Hz, 1 H), 6,70 (s, 2H), 10,3 ppm (br. s, 1 H).
MS: (M + H)⁺ = 259

### Beispiel XXXVI

### (3S)-6-Chlor-4-N-(2-methoxyethoxycarbonyl)-3-methyl-3,4-dihydrochinoxalin-2(1H)-on

Zu einer Lösung von 0,24 ml (3,0 mmol) 2-Methoxyethanol in 10 ml wasserfreiem 1,2-Dimethoxyethan wurde 0,16 g einer 55 %igen Suspension von Natriumhydrid in Mineralöl zugegeben und die Reaktionsmischung bei Raumtemperatur für 30 min gerührt. Unter Eiskühlung gab man anschließend 0,50 g (1,9 mmol) der Verbindung des Beispiels XXXV zu, ließ auf Raumtemperatur erwärmen und weitere 30 min rühren. Man versetzte mit gesättigter wäßriger Natriumchloridösung, extrahierte mehrfach mit Essigsäureethylester, wusch die organische Phase einmal mit gesättigter wäßriger Natriumchloridlösung, trocknete (Magnesiumsulfat) und entfernte das Lösungsmittel im Vakuum. Nach Chromatographie an Kieselgel (Essigsäureethylester/Heptan = 1 : 1) und Kristallisation aus Ether/Heptan erhielt man 0,29 g (51 %) des gewünschten Produkts, Schmp. 93-94 °C.
¹H-NMR (200 MHz, d₆-DMSO): δ = 1,13 (d, JJ = 7,5 Hz, 3 H), 3,32 (s, 3 H), 3,6 (m, 2H), 4,24 (m, 1 H), 4,35 (m, 1 H), 4,81 (q, J = 7,5 Hz, 1 H), 6,98 (d, J = 9 Hz), 1 H), 7,2 (dd, J = 9, 3 Hz, 1 H), 7,66 (d, J = 3 Hz, 1 H), 10,81 ppm (br. 2, 1 H).
MS: (M + H)⁺ = 299

### Beispiel XXXVII

### (3S)-6-Chlor-3-methyl-4-N-[(phenylthio)carbonyl)]-3,4-dihydrochinoxalin-2(1H)-on

Zu einer Lösung von 0,31 ml (3,0 mmol) Thiophenol in 10 ml 1,2-Dimethoxyethan wurden unter Eiskühlung 0,17 g einer 55 %-igen Suspension von Natriumhydrid in Mineralöl zugegeben und 1 h bei Raumtemperatur gerührt. Wieder unter Eiskühlung wurden 0,5 g (1,9 mmol) der Verbindung des Beispiels XXXV eingetragen und anschließend 2 h bei Raumtemperatur nachgerührt. Zur Aufarbeitung wurde mit gesättigter wäßriger Natriumchloridlösung versetzt, zweimal mit Essigsäureethylester extrahiert, getrocknet (Natriumsulfat) und das Lösungsmittel abgezogen. Der feste Rückstand wurde aus Heptan/Isopropanol umkristallisiert, 0,35 g (35 %), Schmp. 194 - 195 °C.
¹H-NMR (200 MHz, d₆-DMSO): δ = 1,10 (d, J = 7 Hz, 3 H), 4,93 (q, J = 7 Hz, 1 H), 7.08 (d, J = 9 Hz, 1 H), 7,33 (dd, J = 9,3 Hz, 1 H), 7,4 - 78,6 (m, 5 H), 7,78 (d, J = 3 Hz, 1 H), 10,16 ppm (br. s, 1 H).
MS: (M + H)⁺ = 333, (M - C₆H₅SH + H)⁺ 223

Die folgenden Verbindungen der Formel I wurden in analoger Weise synthetisiert.

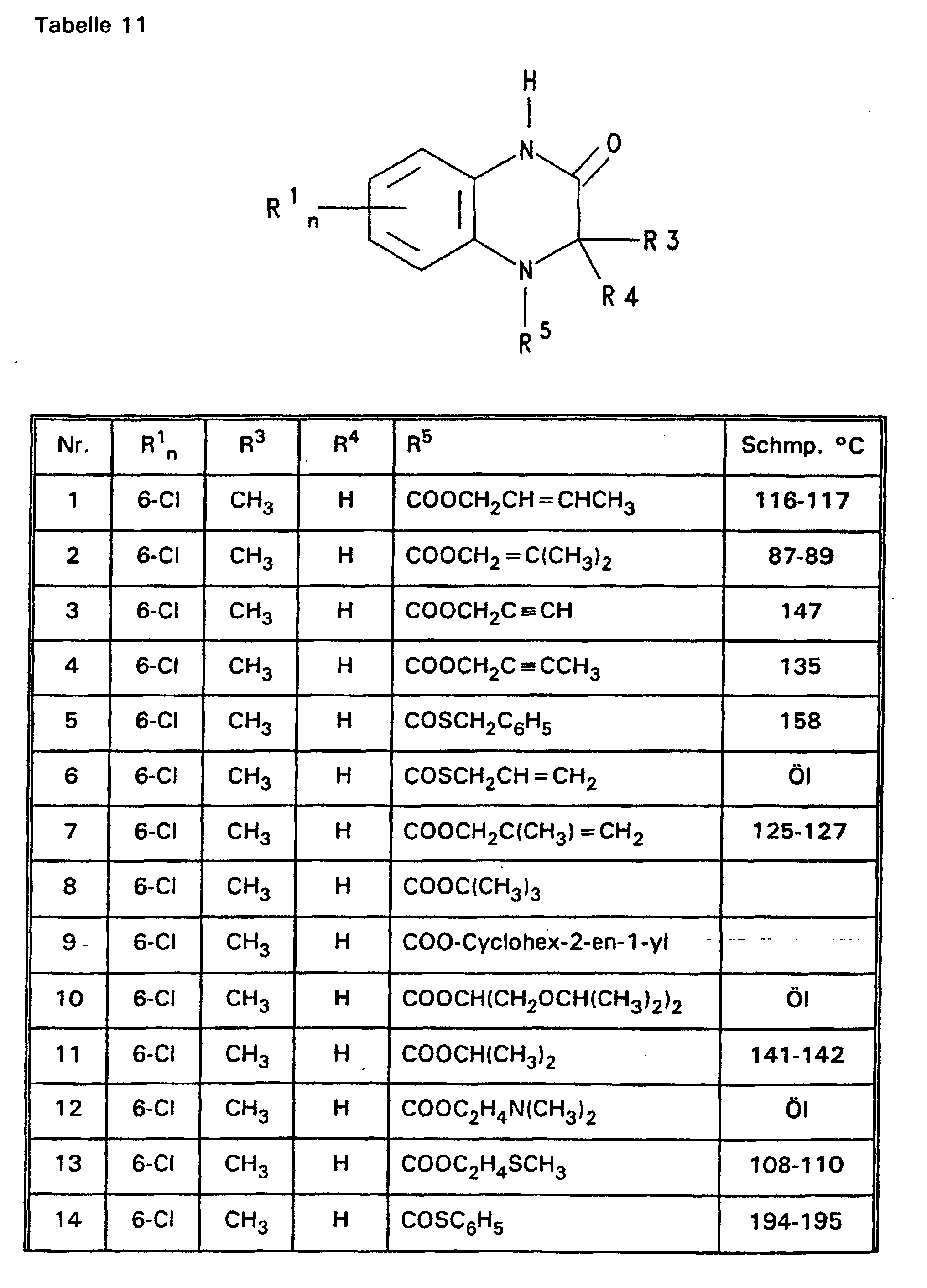

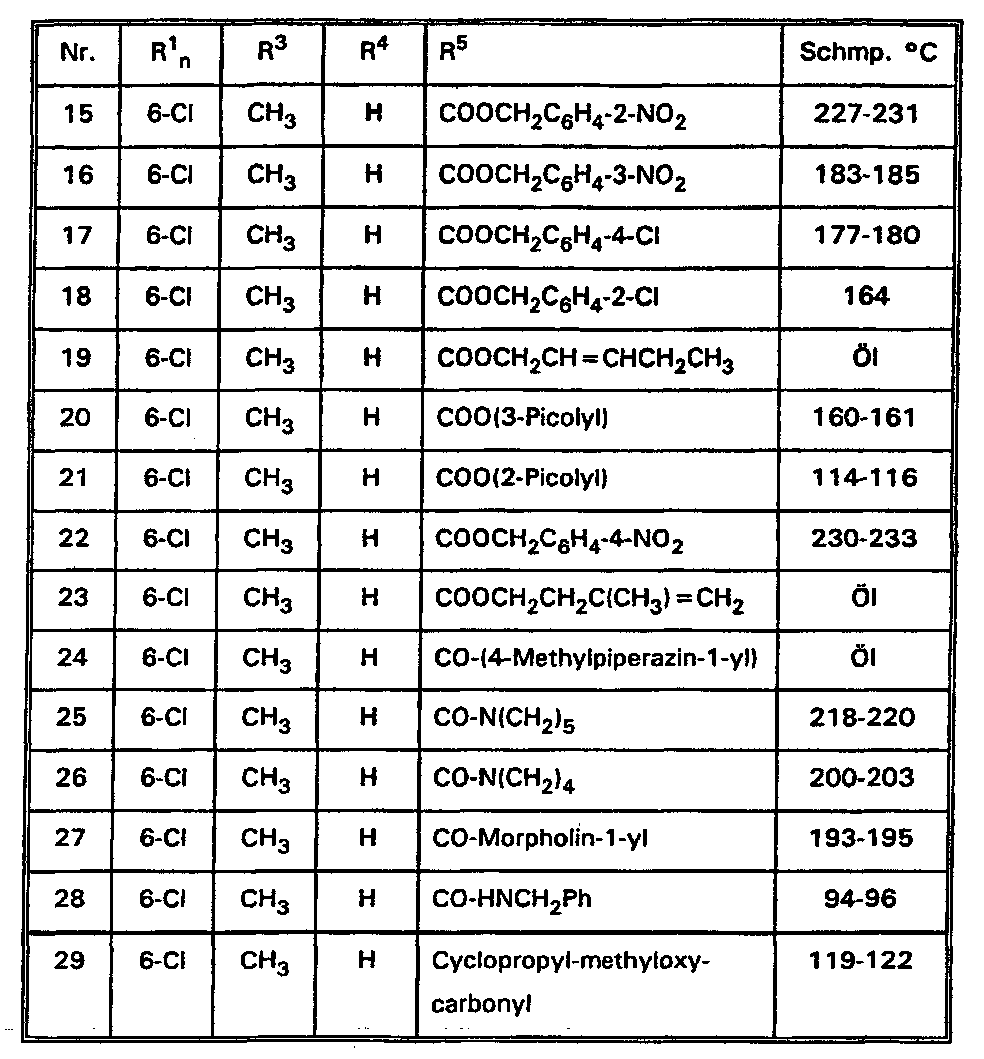

Eine besondere Bedeutung für die vorliegende Erfindung besitzt die Verbindung 31 der Tabelle 7.

## Patentansprüche

1. Kombinationspräparat enthaltend mindestens ein Nukleosid und mindestens eine Verbindung der Formeln I und/oder la, 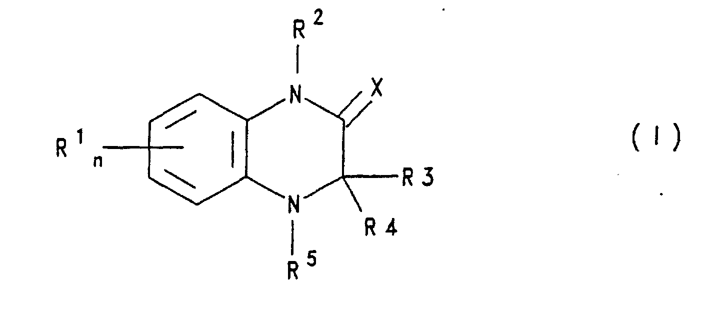 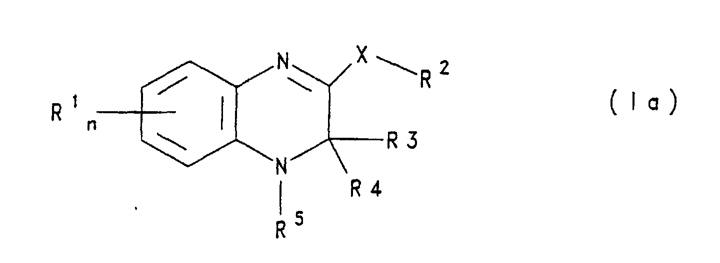 bzw. deren physiologisch verträgliche Salze
oder Prodrugs, wobei in den Formeln I und la bedeuten
n null,
eins
oder zwei,
die einzelnen Substituenten R¹ unabhängig voneinander
Fluor, Chlor, Brom, Trifluormethyl, Hydroxy, C1 - C4-Alkyl,
C1 - C4-Alkoxy, (C1 - C4-Alkoxy)-(C1 - C4-alkoxy), C1- C4-Alkylthio, Nitro, Amino, C1 - C4-Alkylamino, Di(C1 - C4-alkyl)amino, Piperidino, Morpholino, 1-Pyrrolidinyl, 4-Methylpiperazinyl, C1 - C4-Acyl,
C1 - C4-Acyloxy, C1 - C4-Acylamino, Cyano, Carbamoyl, Carboxy, (C1 - C4-Alkyl)-oxycarbonyl, Hydroxysulfonyl, Sulfamoyl
oder
einen mit bis zu zwei
voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C1 - C4-Alkyl, C1 - C4-Alkoxy, (C1 - C4-Alkyl)-oxycarbonyl, Phenyl, Phenoxy,
sein kann,
R² Wasserstoff und R⁵
C1 - C6-Alkyl,
gegebenenfalls substituiert mit Fluor,
Chlor, Hydroxy, C1 - C4-Acyloxy, Benzoyloxy, Benzyloxy, Phenoxy,
C1 - C4-Alkoxy, C1 - C4-Alkylamino, Di(C1 - C4-alkyl)amino,
C1 - C4-Alkylthio, Oxo, Thioxo, Carboxy, Carbamoyl;
C2 - C6-Alkenyl,
gegebenenfalls substituiert mit Fluor,
Chlor, Hydroxy, C1 - C4-Acyloxy, Benzoyloxy, Benzyloxy, Phenoxy,
C1 - C4-Alkoxy, C1 - C4-Alkylamino, Di(C1 - C4-alkyl)amino,
C1 - C4-Alkylthio, Oxo, Thioxo, Carboxy, Carbamoyl;
C3 - C8-Allenyl,
C3 - C8-Alkinyl,
gegebenenfalls substituiert mit Fluor,
Chlor, Hydroxy, C1 - C4-Acyloxy, Benzoyloxy, Benzyloxy, Phenoxy,
C1 - C4-Alkoxy, C1 - C4-Alkylamino, Di(C1 - C4-alkyl)amino,
C1 - C4-Alkylthio, Oxo, Thioxo, Carboxy, Carbamoyl;
C3 - C8-Cycloalkyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C3 - C8-Cycloalkenyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
(C3 - C6-Cycloalkyl)-(C1 - C2-alkyl),
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
(C3 - C6-Cycloalkenyl)-(C1 - C2-alkyl),
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C1 - C6-Alkylcarbonyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, C1 - C4-Alkenylamino, Di(C1 - C4-alkyl)amino, 1-Pyrrolidinyl, Piperidino, Morpholino, 4-Methylpiperazin-1-yl, C1 - C4-Alkylthio, Oxo, Thioxo, Carboxy, Carbamoyl;
C2 - C6-Alkenylcarbonyl, gegebenenfalls substituiert durch Fluor, Chlor oder Hydroxy;
(C3 - C6-Cycloalkyl)carbonyl,
(C5 - C6-Cycloalkenyl)carbonyl,
(C3 - C6-Cycloalkyl)-(C1 - C2-alkyl)carbonyl,
(C5 - C6-Cycloalkenyl)-(C1 - C2-alkyl)carbonyl,
C1 - C6-Alkyloxycarbonyl, gegebenenfalls substituiert durch Fluor, Chlor, Brom, Hydroxy, C1 - C4-Alkoxy, C1 - C4-Alkylamino,
Di(C1 - C4-alkyl)amino, C1 - C4-Alkylthio;
C2 - C6-Alkenyloxycarbonyl, gegebenenfalls substituiert durch Fluor, Chlor, Hydroxy, C1 - C4-Alkoxy;
C2 - C6-Alkinyloxycarbonyl, gegebenenfalls substituiert durch Fluor, Chlor, Hydroxy, C1 - C4-Alkoxy;
C1 - C6-Alkylthiocarbonyl, gegebenenfalls substituiert durch Fluor, Chlor, Hydroxy, C1 - C4-Alkoxy;
C2 - C6-Alkenylthiocarbonyl, gegebenenfalls substituiert durch Fluor, Chlor, Hydroxy, C1 - C4-Alkoxy;
C1 - C6-Alkylamino- und Di(C1 - C6-alkyl)aminocarbonyl, gegebenenfalls substituiert durch Fluor, Chlor, Hydroxy, C1 - C4-Alkoxy;
Pyrrolidin-1-yl, Morpholino-, Piperidino-, Piperazinyl-, oder 4-Methylpiperazin-1-yl-carbonyl;
C2 - C6-Alkenylamino- und Di(C1 - C6-alkenyl)aminocarbonyl, gegebenenfalls substituiert durch Fluor, Chlor, Hydroxy, C1 - C4-Alkoxy;
C1 - C4-Alkylsulfonyl, gegebenenfalls substituiert durch Fluor, Chlor, Hydroxy, C1 - C4-Alkoxy;
C1 - C4-Alkenylsulfonyl;
oder mit bis zu zwei voneinander unabhängigen Resten R⁶ substituiertes Aryl, Arylcarbonyl, (Arylthio)carbonyl, Aryloxycarbonyl, Arylaminocarbonyl, (Arylamino)thiocarbonyl, Arylsulfonyl, Arylalkylaminocarbonyl, Arylalkyl, Arylalkenyl, Arylalkylcarbonyl, Arylalkoxycarbonyl, Aryl(alkylthio)carbonyl, wobei der Alkylrest jeweils 1 bis 3 C-Atome enthalten kann und R⁶ wie oben definiert ist
oder mit bis zu zwei voneinander unabhängigen Resten R⁶ substituiertes 1- oder 2-Naphthylmethyl, 2-, 3- oder 4-Picolyl, 2- oder 3-Furylmethyl, 2-oder 3-Thienylmethyl, 2- oder 3-Pyrrolylmethyl,
2-, 3- oder 4-Pyridylcarbonyl, 2- oder 3-Furylcarbonyl, 2- oder 3-Thienylcarbonyl, 2- oder 3-Thienylacetyl, 2-, 3- oder 4-Picolyloxycarbonyl, 2- oder 3-Furylmethyloxycarbonyl, 2- oder 3-Thienylmethyloxycarbonyl,
und
R³ und R⁴ gleich oder verschieden, unabhängig voneinander Wasserstoff, C1 - C4-Alkyl, gegebenenfalls substituiert mit Fluor, Chlor, Hydroxy, Amino, Mercapto, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
C2 - C6-Alkenyl, gegebenenfalls substituiert mit Fluor oder Chlor;
C3 - C6-Cycloalkyl, gegebenenfalls substituiert mit Fluor, Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
C3 - C8-Cycloalkenyl, gegebenenfalls substituiert mit Fluor oder Chlor;
mit bis zu zwei voneinander unabhängigen Resten R⁶ substituiertes Aryl, Benzyl, Heteroaryl oder Heteroarylmethyl bedeuten,
R³ und R⁴ können ferner auch
Teil eines gesättigten oder ungesättigten carbo- oder heterocyclischen Ringes mit 3 bis 6 C-Atomen sein, der gegebenenfalls mit Fluor, Chlor, Hydroxy, Amino, C1 - C4-Acyloxy, Benzoyloxy, C1 - C4-Alkoxy, Oxo, Thioxo, Carboxy, Carbamoyl substituiert sein kann, und
X bedeutet Sauerstoff oder Schwefel.
**dadurch gekennzeichnet, daß** das Nukleosid ausgewählt ist aus der Gruppe Zidovudin und Lamivudin.

2. Kombinationspräparat gemäß Anspruch 1, **dadurch gekennzeichnet, daß** die Substituenten der Verbindungen der Formel I bzw. la die folgende Bedeutung haben
n null,
eins
oder zwei,
die einzelnen Substituenten R¹ unabhängig voneinander
Fluor, Chlor, Brom, Trifluormethyl, Hydroxy, C1 - C4-Alkyl, C1 - C4-Alkoxy, (C1 - C4-Alkoxy)-(C1 - C2-alkoxy), C1- C4-Alkylthio, Nitro, Amino, C1 - C4-Alkylamino, Di(C1 - C4-alkyl)amino, Piperidino, Morpholino, 1-Pyrrolidinyl, 4-Methylpiperazinyl, C1 - C4-Acyl, C1 - C4-Acyloxy, C1 - C4-Acylamino, Cyano, Carbamoyl, Carboxy, (C1 - C4-Alkyl)-oxycarbonyl, Hydroxysulfonyl, Sulfamoyl
oder
einen mit bis zu zwei
voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C1 - C4-Alkyl, C1 - C4-Alkoxy, (C1 - C4-Alkyl)-oxycarbonyl, Phenyl, Phenoxy
sein kann,
R² Wasserstoff und R⁵
C1 - C6-Alkyl
gegebenenfalls substituiert mit C1 - C4-Alkoxy oder C1 - C4-Alkylthio;
C2 - C6-Alkenyl,
gegebenenfalls substituiert mit Oxo;
C3 - C6-Allenyl;
C3 - C8-Alkinyl, insbesondere 2-Butinyl;
C3 - C6-Cycloalkyl;
C5 - C6-Cycloalkenyl;
(C3 - C6-Cycloalkyl)-(C1 - C2-alkyl), insbesondere Cyclopropylmethyl, gegebenenfalls substituiert mit C1 - C4-Alkyl;
(C3 - C6-Cycloalkenyl)-(C1 - C2-alkyl), insbesondere Cyclohexenylmethyl;
C1 - C6-Alkylcarbonyl,
gegebenenfalls substituiert mit Fluor,
Chlor, Hydroxy, Benzyloxy, Phenoxy, C1 - C4-Alkoxy,
C1 - C4-Alkylamino, C1 - C4-Alkenylamino, Di(C1 - C4-alkyl)amino, 1-Pyrrolidinyl, Piperidino, Morpholino, 4-Methylpiperazin-1-yl,
C1 - C4-Alkylthio;
C2 - C6-Alkenylcarbonyl;
C1 - C6-Alkyloxycarbonyl, gegebenenfalls substituiert durch Fluor, Chlor, Brom, Hydroxy, C1 - C4-Alkoxy, C1 - C4-Alkylamino,
Di(C1 - C4-alkyl)amino, C1 - C4-Alkylthio;
C2 - C6-Alkenyloxycarbonyl, insbesondere Vinyloxycarbonyl, Allyloxycarbonyl, Isopropenyloxycarbonyl, Butenyloxycarbonyl, Pentenyloxycarbonyl;
C2 - C6-Alkinyloxycarbonyl, insbesondere Propinyloxycarbonyl, Butinyloxycarbonyl;
C1 - C6-Alkylthiocarbonyl;
C2 - C6-Alkenylthiocarbonyl, insbesondere Allylthiocarbonyl;
C1 - C6-Alkylamino- und Di(C1 - C6-alkyl)aminocarbonyl;
Pyrrolidin-1-yl, Morpholino-, Piperidino-, Piperazinyl-, oder 4-Methylpiperazin-1-yl-carbonyl;
C2 - C6-Alkenylamino- und Di(C1 - C6-alkenyl)aminocarbonyl;
C1 - C4-Alkylsulfonyl;
C1 - C4-Alkenylsulfonyl;
oder mit bis zu zwei voneinander unabhängigen Resten R⁶ substituiertes Aryl, insbesondere Phenyl,
Arylcarbonyl, insbesondere Benzoyl, (Arylthio)carbonyl, Aryloxycarbonyl, Arylaminocarbonyl, (Arylamino)thiocarbonyl, Arylalkylaminocarbonyl, Arylsulfonyl,
Arylalkyl, insbesondere Benzyl, Phenylethyl, Arylalkenyl,
Arylalkylcarbonyl, Arylalkoxycarbonyl, Aryl(alkylthio)carbonyl, wobei der Alkylrest jeweils 1 bis 3 C-Atome enthalten kann und R⁶ wie oben definiert ist
oder mit bis zu zwei voneinander unabhängigen Resten R⁶ substituiertes 1- oder 2-Naphthylmethyl, 2-, 3- oder 4-Picolyl, 2- oder 3-Furylmethyl, 2-oder 3-Thienylmethyl, 2- oder 3-Pyrrolylmethyl,
2-, 3- oder 4-Pyridylcarbonyl, 2- oder 3-Furylcarbonyl, 2- oder 3-Thienylcarbonyl, 2- oder 3-Thienylacetyl, 2-, 3- oder 4-Picolyloxycarbonyl, 2- oder 3-Furylmethyloxycarbonyl, 2- oder 3-Thienylmethyloxycarbonyl,
und
R³ und R⁴ gleich oder verschieden, unabhängig voneinander
Wasserstoff,
C1 - C4-Alkyl,
gegebenenfalls substituiert mit Hydroxy, Mercapto,
C1 - C4-Alkoxy, C1 - C4-Alkylthio, C1 - C4-Alkylsulfonyl, C1-C4-Alkylsulfinyl, Carboxy, Carbamoyl;
C2 - C6-Alkenyl,
mit bis zu zwei voneinander unabhängigen Resten R⁶ substituiertes Aryl, Benzyl, Thienyl oder Thienylmethyl, wobei R⁶ wie oben definiert ist,
bedeuten,
R³ und R⁴ können auch
Teil eines gesättigten oder ungesättigten carbo- oder heterocyclischen Ringes mit 3 bis 6 C-Atomen sein, der gegebenenfalls mit Oxo oder Thioxo substituiert sein kann und
X bedeutet Sauerstoff oder Schwefel.

3. Kombinationspräparat gemäß Anspruch 1 oder 2, **dadurch gekennzeichnet, daß** die Substituenten der Verbindungen der Formel I bzw. la die folgende Bedeutung haben
n null oder
eins
die einzelnen Substituenten R¹ unabhängig voneinander
Fluor, Chlor, Brom, C1 - C2-Alkyl, C1 - C2-Alkoxy, C2 - C4-Acyl, Cyano
R² Wasserstoff und R⁵
C2-C6-Alkenyl
C3 - C8-Alkinyl, insbesondere 2-Butinyl;
(C3 - C6-Cycloalkyl)-(C1 - C2-alkyl), insbesondere Cyclopropylmethyl, gegebenenfalls substituiert mit C1 - C4-Alkyl;
(C3 - C6-Cycloalkenyl)-(C1 - C2-alkyl), insbesondere Cyclohexenylmethyl;
C2 - C6-Alkylcarbonyl,
C2 - C6-Alkenylcarbonyl;
C1 - C6-Alkyloxycarbonyl;
C2 - C6-Alkenyloxycarbonyl, insbesondere Vinyloxycarbonyl, Allyloxycarbonyl, Isopropenyloxycarbonyl, Butenyloxycarbonyl, Pentenyloxycarbonyl;
C2 - C6-Alkinyloxycarbonyl, insbesondere Propinyloxycarbonyl, Butinyloxycarbonyl;
C2 - C6-Alkenylthiocarbonyl, insbesondere Allylthiocarbonyl;
C1 - C4-Alkylsulfonyl;
C1 - C4-Alkenylsulfonyl;
oder mit bis zu zwei voneinander unabhängigen Resten R⁶ substituiertes Arylalkyl, insbesondere Benzyl oder Arylalkenyl, wobei der Alkylrest jeweils 1 bis 3 C-Atome und der Alkenylrest 2-3 C-Atome enthalten kann
oder mit bis zu zwei voneinander unabhängigen Resten R⁶ substituiertes 1-Naphthylmethyl, 2- oder 3-Picolyl, 2-Furylmethyl, 2- oder 3-Thienylmethyl,
wobei R⁶
Fluor, Chlor, Brom, Cyano, C1 - C2-Alkyl, C1 - C2-Alkoxy
und
R³ und R⁴ gleich oder verschieden, unabhängig voneinander
Wasserstoff,
C1 - C4-Alkyl,
gegebenenfalls substituiert mit Hydroxy, Mercapto, C1 - C4-Alkoxy, C1 - C2-Alkylthio und X bedeutet Sauerstoff oder Schwefel.

4. Kombinationspräparat gemäß einem oder mehreren der Ansprüche 1 - 3, **dadurch gekennzeichnet,**
**daß** es Zidovudin und S-4-lsopropoxycarbonyl-6-methoxy-3-(methylthio-methyl)-3,4-dihydro-chinoxalin-2(1H)-thion enthält.

5. Arzneimittel, enthaltend eine wirksame Menge eines Kombinationspräparates gemäß einem oder mehreren der Ansprüche 1-4 ggf. neben üblichen Hilfsund/oder Trägerstoffen.

6. Kombinationspräparat gemäß einem oder mehreren der Ansprüche 1-4 zur Anwendung als Arzneimittel.

7. Kombinationspräparat gemäß einem oder mehreren der Ansprüche 1-4 zur Herstellung von Arzneimitteln zur Behandlung von Viruserkrankungen.

8. Verfahren zur Herstellung eines Arzneimittels, **dadurch gekennzeichnet, daß** ein Kombinationspräparat gemäß einem oder mehreren der Ansprüche 1-4 ggf. mit üblichen Hilfs- und/oder Trägerstoffen in eine geeignete Darreichungsform gebracht werden.

## Claims

1. A combination preparation comprising at least one nucleoside and at least one compound of the formula I and/or Ia, 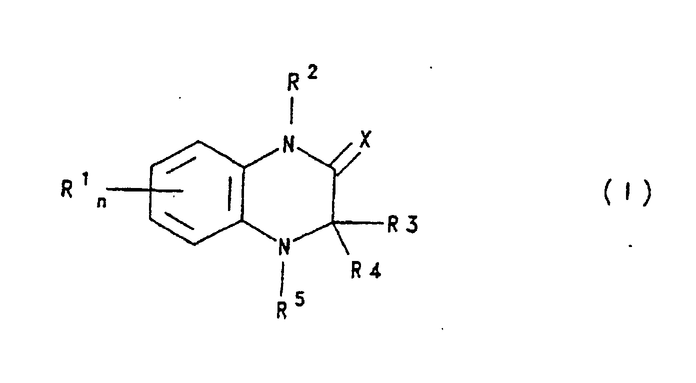 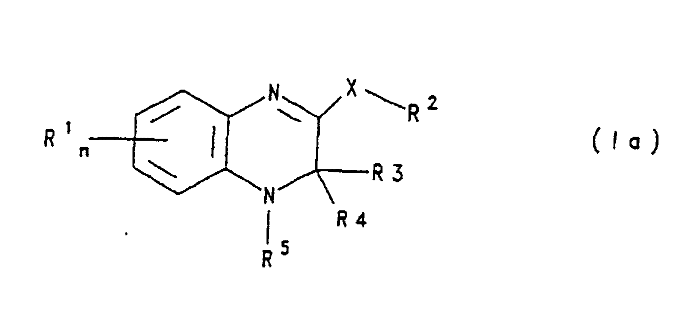 and physiologically acceptable salts and prodrugs thereof, where, in formulae I and Ia,
n is zero,
one
or two,
the individual substituents R¹ independently of one another are
fluorine, chlorine, bromine, trifluoromethyl, hydroxyl, C₁-C₄-alkyl, C₁-C₄-alkoxy, (C₁-C₄-alkoxy)-(C₁-C₄-alkoxy), C₁-C₄-alkylthio, nitro, amino, C₁-C₄-alkylamino, di (C₁-C₄-alkyl) amino, piperidino, morpholino, 1-pyrrolidinyl, 4-methylpiperazinyl, C₁-C₄-acyl, C₁-C₄-acyloxy, C₁-C₄-acylamino, cyano, carbamoyl, carboxyl, (C₁-C₄-alkyl)oxycarbonyl, hydroxysulfonyl or sulfamoyl,
or
a phenyl, phenoxy, phenylthio, phenylsulfonyl, phenoxysulfonyl, benzoyl, 2-pyridyl, 3-pyridyl or 4-pyridyl radical which is substituted by up to two radicals R⁶ which are independent of one another,
where R⁶ can be
fluorine, chlorine, bromine, cyano, trifluoromethyl, nitro, amino, C₁-C₄-alkyl, C₁-C₄-alkoxy, (C₁-C₄-alkyl)oxycarbonyl, phenyl or phenoxy,
R² is hydrogen and R⁵ is
C₁-C₆-alkyl,
optionally substituted by fluorine, chlorine, hydroxyl, C₁-C₄-acyloxy, benzoyloxy, benzyloxy, phenoxy, C₁-C₄-alkoxy, C₁-C₄-alkylamino, di(C₁-C₄-alkyl) amino, C₁-C₄-alkylthio, oxo, thioxo, carboxyl or carbamoyl;
C₂-C₆-alkenyl,
optionally substituted by
fluorine, chlorine, hydroxyl, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, oxo, thioxo, carboxyl or carbamoyl;
C₃-C₈-allenyl,
C₃-C₈-alkynyl,
optionally substituted by
fluorine, chlorine, hydroxyl, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, oxo, thioxo, carboxyl or carbamoyl;
C₃-C₈-cycloalkyl,
optionally substituted by
fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di(C₁-C₄-alkyl) amino, Cᵢ-C₄-alkylthio, oxo, thioxo, carboxyl or carbamoyl;
C₃-C₈-cycloalkenyl,
optionally substituted by
fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di(C₁-C₄-alkyl) amino, C₁-C₄-alkylthio, oxo, thioxo, carboxyl or carbamoyl;
(C₃-C₆-cycloalkyl) - (C₁-C₂-alkyl),
optionally substituted by
fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di(C₁-C₄-alkyl) amino, C₁-C₄-alkylthio, oxo, thioxo, carboxyl or carbamoyl;
(C₃-C₆-cycloalkenyl) - (C₁-C₂-alkyl),
optionally substituted by
fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di (C₁-C₄-alkyl) amino, C₁-C₄-alkylthio, oxo, thioxo, carboxyl or carbamoyl;
C₁-C₆-alkylcarbonyl,
optionally substituted by
fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, C₁-C₄-alkenylamino, di(C₁-C₄-alkyl)amino, 1-pyrrolidinyl, piperidino, morpholino, 4-methylpiperazin-1-yl, C₁-C₄-alkylthio, oxo, thioxo, carboxyl or carbamoyl;
C₂-C₆-alkenylcarbonyl, optionally substituted by fluorine, chlorine or hydroxyl;
(C₃-C₆-cycloalkyl)carbonyl,
(C₅-C₆-cycloalkenyl)carbonyl,
(C₃-C₆-cycloalkyl)-(C₁-C₂-alkyl)carbonyl,
(C₅-C₆-cycloalkenyl) - (C₁-C₂-alkyl) carbonyl, C₁-C₆-alkyloxycarbonyl, optionally substituted by fluorine, chlorine, bromine, hydroxyl, C₁-C₄-alkoxy, C₁-C₄-alkylamino, di(C₁-C₄-alkyl)amino or C₁-C₄-alkylthio;
C₂-C₆-alkenyloxycarbonyl, optionally substituted by fluorine, chlorine, hydroxyl, C₁-C₄-alkoxy;
C₂-C₆-alkynyloxycarbonyl, optionally substituted by fluorine, chlorine, hydroxyl, C₁-C₄-alkoxy;
C₁-C₆-alkylthiocarbonyl, optionally substituted by fluorine, chlorine, hydroxyl, C₁-C₄-alkoxy;
C₂-C₆-alkenylthiocarbonyl, optionally substituted by fluorine, chlorine, hydroxyl, C₁-C₄-alkoxy;
C₁-C₆-alkylamino- and di(C₁-C₆-alkyl)aminocarbonyl, in each case optionally substituted by fluorine, chlorine, hydroxyl, C₁-C₄-alkoxy;
pyrrolidin-1-yl, morpholino-, piperidino-, piperazinyl-, or 4-methylpiperazin-1-ylcarbonyl;
C₂-C₆-alkenylamino- and di(C₁-C₆-alkenyl)aminocarbonyl, in each case optionally substituted by fluorine, chlorine, hydroxyl, C₁-C₄-alkoxy;
C₁-C₄-alkylsulfonyl, optionally substituted by fluorine, chlorine, hydroxyl, C₁-C₄-alkoxy;
C₁-C₄-alkenylsulfonyl;
or aryl, arylcarbonyl, (arylthio)carbonyl, aryloxycarbonyl, arylaminocarbonyl, (arylamino)thiocarbonyl, arylsulfonyl, arylalkylaminocarbonyl, arylalkyl, arylalkenyl, arylalkylcarbonyl, arylalkoxycarbonyl or aryl(alkylthio)carbonyl, each of which is substituted by up to two radicals R⁶ which are independent of one another, it being possible for the alkyl radical to contain in each case 1 to 3 carbon atoms, and R⁶ being as defined above,
or 1- or 2-naphthylmethyl, 2-, 3- or 4-picolyl, 2-or 3-furylmethyl, 2- or 3-thienylmethyl, 2- or 3-pyrrolylmethyl, 2-, 3- or 4-pyridylcarbonyl, 2-or 3-furylcarbonyl, 2- or 3-thienylcarbonyl, 2- or 3-thienylacetyl, 2-, 3- or 4-picolyloxycarbonyl, 2- or 3-furylmethyloxycarbonyl or 2- or 3-thienylmethyloxycarbonyl, each of which is substituted by up to two radicals R⁶ which are independent of one another,
and
R³ and R⁴ are identical or different and independently of one another are hydrogen, C₁-C₄-alkyl, optionally substituted by fluorine, chlorine, hydroxyl, amino, mercapto, C₁-C₄-acyloxy, benzoyloxy, phenoxy, C₁-C₄-alkoxy, C₁-C₄-alkylamino, di(C₁-C₄-alkyl)amino, C₁-C₄-alkylthio, C₁-C₄-alkylsulfonyl, C₁-C₄-alkylsulfinyl, carboxyl or carbamoyl;
C₂-C₆-alkenyl, optionally substituted by fluorine or chlorine;
C₃-C₆-cycloalkyl, optionally substituted by fluorine, chlorine, hydroxyl, amino, mercapto, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, C₁-C₄-alkylsulfonyl, C₁-C₄-alkylsulfinyl, carboxyl or carbamoyl;
C₃-C₈-cycloalkenyl, optionally substituted by fluorine or chlorine;
aryl, benzyl, heteroaryl or heteroarylmethyl, each of which is substituted by up to two radicals R⁶ which are independent of one another,
R³ and R⁴ can furthermore also be
part of a saturated or unsaturated carbo- or heterocyclic ring which has 3 to 6 carbon atoms and which can optionally be substituted by fluorine, chlorine, hydroxyl, amino, C₁-C₄-acyloxy, benzoyloxy, C₁-C₄-alkoxy, oxo, thioxo, carboxyl or carbamoyl, and
X is oxygen or sulfur,
wherein the nucleoside is selected from the group consisting of zidovudine and lamivudin.

2. A combination preparation as claimed in claim 1, wherein the substituents of the compounds of the formula I or Ia have the following meanings
n is zero,
one
or two,
the individual substituents R¹ independently of one another are
fluorine, chlorine, bromine, trifluoromethyl, hydroxyl, C₁-C₄-alkyl, C₁-C₄-alkoxy, (C₁-C₄-alkoxy)-(C₁-C₂-alkoxy), C₁-C₄-alkylthio, nitro, amino, C₁-C₄-alkylamino, di(C₁-C₄-alkyl)amino, piperidino, morpholino, 1-pyrrolidinyl, 4-methylpiperazinyl, C₁-C₄-acyl, C₁-C₄-acyloxy, C₁-C₄-acylamino, cyano, carbamoyl, carboxyl, (C₁-C₄-alkyl)oxycarbonyl, hydroxysulfonyl or sulfamoyl
or
a phenyl, phenoxy, phenylthio, phenylsulfonyl, phenoxysulfonyl, benzoyl, 2-pyridyl, 3-pyridyl or 4-pyridyl radical, each of which is substituted by up to two radicals R⁶ which are independent of one another,
where R⁶ can be
fluorine, chlorine, bromine, cyano, trifluoromethyl, nitro, amino, C₁-C₄-alkyl, C₁-C₄-alkoxy, (C₁-C₄-alkyl)oxycarbonyl, phenyl or phenoxy,
R² is hydrogen and R⁵ is
C₁-C₆-alkyl,
optionally substituted by C₁-C₄-alkoxy or C₁-C₄-alkylthio;
C₂-C₆-alkenyl,
optionally substituted by oxo;
C₃-C₆-allenyl;
C₃-C₈-alkynyl, in particular 2-butynyl;
C₃-C₆-cycloalkyl;
C₅-C₆-cycloalkenyl;
(C₃-C₆-cycloalkyl)-(C₁-C₂-alkyl), in particular cyclopropylmethyl, optionally substituted by C₁-C₄-alkyl;
(C₃-C₆-cycloalkenyl)-(C₁-C₂-alkyl), in particular cyclohexenylmethyl;
C₁-C₆-alkylcarbonyl,
optionally substituted by
fluorine, chlorine, hydroxyl, benzyloxy, phenoxy, C₁-C₄-alkoxy, C₁-C₄-alkylamino, C₁-C₄-alkenylamino, di(C₁-C₄-alkyl)amino, 1-pyrrolidinyl, piperidino, morpholino, 4-methylpiperazin-1-yl or C₁-C₄-alkylthio;
C₂-C₆-alkenylcarbonyl;
C₁-C₆-alkyloxycarbonyl, optionally substituted by fluorine, chlorine, bromine, hydroxyl, C₁-C₄-alkoxy, C₁-C₄-alkylamino, di(C₁-C₄-alkyl) amino or C₁-C₄-alkylthio;
C₂-C₆-alkenyloxycarbonyl, in particular vinyloxycarbonyl, allyloxycarbonyl, isopropenyloxycarbonyl, butenyloxycarbonyl or pentenyloxycarbonyl;
C₂-C₆-alkynyloxycarbonyl, in particular propynyloxycarbonyl or butynyloxycarbonyl;
C₁-C₆-alkylthiocarbonyl;
C₂-C₆-alkenylthiocarbonyl, in particular allylthiocarbonyl;
C₁-C₆-alkylamino- and di(C₁-C₆-alkyl)aminocarbonyl;
pyrrolidin-1-yl, morpholino-, piperidino-, piperazinyl-, or 4-methylpiperazin-1-ylcarbonyl;
C₂-C₆-alkenylamino- and di(C₁-C₆-alkenyl)aminocarbonyl;
C₁-C₄-alkylsulfonyl;
C₁-C₄-alkenylsulfonyl;
or aryl which is substituted by up to two radicals R⁶ which are independent of one another, in particular phenyl, arylcarbonyl, in particular benzoyl, (arylthio)carbonyl, aryloxycarbonyl, arylaminocarbonyl, (arylamino)thiocarbonyl, arylalkylaminocarbonyl, arylsulfonyl, arylalkyl, in particular benzyl, phenylethyl, arylalkenyl, arylalkylcarbonyl, arylalkoxycarbonyl or aryl(alkylthio)carbonyl, it being possible for the alkyl radical to contain in each case 1 to 3 carbon atoms and R⁶ being as defined above,
or 1- or 2-naphthylmethyl, 2-, 3- or 4-picolyl, 2-or 3-furylmethyl, 2- or 3-thienylmethyl, 2- or 3-pyrrolylmethyl, 2-, 3- or 4-pyridylcarbonyl, 2-or 3-furylcarbonyl, 2- or 3-thienylcarbonyl, 2- or 3-thienylacetyl, 2-, 3- or 4-picolyloxycarbonyl, 2- or 3-furylmethyloxycarbonyl, or 2- or 3-thienylmethyloxycarbonyl, each of which is substituted by up to two radicals R⁶ which are independent of one another,
and
R³ and R⁴ are identical or different and independently of one another are
hydrogen,
C₁-C₄-alkyl,
optionally substituted by hydroxyl, mercapto, C₁-C₄-alkoxy, C₁-C₄-alkylthio, C₁-C₄-alkylsulfonyl, C₁-C₄-alkylsulfinyl, carboxyl or carbamoyl;
C₂-C₆-alkenyl,
aryl, benzyl, thienyl or thienylmethyl, each of which is substituted by up to two radicals R⁶ which are independent of one another, R⁶ being as defined above,
R³ and R⁴ can also be
part of a saturated or unsaturated carbo- or heterocyclic ring which has 3 to 6 carbon atoms and can optionally be substituted by oxo or thioxo, and
X is oxygen or sulfur.

3. A combination preparation as claimed in claim 1 or 2, wherein the substituents of the compounds of the formula I or Ia have the following meanings
n is zero or
one,
the individual substituents R¹ independently of one another are
fluorine, chlorine, bromine, C₁-C₂-alkyl, C₁-C₂-alkoxy, C₂-C₄-acyl or cyano,
R² is hydrogen and R⁵ is
C₂-C₆-alkenyl,
C₃-C₈-alkynyl, in particular 2-butynyl;
(C₃-C₆-cycloalkyl)-(C₁-C₂-alkyl), in particular cyclopropylmethyl, optionally substituted by C₁-C₄-alkyl;
(C₃-C₆-cycloalkenyl)-(C₁-C₂-alkyl), in particular cyclohexenylmethyl;
C₂-C₆-alkylcarbonyl,
C₂-C₆-alkenylcarbonyl;
C₁-C₆-alkyloxycarbonyl;
C₂-C₆-alkenyloxycarbonyl, in particular vinyloxycarbonyl, allyloxycarbonyl, isopropenyloxycarbonyl, butenyloxycarbonyl or pentenyloxycarbonyl;
C₂-C₆-alkynyloxycarbonyl, in particular propynyloxycarbonyl or butynyloxycarbonyl;
C₂-C₆-alkenylthiocarbonyl, in particular allylthiocarbonyl;
C₁-C₄-alkylsulfonyl;
C₁-C₄-alkenylsulfonyl;
or arylalkyl, in particular benzyl or arylalkenyl, which is substituted by up to two radicals R⁶ which are independent of one another, it being possible for the alkyl radical to contain in each case 1 to 3 carbon atoms and for the alkenyl radical to contain 2-3 carbon atoms,
or 1-naphthylmethyl, 2- or 3-picolyl, 2-furylmethyl or 2- or 3-thienylmethyl, each of which is substituted by up to two radicals R⁶ which are independent of one another,
where R⁶ is
fluorine, chlorine, bromine, cyano, C₁-C₂-alkyl or C₁-C₂-alkoxy,
and
R³ and R⁴ are identical or different and independently of
one another are
hydrogen,
C₁-C₄-alkyl,
optionally substituted by hydroxyl, mercapto, C₁-C₄-alkoxy, C₁-C₂-alkylthio, and
X is oxygen or sulfur.

4. A combination preparation as claimed in one or more of claims 1-3 which comprises zidovudine and S-4-isopropoxycarbonyl-6-methoxy-3-(methylthiomethyl)-3,4-dihydroquinoxaline-2-(1H)-thione.

5. A pharmaceutical which comprises an effective amount of a combination preparation as claimed in one or more of claims 1-4, if appropriate in addition to customary auxiliaries and/or excipients.

6. A combination preparation as claimed in one or more of claims 1-4 for use as pharmaceutical.

7. A combination preparation as claimed in one or more of claims 1-4 for the preparation of pharmacueticals for treating viral diseases.

8. A process for the preparation of a pharmacuetical, which comprises bringing a combination preparation as claimed in one or more of claims 1-4, if appropriate together with customary auxiliaries and/or excipients, into a suitable dosage form.

## Revendications

1. Préparation en combinaison, contenant au moins un nucléoside et au moins un composé ayant les formule I et/ou Ia, 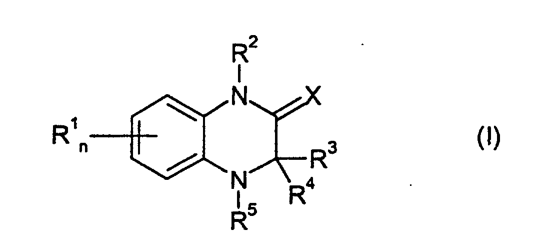 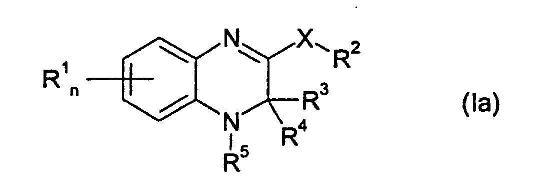 ou leurs sels ou prodrogues compatibles d'un point de vue physiologique, où, dans les formules I et Ia,
n vaut zéro, un ou deux,
les différents substituants R¹ représentent chacun indépendamment de l'autre un groupe fluoro, chloro, bromo, trifluorométhyle, hydroxy, alkyle en C1-C4, alcoxy en C1-C4, (alcoxy en C1-C4)-(alcoxy en C1-C4), alkylthio en C1-C4, nitro, amino, alkylamino en C1-C4, di(alkyle en C1-C4)amino, pipéridino, morpholino, 1-pyrrolidinyle, 4-méthylpipérazinyle, acyle en C1-C4, acyloxy en C1-C4, acylamino en C1-C4, cyano, carbamoyle, carboxy, (alkyle en C1-C4)-oxycarbonyle, hydroxysulfonyle, sulfamoyle ;
ou encore représentent chacun un radical phényle, phénoxy, phénylthio, phénylsulfonyle, phénoxysulfonyle, benzoyle, 2-pyridyle, 3-pyridyle ou 4-pyridyle, substitué par jusqu'à deux radicaux R⁶ indépendants l'un de l'autre,
où R⁶ peut être un groupe fluoro, chloro, bromo, cyano, trifluorométhyle, nitro, amino, alkyle en C1-C4, alcoxy en C1-C4, (alkyle en C1-C4)-oxycarbonyle, phényle, phénoxy ;
R² est un atome d'hydrogène, et R⁵ est un groupe
alkyle en C1-C6, éventuellement substitué par un ou plusieurs substituants fluoro, chloro, hydroxy, acyloxy en C1-C4, benzoyloxy, benzyloxy, phénoxy, alcoxy en C1-C4, alkylamino en C1-C4, di(alkyle en C1-C4)amino, alkylthio en C1-C4, oxo, thioxo, carboxy, carbamoyle ;
alcényle en C2-C6, éventuellement substitué par un ou plusieurs substituants fluoro, chloro, hydroxy, acyloxy en C1-C4, benzoyloxy, benzyloxy, phénoxy, alcoxy en C1-C4, alkylamino en C1-C4, di(alkyle en C1-C4)amino, alkylthio en C1-C4, oxo, thioxo, carboxy, carbamoyle ;
allényle en C3-C8 ;
alcynyle en C3-C8, éventuellement substitué par un ou plusieurs substituants fluoro, chloro, hydroxy, acyloxy en C1-C4, benzoyloxy, benzyloxy, phénoxy, alcoxy en C1-C4, alkylamino en C1-C4, di(alkyle en C1-C4)amino, alkylthio en C1-C4, oxo, thioxo, carboxy, carbamoyle ;
cycloalkyle en C3-C8, éventuellement substitué par un ou plusieurs substituants fluoro, chloro, hydroxy, alkyle en C1-C4, acyloxy en C1-C4, benzoyloxy, benzyloxy, phénoxy, alcoxy en C1-C4, alkylamino en C1-C4, di(alkyle en C1-C4)amino, alkylthio en C1-C4, oxo, thioxo, carboxy, carbamoyle ;
cycloalcényle en C3-C8, éventuellement substitué par un ou plusieurs substituants fluoro, chloro, hydroxy, alkyle en C1-C4, acyloxy en C1-C4, benzoyloxy, benzyloxy, phénoxy, alcoxy en C1-C4, alkylamino en C1-C4, di(alkyle en C1-C4)amino, alkylthio en C1-C4, oxo, thioxo, carboxy, carbamoyle ;
(cycloalkyle en C3-C6)-(alkyle en C1-C2), éventuellement substitué par un ou plusieurs substituants fluoro, chloro, hydroxy, alkyle en C1-C4, acyloxy en C1-C4, benzoyloxy, benzyloxy, phénoxy, alcoxy en C1-C4, alkylamino en C1-C4, di(alkyle en C1-C4)amino, alkylthio en C1-C4, oxo, thioxo, carboxy, carbamoyle ;
(cycloalcényle en C3-C6)-(alkyle en C1-C2), éventuel-lement substitué par un ou plusieurs substituants fluoro, chloro, hydroxy, alkyle en C1-C4, acyloxy en C1-C4, benzoyloxy, benzyloxy, phénoxy, alcoxy en C1-C4, alkyl-amino en C1-C4, di(alkyle en C1-C4)amino, alkylthio en C1-C4, oxo, thioxo, carboxy, carbamoyle ;
(alkyle en C1-C6)carbonyle, éventuellement substitué par un ou plusieurs substituants fluoro, chloro, hydroxy, alkyle en C1-C4, acyloxy en C1-C4, benzoyloxy, benzyloxy, phénoxy, alcoxy en C1-C4, alkylamino en C1-C4, alcénylamino en C1-C4, di(alkyle en C1-C4)amino, 1-pyrrolidinyle, pipéridino, morpholino, 4-méthylpipérazine-1-yle, alkyl-thio en C1-C4, oxo, thioxo, carboxy, carbamoyle ;
(alcényle en C2-C6)carbonyle, éventuellement substitué par un ou plusieurs substituants fluoro, chloro ou hydroxy ;
(cycloalkyle en C3-C6)carbonyle,
(cycloalcényle en C5-C6)carbonyle,
(cycloalkyle en C3-C6)-(alkyle en C1-C2)carbonyle,
(cycloalcényle en C5-C6)-(alkyle en C1-C2)carbonyle,
(alkyle en C1-C6)oxycarbonyle, éventuellement substitué par un ou plusieurs substituants fluoro, chloro, bromo, hydroxy, alcoxy en C1-C4, alkylamino en C1-C4, di(alkyle en C1-C4)amino, alkylthio en C1-C4 ;
(alcényle en C2-C6)oxycarbonyle, éventuellement substitué par un ou plusieurs substituants fluoro, chloro, hydroxy, alcoxy en C1-C4 ;
(alcynyle en C2-C6)oxycarbonyle, éventuellement substitué par un ou plusieurs substituants fluoro, chloro, hydroxy, alcoxy en C1-C4 ;
(alkyle en C1-C6)thiocarbonyle, éventuellement substitué par un ou plusieurs substituants fluoro, chloro, hydroxy, alcoxy en C1-C4 ;
(alcényle en C2-C6)thiocarbonyle, éventuellement substitué par un ou plusieurs substituants fluoro, chloro, hydroxy, alcoxy en C1-C4 ;
(alkylamino en C1-C6)- et di(alkyle en C1-C6)aminocarbonyle, éventuellement substitué par un ou plusieurs substituants fluoro, chloro, hydroxy, alcoxy en C1-C4 ;
pyrrolidine-1-yle, morpholino, pipéridino, pipérazinyle ou 4-méthylpipérazine-1-yl-carbonyle ;
(alcénylamino en C2-C6)- et di(alcényle en C1-C6)-aminocarbonyle, éventuellement substitué par un ou plusieurs substituants fluoro, chloro, hydroxy, alcoxy en C1-C4 ;
alkylsulfonyle en C1-C4, éventuellement substitué par un ou plusieurs substituants fluoro, chloro, hydroxy, alcoxy en C1-C4 ;
alcénylsulfonyle en C1-C4 ;
ou encore un radical aryle, arylcarbonyle, (arylthio)carbonyle, aryloxycarbonyle, arylaminocarbonyle, (arylamino)thiocarbonyle, arylsulfonyle, arylalkylaminocarbonyle, arylalkyle, arylalcényle, arylalkylcarbonyle, arylalcoxycarbonyle, aryl(alkylthio)carbonyle substitué par jusqu'à deux radicaux R⁶ indépendants l'un de l'autre, le radical alkyle pouvant dans chaque cas contenir de 1 à 3 atomes de carbone, et R⁶ étant tel que défini ci-dessus ;
ou encore un groupe 1- ou 2-naphtylméthyle, 2-, 3-ou 4-picolyle, 2- ou 3-furylméthyle, 2- ou 3-thiénylméthyle, 2- ou 3-pyrrolylméthyle, 2-, 3- ou 4-pyridylcarbonyle, 2- ou 3-furylcarbonyle, 2- ou 3-thiénylcarbonyle, 2- ou 3-thiénylacétyle, 2-, 3- ou 4-picolyloxycarbonyle, 2- ou 3-furylméthyloxycarbonyle, 2-ou 3-thiénylméthyloxy-carbonyle, substitué par jusqu'à deux radicaux R⁶ indépen-dants l'un de l'autre,
et
R³ et R⁴ sont identiques ou différents et représentent chacun indépendamment de l'autre un atome d'hydrogène, un groupe alkyle en C1-C4, éventuellement substitué par un ou plusieurs substituants fluoro, chloro, hydroxy, amino, mercapto, acyloxy en C1-C4, benzoyloxy, phénoxy, alcoxy en C1-C4, alkylamino en C1-C4, di(alkyle en C1-C4)amino, alkylthio en C1-C4, alkylsulfonyle en C1-C4, alkylsulfinyle en C1-C4, carboxy, carbamoyle ;
alcényle en C2-C6, éventuellement substitué par un ou plusieurs substituants fluoro ou chloro ;
cycloalkyle en C3-C6, éventuellement substitué par un ou plusieurs substituants fluoro, chloro, hydroxy, amino, mercapto, acyloxy en C1-C4, benzoyloxy, phénoxy, alcoxy en C1-C4, alkylamino en C1-C4, di(alkyle en C1-C4)amino, alkylthio en C1-C4, alkylsulfonyle en C1-C4, alkylsulfinyle en C1-C4, carboxy, carbamoyle ;
cycloalcényle en C3-C8, éventuellement substitué par un ou plusieurs substituants fluoro ou chlore ;
aryle, benzyle, hétéroaryle ou hétéroarylméthyle, substitué par jusqu'à deux radicaux R⁶ indépendants l'un de l'autre ;
R³ et R⁴ peuvent en outre représenter une partie d'un noyau carbocyclique ou hétérocyclique saturé ou insaturé ayant de 3 à 6 atomes de carbone, qui peut éventuellement être substitué par un ou plusieurs substituants fluoro, chloro, hydroxy, amino, acyloxy en C1-C4, benzoyloxy, alcoxy en C1-C4, oxo, thioxo, carboxy, carbamoyle, et
X est un atome d'oxygène ou de soufre,
**caractérisée en ce que** le nucléoside est choisi dans l'ensemble comprenant la zidovudine et la lamivudine.

2. Préparation en combinaison selon la revendication 1, **caractérisée en ce que** les substituants des composés de formule I ou Ia ont les significations suivantes :
n vaut zéro, un ou deux,
les différents substituants R¹ représentent chacun indépendamment de l'autre un groupe fluoro, chloro, bromo, trifluorométhyle, hydroxy, alkyle en C1-C4, alcoxy en C1-C4, (alcoxy en C1-C4)-(alcoxy en C1-C2), alkylthio en C1-C4, nitro, amino, alkylamino en C1-C4, di(alkyle en C1-C4)amino, pipéridino, morpholino, 1-pyrrolidinyle, 4-méthylpipérazinyle, acyle en C1-C4, acyloxy en C1-C4, acylamino en C1-C4, cyano, carbamoyle, carboxy, (alkyle en C1-C4)-oxycarbonyle, hydroxysulfonyle, sulfamoyle ;
ou encore représentent chacun un radical phényle, phénoxy, phénylthio, phénylsulfonyle, phénoxysulfonyle, benzoyle, 2-pyridyle, 3-pyridyle ou 4-pyridyle, substitué par jusqu'à deux radicaux R⁶ indépendants l'un de l'autre,
où R⁶ peut être un groupe fluoro, chloro, bromo, cyano, trifluorométhyle, nitro, amino, alkyle en C1-C4, alcoxy en C1-C4, (alkyle en C1-C4)-oxycarbonyle, phényle, phénoxy ;
R² est un atome d'hydrogène, et R⁵ est un groupe
alkyle en C1-C6, éventuellement substitué par un ou plusieurs substituants alcoxy en C1-C4 ou alkylthio en C1-C4 ;
alcényle en C2-C6, éventuellement substitué par un ou plusieurs substituants oxo ;
allényle en C3-C6 ;
alcynyle en C3-C8, en particulier 2-butynyle ;
cycloalkyle en C3-C6 ;
cycloalcényle en C5-C6 ;
(cycloalkyle en C3-C6)-(alkyle en C1-C2), en particulier cyclopropylméthyle, éventuellement substitué par un ou plusieurs substituants alkyle en C1-C4 ;
(cycloalcényle en C3-C6)-(alkyle en C1-C2), en particulier cyclohexénylméthyle ;
(alkyle en C1-C6)carbonyle, éventuellement substitué par un ou plusieurs substituants fluoro, chloro, hydroxy, benzyloxy, phénoxy, alcoxy en C1-C4, alkylamino en C1-C4, alcénylamino en C1-C4, di(alkyle en C1-C4)amino, 1-pyrrolidinyle, pipéridino, morpholino, 4-méthylpipérazine-1-yle, alkylthio en C1-C4 ;
(alcényle en C2-C6)carbonyle ;
(alkyle en C1-C6)oxycarbonyle, éventuellement substitué par un ou plusieurs substituants fluoro, chloro, bromo, hydroxy, alcoxy en C1-C4, alkylamino en C1-C4, di(alkyle en C1-C4)amino, alkylthio en C1-C4 ;
(alcényle en C2-C6)oxycarbonyle, en particulier vinyloxycarbonyle, allyloxycarbonyle, isopropényloxycarbonyle, butényloxycarbonyle, pentényloxycarbonyle ;
(alcynyle en C2-C6)oxycarbonyle, en particulier propynyloxycarbonyle, butynyloxycarbonyle ;
(alkyle en C1-C6)thiocarbonyle ;
(alcényle en C2-C6)thiocarbonyle, en particulier allylthiocarbonyle ;
(alkylamino en C1-C6)- et di(alkyle en C1-C6)aminocarbonyle ;
pyrrolidine-1-yle, morpholino, pipéridino, pipérazinyle ou 4-méthylpipérazine-1-yl-carbonyle ;
(alcénylamino en C2-C6)- et di(alcényle en C1-C6)aminocarbonyle,
alkylsulfonyle en C1-C4 ;
alcénylsulfonyle en C1-C4 ;
ou aryle, en particulier phényle ; arylcarbonyle, en particulier benzoyle, (arylthio)carbonyle, aryloxycarbonyle, arylaminocarbonyle, (arylamino)thiocarbonyle, arylalkylaminocarbonyle, arylsulfonyle ; arylalkyle, en particulier benzyle, phényléthyle, arylalcényle ; arylalkylcarbonyle, arylalcoxycarbonyle, aryl(alkylthio)-carbonyle, substitué par jusqu'à deux radicaux R⁶ indépendants l'un de l'autre, le radical alkyle pouvant dans chaque cas contenir de 1 à 3 atomes de carbone, et R⁶ étant tel que défini ci-dessus,
ou encore un groupe 1- ou 2-naphtylméthyle, 2-, 3-ou 4-picolyle, 2- ou 3-furylméthyle, 2- ou 3-thiénylméthyle, 2- ou 3-pyrrolylméthyle, 2-, 3- ou 4-pyridylcarbonyle, 2- ou 3-furylcarbonyle, 2- ou 3-thiénylcarbonyle, 2- ou 3-thiénylacétyle, 2-, 3- ou 4-picolyloxycarbonyle, 2- ou 3-furylméthyloxycarbonyle, 2- ou 3-thiénylméthyloxycarbonyle, substitué par jusqu'à deux radicaux R⁶ indépendants l'un de l'autre, et
R³ et R⁴ sont identiques ou différents et représentent chacun indépendamment de l'autre
un atome d'hydrogène,
un groupe alkyle en C1-C4, éventuellement substitué par un ou plusieurs substituants hydroxy, mercapto, alcoxy en C1-C4, alkylthio en C1-C4, alkylsulfonyle en C1-C4, alkylsulfinyle en C1-C4, carboxy, carbamoyle ;
alcényle en C2-C6 ;
aryle, benzyle, thiényle ou thiénylméthyle substitué par jusqu'à deux radicaux R⁶ indépendamment l'un de l'autre, R⁶ étant tel que défini ci-dessus,
R³ et R⁴ peuvent aussi représenter une partie d'un noyau carbocyclique ou hétérocyclique saturé ou insaturé ayant de 3 à 6 atomes de carbone, qui peut être éventuellement substitué par un ou plusieurs substituants oxo ou thioxo, et
X représente l'oxygène ou le soufre.

3. Préparation en combinaison selon la revendication 1 ou 2, **caractérisée en ce que** les substituants des compo-sés de la formule I et Ia ont les significations suivantes :
n vaut zéro ou un,
les différents substituants R¹ représentent chacun indépendamment des autres un groupe fluoro, chloro, bromo, alkyle en C1-C2, alcoxy en C1-C2, acyle en C2-C4, cyano,
R² est un atome d'hydrogène et R⁵ est un groupe alcényle en C2-C6,
alcynyle en C3-C8, en particulier 2-butynyle ;
(cycloalkyle en C3-C6)-(alkyle en C1-C2), en particulier cyclopropylméthyle, éventuellement substitué par un ou plusieurs substituants alkyle en C1-C4 ;
(cycloalcényle en C3-C6)-(alkyle en C1-C2), en particulier cyclohexénylméthyle ;
(alkyle en C2-C6)carbonyle,
(alcényle en C2-C6)carbonyle ;
(alkyle en C1-C6)oxycarbonyle ;
(alcényle en C2-C6)oxycarbonyle, en particulier vinyloxycarbonyle, allyloxycarbonyle, isopropényloxycarbonyle, butényloxycarbonyle, pentényloxycarbonyle ;
(alcynyle en C2-C6)oxycarbonyle, en particulier propynyloxycarbonyle, butynyloxycarbonyle ;
(alcényle en C2-C6)thiocarbonyle, en particulier allylthiocarbonyle ;
alkylsulfonyle en C1-C4 ;
alcénylsulfonyle en C1-C4 ;
ou encore arylalkyle en particulier benzyle ou arylalcényle, substitué par jusqu'à deux radicaux R⁶ indépendamment l'un de l'autre, le radical alkyle pouvant dans chaque avoir de 1 à 3 atomes de carbone et le radical alcényle 2-3 atomes de carbone,
ou encore 1-naphtylméthyle, 2- ou 3-picolyle, 2-furylméthyle, 2- ou 3-thiénylméthyle substitué par jusqu'à deux radicaux R⁶ indépendants l'un de l'autre,
où R⁶ est un groupe fluoro, chloro, bromo, cyano, alkyle en C1-C2, alcoxy en C1-C2,
et
R³ et R⁴ sont identiques ou différents et représentent chacun indépendamment de l'autre
un atome d'hydrogène,
un groupe alkyle en C1-C4, éventuellement substitué par un ou plusieurs substituants hydroxy, mercapto, alcoxy en C1-C4, alkylthio en C1-C2, et X est un atome d'oxygène ou de soufre.

4. Préparation en combinaison selon l'une ou plusieurs des revendications 1 à 3, **caractérisée en ce qu'**elle contient de la zidovudine et de la S-4-isopropoxy-carbonyl-6-méthoxy-3-(méthylthio-méthyl)-3,4-dihydro-quinoxaline-2(1H)-thione.

5. Médicament contenant une quantité active d'une préparation en combinaison selon l'une ou plusieurs des revendications 1 à 4, éventuellement en plus d'adjuvants et/ou excipients usuels.

6. Préparation en combinaison selon l'une ou plusieurs des revendications 1 à 4, pour utilisation en tant que médicament.

7. Préparation en combinaison selon l'une ou plusieurs des revendications 1 à 4 pour préparer des médicaments destinés au traitement de maladies virales.

8. Procédé de préparation d'un médicament, **caractérisé en ce qu'**on met sous une forme pharmaceutique appropriée une préparation en combinaison selon l'une ou plusieurs des revendications 1 à 4, éventuellement avec des adjuvants et/ou excipients usuels.
